# EUROPÄISCHE PATENTSCHRIFT

(11) **EP 1 737 952 B1**
(45) Veröffentlichungstag und Bekanntmachung des Hinweises auf die Patenterteilung: **21.11.2007**
(21) Anmeldenummer: 05738142.8
(22) Anmeldetag: 15.04.2005
(51) Int. Cl.: C12N 9/52, C12N 15/57, C11D 3/00

(54) **NEUE ALKALISCHE PROTEASEN UND WASCH- UND REINIGUNGSMITTEL, ENTHALTEND DIESE NEUEN ALKALISCHEN PROTEASEN**
NOVEL ALKALINE PROTEASES, AND DETERGENTS AND CLEANERS CONTAINING THE SAME
NOUVELLES PROTEASES ALCALINES ET AGENTS DE LAVAGE ET DETERGENTS RENFERMANT CELLES-CI

(30) Priorität: 23.04.2004 DE 102004019751
(43) Veröffentlichungstag der Anmeldung: 03.01.2007
(73) Patentinhaber: Henkel Kommanditgesellschaft auf Aktien, 40589 Düsseldorf (DE); B.R.A.I.N. Biotechnology Research And Information Network AG, 64673 Zwingenberg (DE)
(72) Erfinder: WIELAND, Susanne, 41541 Dormagen-Zons (DE); MAURER, Karl-Heinz, 40699 Erkrath (DE); KOTTWITZ, Beatrix, 40699 Erkrath (DE); NIEHAUS, Frank, 64646 Heppenheim (DE); LORENZ, Patrick, 64653 Lorsch (DE)
(86) Internationale Anmeldenummer: PCT/EP2005/003983
(87) Internationale Veröffentlichungsnummer: WO 2005/103244

(56) Entgegenhaltungen:
- WO-A-20/04033668
- DATABASE UniProt [Online] 1. November 1991 (1991-11-01), "Extracellular protease precursor (EC 3.4.21.-)." XP002336472 gefunden im EBI accession no. UNIPROT:EXPR_XANCP Database accession no. EXPR_XANCP & LIU Y N ET AL: "A multipurpose broad host range cloning vector and its use to characterise an extracellular protease gene of Xanthomonas campestris pathovar campestris." MOLECULAR & GENERAL GENETICS : MGG. FEB 1990, Bd. 220, Nr. 3, Februar 1990 (1990-02), Seiten 433-440, XP001207135 ISSN: 0026-8925

## Beschreibung

Die vorliegende Anmeldung betrifft zwei zueinander ähnliche neue Alkalische Proteasen, deren DNA aus Bodenproben erhalten wurde, C-terminal deletierte, ebenfalls proteolytisch aktive Fragmente davon und alle hinreichend ähnlichen Alkalischen Proteasen und Nukleinsäuren sowie technische Einsatzmöglichkeiten für diese Proteasen, vor allem ihren Einsatz in Wasch- und Reinigungsmitteln.

Proteasen gehören zu den technisch bedeutendsten Enzymen überhaupt. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren den Serin-Proteasen zugerechnet werden. Sie wirken als unspezifische Endopeptidasen, das heißt, sie hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisin-like Proteases" von R.Siezen, Seite 75-95 in "Subtilisin enzymes", herausgegegeben von R.Bott und C.Betzel, New York, 1996. Subtilasen werden natürlicherweise von Mikroorganismen, gebildet; hierunter sind insbesondere die von Bacillus-Spezies gebildeten und sekretierten Subtilisine als bedeutendste Gruppe innerhalb der Subtilasen zu erwähnen.

Proteasen sind neben anderen Enzymen etablierte aktive Inhaltsstoffe von Wasch- und Reinigungsmitteln. Sie bewirken dabei den Abbau proteinhaltiger Anschmutzungen auf dem Reinigungsgut. Günstigenfalls ergeben sich Synergieeffekte zwischen den Enzymen und den übrigen Bestandteilen der betreffenden Mittel. Unter den Wasch- und Reinigungsmittelproteasen nehmen Subtilasen aufgrund ihrer günstigen enzymatischen Eigenschaften wie Stabilität oder pH-Optimum eine herausragende Stellung ein. Sie eignen sich daneben noch für eine Vielzahl weiterer technischer Verwendungsmöglichkeiten, beispielsweise als Bestandteile von Kosmetika oder in der organisch-chemischen Synthese.

Das klassische Vorgehen zur Gewinnung neuer Enzyme besteht darin, Mikroorganismen-haltige Proben aus natürlichen Habitaten zu entnehmen und unter den als geeignet angesehenen Bedingungen - zum Beispiel in alkalischem Milieu - zu kultivieren. Auf diese Weise erhält man Anreicherungskulturen von Mikroorganismen, die mit einer gewissen Wahrscheinlichkeit auch Enzyme, darunter Alkalische Proteasen enthalten, welche unter den betreffenden Bedingungen aktiv sind. Hieraus werden dann beispielsweise über Ausplattieren auf proteinhaltigen Agarplatten und Ausmessen der gebildeten Lysehöfe die Mikroorganismen mit den leistungsfähigsten Enzyme ausgewählt und aufgereinigt beziehungsweise die betreffenden Gene kloniert. Solch ein Vorgehen wird beispielsweise in dem Lehrbuch "Alkalophilic Mikroorganisms. A new microbial world" von K.Horikoshi und T.Akiba (1982), Japan Scientific Societies Press, Springer-Verlag, New York, Heidelberg, Berlin, ISBN 0-387-10924-2, Kapitel 2, Seiten 9-26 beschrieben.

So werden bereits natürlicherweise, vorzugsweise mikrobiell gebildete Alkalische Proteasen in Wasch- und Reinigungsmitteln eingesetzt. Beispielsweise ist nach der Anmeldung WO 93/07276 A1 die aus *Bacillus spec.* 164-A1 erhältliche Protease 164-A1 der Firmen Chemgen Corp., Gaithersburg, MD, USA, und Vista Chemical Company, Austin, TX, USA, für den Gebrauch in Wasch- und Reinigungsmitteln geeignet. Andere Beispiele sind die Alkalische. Protease aus *Bacillus sp.* PD138, NCIMB 40338 der Fa. Novozymes A/S, Bagsvaerd, Dänemark, (WO 93118140 A1), die aus *Bacillus sp.* ferm. BP-3376 stammende Proteinase K-16 der Fa. Kao Corp., Tokyo, Japan, (US-Patent 5344770) und gemäß WO 96/25489 A1 (Fa. Procter & Gamble, Cincinatti, OH, USA) die Protease aus dem psychrophilen Organismus *Flavobacterium balustinum.*

Natürliche Proteasen werden über an sich bekannte Mutagenese-Verfahren beispielsweise für den Einsatz in Wasch- und Reinigungsmitteln optimiert. Dazu gehören Punktmutagenese, Deletion, Insertion oder Fusion mit anderen Proteinen oder Proteinteilen oder über sonstige Modifikationen. Die Strategie, in die bekannten Moleküle gezielte Punktmutationen einzuführen, etwa um die Waschleistung der Subtilisine zu verbessern, wird auch als Rationales Protein-Design bezeichnet. Eine ähnliche Strategie der Leistungsverbesserung besteht darin, die Oberflächenladungen und/oder den isoelektrischen Punkt der Moleküle und darüber ihre Wechselwirkungen mit dem Substrat zu verändern. So kann beispielsweise über Punktmutationen die Nettoladung der Subtilisine verändert werden, um darüber die Substratbindung insbesondere für den Einsatz in Wasch- und Reinigungsmitteln zu beeinflussen. Eine weitere, insbesondere ergänzende Strategie besteht darin, die Stabilität der betreffenden Proteasen zu erhöhen und damit ihre Wirksamkeit zu erhöhen. Eine Stabilisierung über Kopplung an ein Polymer ist für Proteasen für Kosmetika beispielsweise in dem Patent US 5230891 beschrieben; sie geht mit einer besseren Hautverträglichkeit einher. Insbesondere für Wasch- und Reinigungsmittel sind dagegen Stabilisierungen durch Punktmutationen geläufiger.

Eine moderne Richtung der Enzymentwicklung besteht darin, Elemente aus bekannten, miteinander verwandten Proteinen über statistische Verfahren zu neuen Enzymen mit bislang nicht erreichten Eigenschaften zu kombinieren. Solche Verfahren werden auch unter dem Oberbegriff Directed Evolution zusamengefaßt. Dazu gehören beispielsweise folgende Verfahren: Die StEP-Methode (Zhao et al. (1998), Nat. Biotechnol., Band 16, S. 258-261), Random priming recombination (Shao et al., (1998), Nucleic Acids Res., Band 26, S. 681-683), DNA-Shuffling (Stemmer, W.P.C. (1994), Nature, Band 370, S. 389-391) oder RACHITT (Coco, W.M. et al. (2001), Nat. Biotechnol., Band 19, S. 354-359). Eine weitere Shuffling-Methode mit der Bezeichnung "Recombining ligation reaction" (RLR) ist in WO 00/09679 A1 beschrieben.

Im folgenden soll ein Überblick über die technisch wichtigsten Alkalischen Proteasen vom Subtilisin-Typ gegeben werden. Subtilisin BPN', welches aus *Bacillus amyloliquefaciens,* beziehungsweise *B. subtilis* stammt, ist aus den Arbeiten von Vasantha et al. (1984) in J. Bacteriol., Volume 159, S. 811-819 und von J. A. Wells et al. (1983) in Nucleic Acids Research, Volume 11, S. 7911-7925 bekannt. Subtilisin BPN' dient insbesondere hinsichtlich der Numerierung der Positionen als Referenzenzym der Subtilisine.

So werden beispielsweise die auf alle Subtilisine bezogenen Punktmutationen der Anmeldung EP 251446 A1 in der Numerierung von BPN' angegeben, deren Anmeldungsgegenstand von der Fa. Procter & Gamble Comp., Cincinnati, Ohio, USA, als "Protease B" bezeichnet wird. Die BPN'-Varianten der Anmeldung EP 199404 A1 werden von Procter & Gamble Comp. als "Protease A" bezeichnet. "Proteasen C" zeichnen sich gemäß der Anmeldung WO 91/06637 A1 durch wiederum andere Punktmutationen von BPN' aus. Bei der "Protease D" handelt es sich gemäß WO 95/10591 A1 um Varianten der Protease aus *Bacillus lentus.*

Die Protease Subtilisin Carlsberg wird in den Publikationen von E. L. Smith et al. (1968) in J. Biol. Chem., Volume 243, S. 2184-2191, und von Jacobs et al. (1985) in Nucl. Acids Res., Band 13, S. 8913-8926 beschrieben. Sie wird natürlicherweise von *Bacillus licheniformis* gebildet und war, beziehungsweise ist unter dem Handelsnamen Maxatase^{®} von der Firma Genencor International Inc., Rochester, New York, USA, sowie unter dem Handelsnamen Alcalase^{®} von der Firma Novozymes A/S, Bagsværd, Dänemark, erhältlich.

Die Protease PB92 wird natürlicherweise von dem alkaliphilen Bakterium *Bacillus nov. spec.* 92 produziert und war unter dem Handelsnamen Maxacal^{®} von der Fa. Gist-Brocades, Delft, Niederlande, erhältlich. In ihrer ursprüglichen Sequenz wird sie in der Patentanmeldung EP 283075 A2 beschrieben.

Die Subtilisine 147 und 309 werden unter den Handelsnamen Esperase^{®}, beziehungsweise Savinase^{®} von der Fa. Novozymes vertrieben. Sie stammen ursprünglich aus Bacillus-Stämmen, die mit der Anmeldung GB 1243784 A offenbart werden.

Das Subtilisin DY ist ursprünglich von Nedkov et al. 1985 in Biol. Chem. Hoppe-Seyler, Band 366, S. 421-430 beschrieben worden.

Bei der Alkalischen Protease aus *B. lentus* handelt es sich um eine Alkalische Protease aus *Bacillus species,* die in der Anmeldung WO 91/02792 A1 beschrieben ist. Sie besitzt an sich bereits eine vergleichsweise hohe Stabilität gegenüber Oxidation und dem Einwirken von Detergenzien. In den Anmeldungen WO 91/02792 A1, beziehungsweise den Patenten EP 493398 B1 und US 5352604, wird deren heterologe Expression in dem Wirt B. *licheniformis* ATCC 53926 beschrieben. In den Ansprüchen des genannten US-Patents werden die Positionen 208, 210, 212, 213 und 268 als charakteristisch für die B. lentus-Alkalische Protease bezeichnet; diese entsprechen in der Zählung des maturen Proteins den Positionen 97, 99, 101, 102 und 157, in denen sich dieses Enzym von dem maturen Subtilisin 309 (Savinase^{®}) unterscheidet. Die dreidimensionale Struktur dieses Enzyms wird in der Veröffentlichung von Goddette et al. (1992) in J. Mol. Biol., Band 228, S. 580-595: "The crystal structure of the *Bacillus lentus* alkaline protease, Subtilisin BL, at 1.4 Å resolution" beschrieben. Technisch wichtige, insbesondere für den Einsatz in Wasch- und Reinigungsmitteln geeignete, über Punktmutagenese stabilisierte Varianten dieses Enzyms werden unter anderem in den Anmeldungen WO 92/21760 A1, WO 95/23221 A1, WO 02/088340 A2) und WO 03/038082 A2 offenbart.

Das von *Thermoactinomyces vulgaris* natürlicherweise gebildete Enzym Thermitase ist ursprünglich von Meloun et al. (FEBS Lett., 1983, S. 195-200) beschrieben worden. Dabei handelt es sich um ein Molekül, das insgesamt erhebliche Sequenzabweichungen gegenüber den übrigen Subtilisinen aufweist. So beträgt die Homologie zwischen den maturen Proteinen Thermitase und der Alkalischen Protease aus *B. lentus* DSM 5483 (siehe unten) 45% Identität (62% ähnliche Aminosäuren).

Auch bei der Proteinase K handelt es sich um eine Protease, die zu der Alkalischen Protease aus *B. lentus* eine vergleichsweise geringe Homologie aufweist. Sie beträgt auf der Ebene der maturen Proteine nur 33% Identität (46% ähnliche Aminosäuren). Die Proteinase K stammt ursprünglich aus dem Mikroorganismus *Tritirachium album Limber* und ist von K.-D. Jany und B. Mayer 1985 in Biol. Chem. Hoppe-Seyler, Band 366, Seite 485-492 beschrieben worden.

WO 88/07581 A1 offenbart die zueinander sehr ähnlichen Proteasen TW3 und TW7 unter anderem für den Einsatz in Wasch- und Reinigungsmitteln.

Die Bacillopeptidase F aus *Bacillus* subtilis besitzt auf Aminosäureebene nur eine Ähnlichkeit von 30% Identität zu der *B. lentus-Alkalischen* Protease. Dieses Enzym ist in der oben erwähnten Arbeit von Siezen et al. aufgeführt, bislang aber noch nicht für den Einsatz in Wasch- und Reinigungsmitteln beschrieben oder beansprucht.

Aus der Anmeldung WO 01/68821 A2 gehen neue Subtilsine mit einer guten Leistung gegenüber Ei-Anschmutzungen hervor.

Weitere Alkalische Proteasen, die von Mikroorganismen gebildet werden, die aus natürlichen Habitaten isolierbar sind, gehen beispielsweise aus den Anmeldungen WO 03/054185 A1 (aus *Bacillus gibsonii* (DSM 14391)), WO 03/056017 A2 (aus *Bacillus sp.* (DSM 14390)), WO 03/055974 A2 (aus *Bacillus sp.* (DSM 14392)) und WO 03/054184 A1 (aus *Bacillus gibsonii* (DSM 14393)) hervor. All diese Anmeldungen offenbaren auch entsprechende Wasch- und Reinigungsmittel, enthaltend diese neuen Alkalischen Proteasen.

Eine weitere Gruppe technisch wichtiger Proteasen stellen die Metalloproteasen dar, das heißt solche, die ein Metallkation als Cofaktor benötigen. Vertreter hiervon können ebenfalls der Familie der Subtilasen zugeordnet werden. So gehen beispielsweise aus der Anmeldung US 2003/0113895 A1 Metalloproteasen aus grampositiven Mikroorganismen wie *B. subtilis,* aber auch aus *S*. *cerevisiae, S. pombe, E. coli* und *H. influenzae* hervor. Wasch- und Reinigungsmittel mit Metalloproteasen offenbaren beispielsweise die Anmeldungen WO 00/60042 A1 und WO 02/36727 A1. Die nicht vorveröffentlichte Anmeldung DE 10360805.2 offenbart eine alkalische Metalloprotease, deren zugehörige DNA aus einer Bodenprobe isoliert werden konnte, sowie deren Einsatz in Wasch- und Reinigungsmitteln.

Eine Vielzahl neuer Proteasen für prinzipiell alle etablierten Anwendungsgebiete geht aus der Anmeldung WO 2004/033668 A2 hervor. Eine weitere bekannte Protease ist StmPr2 aus *Stenotrophomonas maltophilia,* die unter dem Eintrag AY253983 bei GenBank (National Center for Biotechnology Information NCBI, National Institutes of Health, Bethesda, MD, USA) hinterlegt und veröffentlicht worden ist.

Weitere bekannte Proteasen sind die unter den Handelsnamen Durazym^{®}, Relase^{®}, Everlase^{®}, Nafizym, Natalase^{®} und Kannase^{®} von der Firma Novozymes, unter den Handelsnamen, Maxapem^{®}, Purafect^{®}, Purafect OxP^{®} und Properase^{®} von der Firma Genencor, unter dem Handelsnamen Protosol^{®} von der Firma Advanced Biochemicals Ltd., Thane, Indien und unter dem Handelsnamen Wuxi^{®} von der Firma Wuxi Snyder Bioproducts Ltd., China, erhältlichen Enzyme.

Wie all diese über einen langen Zeitraum durchgeführten Arbeiten belegen, besteht ein hoher Bedarf an technisch einsetzbaren Proteasen, die sich zum Teil drastisch, zum Teil nur in wenigen Positionen von bisher bekannten Proteasen unterscheiden. Sie sollen damit ein breites Spektrum an Leistungsunterschieden abdecken, was vor allem für ihren Einsatz in Wasch- und Reinigungsmitteln gilt, welche ihrerseits ein breites Anwendungsgebiet darstellen. So zeichnet sich eine geeignete Protease für Wasch- oder Reinigungsmittel vorzugsweise durch eine gewisse Unempfindlichkeit gegenüber den entsprechenden Bedingungen - wie Anwesenheit von an sich denaturierenden Tensiden, von Bleiche, hohen Temperaturen etc. - und durch gute Leistungen gegenüber entsprechenden Substraten wie etwa den in Lebensmittelresten zu findenden Proteinen aus.

Wie ebenfalls deutlich wird, besteht nach wie vor auch ein ungebrochener Bedarf nach neuen Alkalischen Proteasen, die an sich bereits brauchbar sind und die über die diversen Mutagenesemöglichkeiten spezifisch weiter optimiert werden können. Insbesondere aufgrund der jüngst etablierten Shuffling-Technologien sind derartige neue Proteasen von Interesse. Denn deren bislang unbekannte Nukleotidsequenzen erweitern - auch wenn das betreffende Enzym selbst eine vergleichsweise bescheidene Leistung erbringen sollte - den Varianzraum für neue Shuffling-Ansätze etwa mit bekannten Sequenzen und damit für wiederum völlig neue artifizielle Enzyme.

Der vorliegenden Erfindung lag somit die Aufgabe zugrunde, neue Alkalische Proteasen aufzufinden. Insbesondere sollten solche gefunden werden, die naturgemäß bereits eine Leistungsverbesserung von Wasch- oder Reinigungsmitteln bewirken.

Hiermit war die Teilaufgabe verbunden, ein geeignetes Verfahren zur Isolierung solch einer Protease zu etablieren. Weitere Teilaufgaben bestanden darin, Nukleinsäuren zur Verfügung zu stellen, die für derartige Proteasen codieren, um darüber gentechnische und mikrobiologische Elemente zu erhalten, die zur Gewinnung und Weiterentwicklung derartiger Proteasen genutzt werden können und gegebenenfalls in ein Shuffling eingebracht werden können. Ferner sollten entsprechende Mittel, insbesondere Wasch- und Reinigungsmittel, entsprechende Wasch- und Reinigungsverfahren sowie entsprechende Verfahren und Verwendungsmöglichkeiten für derartige Proteasen zur Verfügung gestellt werden. Schließlich sollten technische Einsatzmöglichkeiten für die gefundenen Proteasen definiert werden.

Zur Bewältigung dieser Aufgabe wurde ein bislang kaum beschrittener Weg genutzt. So wurde keine klassische Anreichungskultur für alkaliphile, proteasepositive Mikroorganismen angelegt sondern ohne diesen Umweg eventuelle für Alkalische Proteasen codierende Nukleinsäuren aus Bodenproben isoliert. Weil die betreffenden Nukleinsäuren originär nicht einem speziellen Stamm, das heißt nicht einem speziellen Genom zugeordnet werden können, handelt es sich dabei um sogenannte Metagenom-DNA.

Überraschenderweise konnten nach diesem Verfahren zwei neue Proteasen gefunden werden, die zueinander eine außerordentliche Ähnlichkeit aufweisen und als vollständige mature Enzyme oder als C-terminale Deletionsmutanten erfolgreich in Wasch- und Reinigungsmitteln einsetzbar sind.

Die gestellte Aufgabe wird somit durch eine Alkalische Protease gelöst, deren Aminosäuresequenz zu der in SEQ ID NO. 4 angebenen Aminosäuresequenz mindestens zu 90% oder zu der in SEQ ID NO. 7 angebenen Aminosäuresequenz mindestens zu 87,5% identisch ist.

Hiermit sind als weitere Erfindungsgegenstände die zugehörigen Nukleinsäuren, entsprechende natürliche Zellen, geeignete Verfahren zu ihrer Identifizierung, insbesondere auf den Nukleinsäuren aufbauende molekularbiologische Verfahren und Verfahrenselemente sowie Mittel, Wasch- und Reinigungsmittel, Wasch- und Reinigungsverfahren und über die betreffenden Proteasen gekennzeichnete Verwendungsmöglichkeiten verbunden.

Wie die Ausführungsbeispiele belegen, weisen bereits die in SEQ ID NO. 4 und 7 angegebenen Enzyme beziehungsweise die zugehörigen maturen Enzyme für Wasch- und Reinigungsmittel brauchbare proteolytische Aktivitäten auf. Aufgrund der zur Verfügung gestellten DNA sind zusätzliche Optimierungen dieser Enzyme, beispielsweise über weitere Punktmutationen möglich. Ferner können diese DNA in Shuffling-Ansätze eingebracht und damit zur Erzeugung völlig neuartiger Proteasen genutzt werden.

Unter einem Protein ist im Sinne der vorliegenden Anmeldung ein aus den natürlichen Aminosäuren zusammengesetztes, weitgehend linear aufgebautes, zur Ausübung seiner Funktion zumeist dreidimensionale Struktur annehmendes Polymer zu verstehen. In der vorliegenden Anmeldung werden die 19 proteinogenen, natürlich vorkommenden L-Aminosäuren mit den international gebräuchlichen 1- und 3-Buchstaben-Codes bezeichnet. Die Kombination einer dieser Bezeichnungen mit einer Nummer bezeichnet für das jeweilige Protein, welchen Aminosäure-Rest es in der jeweiligen Position trägt. Für Punktmutationen sind analoge Bezeichnungen etabliert. Positionsangaben beziehen sich, soweit nicht anders angegeben, auf die jeweils maturen Formen der betreffenden Proteine, also ohne die Signalpeptide (siehe unten).

Unter einem Enzym ist im Sinne der vorliegenden Anmeldung ein Protein zu verstehen, das eine bestimmte biochemische Funktion ausübt. Beispielsweise unter proteolytischen Enzymen oder Enzymen mit proteolytischer Funktion sind im allgemeinen solche zu verstehen, die die Säureamidbindungen von Proteinen hydrolysieren.

Zahlreiche Proteine werden als sogenannte Präproteine, also zusammen mit einem Signalpeptid gebildet. Darunter ist dann der N-terminale Teil des Proteins zu verstehen, dessen Funktion zumeist darin besteht, die Ausschleusung des gebildeten Proteins aus der produzierenden Zelle in das Periplasma oder das umgebende Medium und/oder dessen korrekte Faltung zu gewährleisten. Anschließend wird das Signalpeptid unter natürlichen Bedigungen durch eine Signalpeptidase vom übrigen Protein abgespalten, so daß dieses seine eigentliche katalytische Aktivität ohne die zunächst vorhandenen N-terminalen Aminosäuren ausübt.

Für technische Anwendungen sind aufgrund ihrer enzymatischen Aktivität die maturen Peptide, das heißt die nach ihrer Herstellung prozessierten Enzyme gegenüber den Präproteinen bevorzugt.

Pro-Proteine sind inaktive Vorstufen von Proteinen. Deren Vorläufer mit Signalsequenz werden als Prä-Pro-Proteine bezeichnet.

Unter Nukleinsäuren sind im Sinne der vorliegenden Anmeldung die natürlicherweise aus Nukleotiden aufgebauten als Informationsträger dienenden Moleküle zu verstehen, die für die lineare Aminosäureabfolge in Proteinen oder Enzymen codieren. Sie können als Einzelstrang, als ein zu diesem Einzelstrang komplementärer Einzelstrang oder als Doppelstrang vorliegen. Als der natürlicherweise dauerhaftere Informationsträger ist die Nukleinsäure DNA für molekularbiologische Arbeiten bevorzugt. Demgegenüber wird für die Realisierung der Erfindung in natürlicher Umgebung, wie beispielsweise in einer exprimierenden Zelle, eine RNA gebildet, weshalb erfindungswesentliche RNA-Moleküle ebenfalls Ausführungsformen der vorliegenden Erfindung darstellen. Aus ihnen können beispielsweise über reverse Transkription wiederum (c-)DNA-Moleküle abgeleitet werden.

Die einem Protein entsprechende Informationseinheit einer Nukleinsäure wird auch im Sinne der vorliegenden Anmeldung als Gen bezeichnet. Bei DNA sind die Sequenzen beider komplementärer Stränge in jeweils allen drei möglichen Leserastern zu berücksichtigen. Ferner ist zu berücksichtigen, daß verschiedene Codon-Triplets für dieselben Aminosäuren codieren können, so daß eine bestimmte Aminosäure-Abfolge von mehreren unterschiedlichen und möglicherweise nur geringe Identität aufweisenden Nukleotidsequenzen abgeleitet werden kann (Degeneriertheit des genetischen Codes). Außerdem weisen verschiedene Organismen Unterschiede im Gebrauch dieser Codons auf. Aus diesen Gründen müssen sowohl Aminosäuresequenzen als auch Nukleotidsequenzen in die Betrachtung des Schutzbereichs einbezogen und angegebene Nukleotidsequenzen jeweils nur als eine beispielhafte Codierung für eine bestimmte Aminosäurefolge angesehen werden.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen vollständige Gene herzustellen. Derartige Methoden sind beispielsweise aus dem "Lexikon der Biochemie", Spektrum Akademischer Verlag, Berlin, 1999, Band 1, S. 267-271 und Band 2, S. 227-229, bekannt. Dies ist insbesondere dann möglich, wenn auf einen bei einer Stammsammlung hinterlegten Stamm zurückgegriffen werden kann. Beispielsweise mit PCR-Primern, die anhand einer bekannten Sequenz synthetisierbar sind, und/oder über isolierte mRNA-Moleküle können aus solchen Stämmen die betreffenden Gene synthetisiert, kloniert und gewünschtenfalls weiter bearbeitet, beispielsweise mutagenisiert werden.

Änderungen der Nukleotidsequenz, wie sie beispielsweise durch an sich bekannte molekularbiologische Methoden herbeigeführt werden können, werden als Mutationen bezeichnet. Je nach Art der Änderung kennt man beispielsweise Deletions-, Insertions- oder Substitutionsmutationen oder solche, bei denen verschiedene Gene oder Teile von Genen miteinander fusioniert (shuffling) werden; dies sind Genmutationen. Die zugehörigen Organismen werden als Mutanten bezeichnet. Die von mutierten Nukleinsäuren abgeleiteten Proteine werden als Varianten bezeichnet. So führen beispielsweise Deletions-, Insertions-, Substitutionsmutationen oder Fusionen zu deletions-, insertions-, substitutionsmutierten oder Fusionsgenen und auf Proteinebene zu entsprechenden Deletions-, Insertions- oder Substitutionsvarianten, beziehungsweise Fusionsproteinen.

Für die Beschreibung von Punktmutationen, die genau eine Aminosäureposition betreffen (Aminosäureaustausche), wird folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt.

Unter Vektoren werden im Sinne der vorliegenden Erfindung aus Nukleinsäuren bestehende Elemente verstanden, die als kennzeichnenden Nukleinsäurebereich ein interessierendes Gen enthalten. Sie vermögen dieses in einer Spezies oder einer Zellinie über mehrere Generationen oder Zellteilungen hinweg als vom übrigen Genom unabhängig replizierendes, stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den sogenannten Klonierungsvektoren, und andererseits denen, die die Funktion erfülllen, das interessierende Gen in der Wirtszelle zu realisieren, das heißt, die Expression des betreffenden Proteins zu ermöglichen. Diese Vektoren werden als Expressionsvektoren bezeichnet.

Sowohl Bakterienzellen als auch eukaryontische Zellen, die die genannten Vektoren enthalten, werden ungeachtet ihrer Unterschiede allgemein als Zellen bezeichnet. Solche Zellen, die einen Vektor, insbesondere einen Expressionsvektor enthalten und somit zur Expression eines Transgens angeregt werden können, werden als Wirtszellen bezeichnet, denn sie beherbergen das betreffende genetische System.

Homologisierung ist der Vergleich einer Nukleinsäure- oder Aminosäuresequenz mit der von bekannten Genen oder Proteinen. Sie wird beispielsweise über ein Alignment vorgenommen. Das Maß für die Homologie ist ein Prozentsatz an Identität, wie er beispielsweise nach der von D. J. Lipman und W. R. Pearson in Science, Band 227 (1985), Seite 1435-1441 angegebenen Methode bestimmt werden kann. Vorzugsweise geschieht dies über Algorithmen, welche inzwischen von kommerziell erhältlichen Computergrogrammen angewendet werden. Hierzu gehört beispielsweise das Programm Vector NTI^{®} Suite 7.0, erhältlich von der Firma InforMax, Inc., Bethesda, USA, vorzugsweise mit den vorgegebenen Default-Parametern. Die Homologie-Angabe kann sich auf das gesamte Protein oder auf den jeweils zuzuordnenden Bereich beziehen. Ein weiter gefaßter Homologie-Begriff, die Ähnlichkeit, bezieht auch konservierte Variationen, also Aminosäuren mit ähnlicher chemischer Aktivität in die Betrachtung mit ein, da diese innerhalb des Proteins meist ähnliche chemische Aktivitäten ausüben. Bei Nukleinsäuren kennt man nur den Prozentsatz an Identität.

Durch Homologisierung lassen sich aus der Aminosäure- oder Nukleotid-Sequenz die Funktionen einzelner Sequenzbereiche sowie die enzymatische Aktivität des betrachteten gesamten Enzyms folgern. Homologe Bereiche von verschiedenen Proteinen sind solche mit vergleichbaren Funktionen, die sich durch Identität oder konservierte Austausche in der primären Aminosäuresequenz erkennen lassen. Sie umfassen einzelne Aminosäuren, kleinste Bereiche, sogenannte Boxen, die wenige Aminosäuren lang sind, bis hin zu langen Bereichen in der primären Aminosäuresequenz. Unter den Funktionen der homologen Bereiche sind somit auch kleinste Teilfunktionen der vom gesamten Protein ausgeübten Funktion zu verstehen, wie beispielsweise die Ausbildung einzelner Wasserstoffbrückenbindungen zur Komplexierung eines Substrats oder Übergangskomplexes. Andere Bereiche des Proteins, die nicht an der eigentlichen enzymatischen Reaktion beteiligt sind, können sie qualitativ oder quantitativ modifizieren. Dies betrifft beispielsweise die Enzymstabilität, die Aktivität, die Reaktionsbedingungen oder die Substratspezifität.

Unter dem Begriff eines proteolytischen Enzyms oder dem einer Protease sind deshalb über die Funktionen der wenigen Aminosäurereste des katalytisch aktiven Zentrums hinaus alle Funktionen zu verstehen, wie sie sich durch das Einwirken des gesamten übrigen Proteins oder eines Teils oder mehrerer Teile des übrigen Proteins auf die eigentlich katalytisch aktiven Bereiche ergeben. Es ist darüberhinaus möglich, daß auch die Aktivitäten anderer Proteasen durch einen oder mehrere Teile, beispielsweise des erfindungsgemäßen Proteins qualitativ oder quantitativ modifiziert werden. Diese Beeinflussung anderer Faktoren wird ebenfalls als proteolytische Aktivität angesehen. Proteolytisch aktive Enzyme sind auch solche Proteasen, deren Aktivität zu einem gegebenen Zeitpunkt, etwa durch einen Inhibitor blockiert ist. Entscheidend ist ihre prinzipielle Eignung zur entsprechenden Proteolyse-Reaktion.

Unter Fragmenten werden alle Proteine oder Peptide verstanden, die kleiner sind als natürliche Proteine oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Unter chimären oder hybriden Proteinen sind im Sinne der vorliegenden Anmeldung solche Proteine zu verstehen, die aus Elementen zusammengesetzt sind, die natürlicherweise von verschiedenen Polypeptidketten aus demselben Organismus oder aus verschiedenen Organismen stammen. Dieses Vorgehen wird auch Shuffling oder Fusionsmutagenese genannt. Der Sinn einer solchen Fusion besteht beispielsweise darin, mithilfe des heranfusionierten erfindungsgemäßen Proteinteils eine enzymatische Funktion herbeizuführen oder zu modifizieren.

Unter durch Insertionsmutation erhaltenen Proteinen sind solche Varianten zu verstehen, die über an sich bekannte Methoden durch Einfügen eines Nukleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

Inversionsmutagenese, also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Unter Derivaten werden im Sinne der vorliegenden Anmeldung solche Proteine verstanden, deren reine Aminosäurekette chemisch modifiziert worden ist. Solche Derivatisierungen können beispielsweise biologisch im Zusammenhang mit der Proteinbiosynthese durch den Wirtsorganismus erfolgen. Hierfür können beispielsweise molekularbiologische Methoden, etwa die Cotransformation mit Genen, die für die betreffende Modifikation sorgen, eingesetzt werden. Derivatisierungen können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise auch um andere Proteine handeln, die beispielsweise über bifunktionelle chemische Verbindungen an erfindungsgemäße Proteine gebunden werden. Derartige Modifikationen beeinflussen beispielsweise die Substratspezifität oder die Bindungsstärke an das Substrat oder führen eine vorübergehende Blockierung der enzymatischen Aktivität herbei, wenn es sich bei der angekoppelten Substanz um einen Inhibitor handelt. Dies ist beispielsweise für den Zeitraum der Lagerung sinnvoll. Ebenso ist unter Derivatisierung die kovalente Bindung an einen makromolekularen Träger zu verstehen.

Im Sinne der vorliegenden Erfindung werden alle Enzyme, Proteine, Fragmente, Fusionsproteine und Derivate, sofern sie nicht explizit als solche angesprochen zu werden brauchen, unter dem Oberbegriff Proteine zusammengefaßt.

Unter der Leistung eines Enzyms wird dessen Wirksamkeit im jeweils betrachteten technischen Bereich, vorzugsweise im Rahmen eines entsprechend ausgerichteten Mittels verstanden. Diese basiert auf der eigentlichen enzymatischen Aktivität, hängt darüberhinaus aber von weiteren, für den jeweiligen Prozeß relevanten Faktoren ab. Dazu gehören beispielsweise Stabilität, Substratbindung, Wechselwirkung mit dem das Substrat tragenden Material oder Wechselwirkungen mit anderen Inhaltsstoffen, insbesondere Synergien.

Unter der Waschleistung oder der Reinigungsleistung eines Wasch-, beziehungsweise Reinigungsmittels ist im Sinne der vorliegenden Anmeldung der Effekt zu verstehen, den das betrachtete Mittel auf die verschmutzten Artikel, beispielsweise Textilien oder Gegenstände mit harten Oberflächen ausübt. Einzelne Komponenten solcher Mittel, beispielsweise einzelne Enzyme, werden hinsichtlich ihres Beitrags zur Wasch- oder Reinigungsleistung des gesamten Wasch-, beziehungsweise Reinigungsmittels beurteilt. Denn aus den enzymatischen Eigenschaften eines Enzyms kann nicht ohne weiteres auf seinen Beitrag zur Waschleistung eines Mittels geschlossen werden. Hier spielen als weitere Faktoren beispielsweise Stabilität, Substratbindung, Bindung an das Reinigungsgut oder Wechselwirkungen mit anderen Inhaltsstoffen der Wasch- oder Reinigungsmittel, insbesondere Synergien bei der Entfernung der Verschmutzungen eine Rolle.

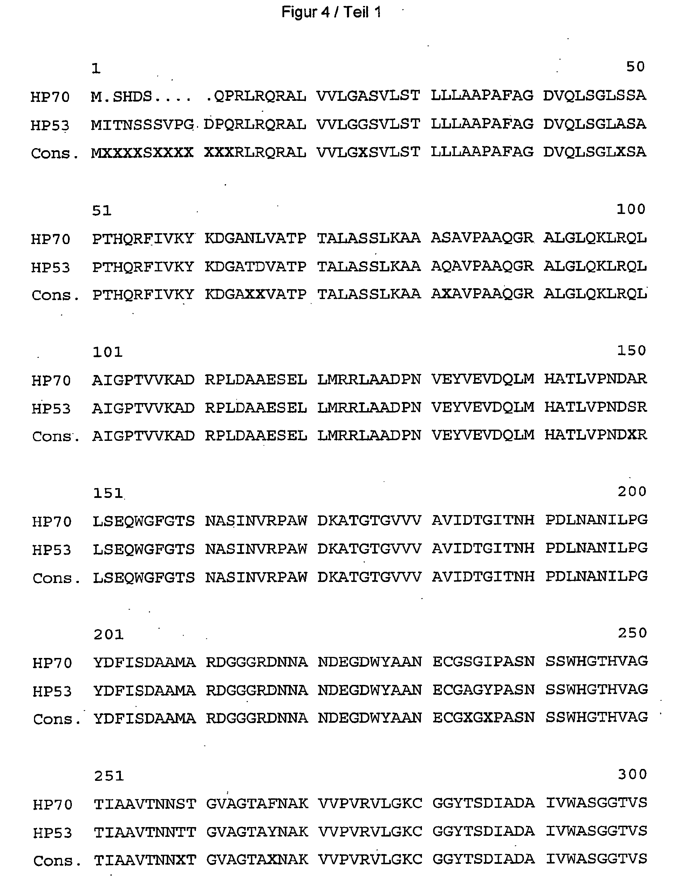

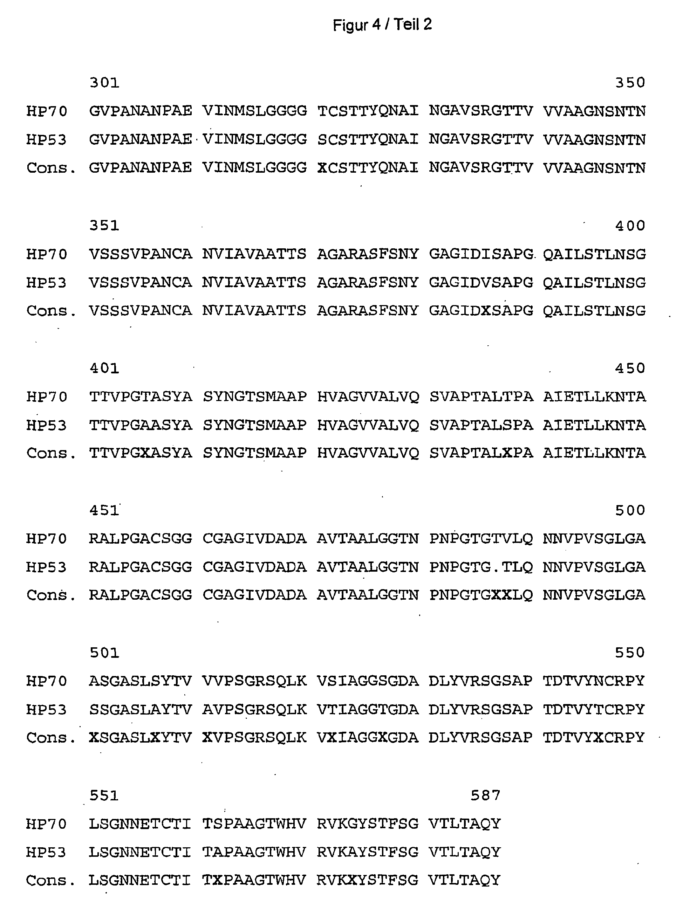

Die in SEQ ID NO. 4 und 7 angegebenen Aminosäuresequenzen sind, wie in den Beispielen zur vorliegenden Anmeldung beschrieben, von Nukleinsäuren abgeleitet worden, die aus Bodenproben isoliert worden sind. Deren Sequenzen sind unter SEQ ID NO. 3 beziehungsweise 6 angegeben. Die abgeleiteten Proteine werden erfindungsgemäß als Protease HP70 (für SEQ ID NO. 3 und 4) beziehungsweise HP53 (für SEQ ID NO. 6 und 7) bezeichnet. Wie anhand eines Alignments, beispielsweise über Figur 4 nachvollzogen werden kann, weisen sie zueinander auf Aminosäureebene eine Homologie von 93,9% Identität auf.

Als zu diesen beiden gefundenen und gemäß den Beispielen der vorliegenden Anmeldung charakterisierten Proteasen nächstähnliches beschriebenes Enzym wurde eine extrazelluläre Serin-Protease (E.C. 3.4.21.-) aus *Xanthomonas campestris pv. campestris* (ATCC 33913) ermittelt, die bei GenBank (National Center for Biotechnology Information NCBI, National Institutes of Health, Bethesda, MD, USA) die Zugangsnummer NP_636242 trägt. Die wie alle nachfolgenden Homologiewerte über das Computer-Programm Vector NTI^{®} Suite 7.0, erhältlich von der Firma Informax, Inc., Bethesda, USA, mit den vorgegebenen Default-Parametern ermittelte Homologie dieses Enzyms beträgt auf Aminosäureebene zu HP70 75,0% Identität und zu HP53 75,4% Identität.

Die nächstähnlichen, ermittelten Enzyme sind in den Beispielen 4 und 5 in Tabellenform zusammengestellt; es handelt sich dabei jeweils um extrazelluläre Proteasen aus *Xanthomonas campestris* und *X axonopodis.* Zu der etablierten *B*. *lentus*-Alkalischen Protease (WO 92/21760 A1) ergibt sich über die Gesamtlänge der Alkalischen Protease HP70 auf Aminosäureebene eine Homologie von 26,2% Identität und auf Nukleotidebene von 33,6%. HP53 weist zu der B. lentus-Alkalischen Protease Homologiewerte von 25,9% beziehungsweise 33,5% Identität auf.

Diese nach den Beispielen erarbeiteten Angaben können folgendermaßen aktualisiert werden: Die Protease StmPr2 aus *St*. *maltophilia,* (GenBank: AY253983) weist zu der erfindungsgemäßen Protease HP70 (SEQ ID NO. 4) eine Sequenzhomologie von 84,7 und zu HP53 (SEQ ID NO. 7) von 82,5% Identität auf. Die unter SEQ ID NO. 66 in WO 2004/033668 A2 offenbarte Protease ist zu HP70 zu 83,1 % und zu HP53 zu 81,1 % identisch. Im Vergleich zu der unter SEQ ID NO. 70 in WO 2004/033668 A2 offenbarten Protease ergeben sich Homologiewerte von 85,0% Identität zu HP70 und von 82,3% Identität zu HP53.

Damit können alle Alkalischen Proteasen in den Schutzbereich der vorliegenden Anmeldung eingeschlossen werden, deren Aminosäuresequenzen zu SEQ ID NO. 4 mindestens zu 90% oder zu SEQ ID NO. 7 mindestens zu 87,5% identisch sind.

Darunter sind funktionelle Alkalische Proteasen bevorzugt, das heißt keine defekten oder lediglich putativen Enzyme sondern solche, die tatsächlich aufgrund dieser enzymatischen Aktivität für eine technische Anwendnung genutzt werden können.

Zunehmend bevorzugt sind alle derartigen Alkalischen Proteasen, deren Aminosäuresequenz zu der in SEQ ID NO. 4 angegebenen Aminosäuresequenz zu mindestens 95% und zunehmend bevorzugt zu mindestens 96%, 97%, 98%, 99% und ganz besonders bevorzugt zu 100% oder zu der in SEQ ID NO. 7 angegebenen Aminosäuresequenz zu mindestens 90% und zunehmend bevorzugt zu mindestens 91%, 92%, 93%, 94%, 95%, 96%, 97%, 98%, 99% und ganz besonders bevorzugt zu 100% identisch sind, wobei jeweils alle ganzzahligen oder gebrochenen Zwischenwerte entsprechend eingeschlossen werden.

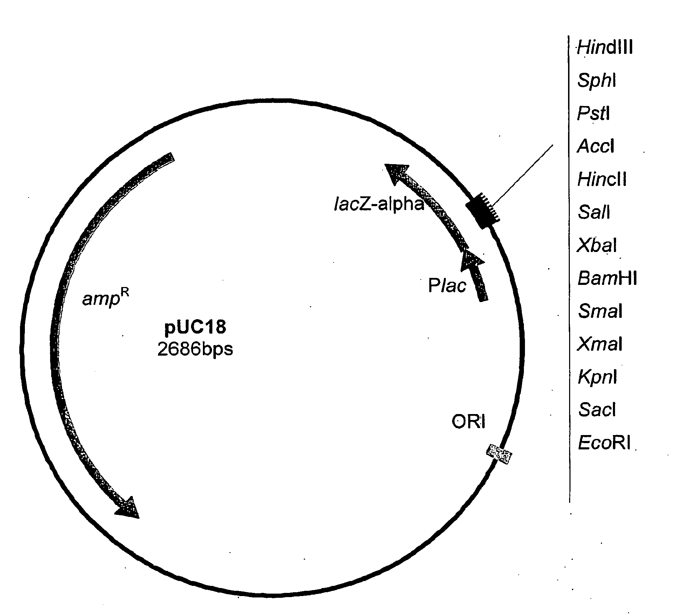

Die zugehörigen in den Beispielen beschriebenen, für die zu 100% identischen Proteine codierenden Vektoren, die sich von dem in Figur 3 dargestellten Vektor ableiten, erhielten die Bezeichnungen 70-pUC(AWB403) für HP70 und 53-pUC(AWB403) für HP53. Sie wurden unter diesen Namen am 2.10.2003 bei der Deutschen Sammlung von Mikroorganismen und Zellkulturen GmbH, Mascheroder Weg 1b, 38124 Braunschweig (http://www.dsmz.de) hinterlegt und tragen dort die Hinterlegungsnummern DSM 15977 beziehungsweise DSM 15976. Die jeweilige Lebensfähigkeit wurde von der DSMZ am 17.10.2003 bestätigt. Die von diesen Vektoren codierten, in den Beispielen der vorliegenden Anmeldung untersuchten und deshalb am stärksten bevorzugten Proteasen werden als HP70 beziehungsweise HP53 bezeichnet.

Weiter bevorzugt sind solche erfindungsgemäßen Alkalischen Proteasen, bei denen die Homologiewerte jeweils für den Bereich gelten, der den Aminosäurepositionen 33 bis 581 gemäß SEQ ID NO. 4 oder 39 bis 586 gemäß SEQ ID NO. 7 entspricht.

Hiermit ist jeweils das tatsächlich aktive mature Protein gemeint, weil dieses die technisch relevante Funktion ausübt. Zum gegenwärtigen Zeitpunkt kann noch nicht zweifelsfrei gesagt werden, welche Aminosäure jeweils den N-Terminus des maturen Proteins darstellt. Derzeit erscheint der Beginn in den Positionen 33 beziehungsweise 39 lediglich am wahrscheinlichsten. Sollte sich zu einem späteren Zeitpunkt herausstellen, daß andere Aminosäuren den jeweiligen N-Terminus darstellen, so wird der beanspruchte Schutzbereich darauf bezogen, wobei die genannte Position jeweils die erste Aminosäure des maturen Proteins bezeichnet.

Ein wie in Beispiel 4 durchgeführter Sequenzvergleich dieser maturen Enzyme mit den nächstähnlichen aus dem Stand der Technik hat folgendes Ergebnis geliefert: die (vermutlich) mature Protease HP70 (SEQ ID NO. 4, Positionen 33 bis 581) ist zu dem homologen Bereich von SEQ ID NO. 66 aus WO 2004/033668 A2 zu 84,2% identisch, zu dem von SEQ ID NO. 70 aus WO 2004/033668 A2 zu 86,2% und zu dem der Protease STmPr2 zu 85,8%. Die (vermutlich) mature Protease HP53 (SEQ ID NO. 7, Positionen 39 bis 586) ist zu dem homologen Bereich von SEQ ID NO. 66 aus WO 2004/033668 A2 zu 83,8% identisch, zu dem von SEQ ID NO. 70 aus WO 2004/033668 A2 zu 85,0% und zu dem der Protease STmPr2 zu 85,2%.

Ähnliches gilt für den C-Terminus. Derzeit erscheinen hierfür die Positionen 581 beziehungsweise 586 gemäß SEQ ID NO. 4 beziehungsweise 7 plausibel, weil die Nukleotidpositionen 1744 bis 1746 gemäß SEQ ID NO. 3 und die Positionen 1759 bis 1761 gemäß SEQ ID NO. 6 jeweils ein Stop-Codon darstellen. Sollte sich zu einem späteren Zeitpunkt jedoch herausstellen, daß durch Prozessierung eine andere Aminosäure den C-Terminus des maturen, aktiven Proteins darstellt, so wird der beanspruchte Schutzbereich darauf bezogen, wobei die angegebenen Nummern jeweils die letzte Aminosäure des maturen, aktiven Proteins bezeichnen. Prinzipiell das gleiche gilt für den Fall, daß bei der Reifung des Proteins eventuell interne Fragmente herausgeschnitten werden. Besonders bevorzugt ist jeweils die Aminosäuresequenz des maturen, aktiven Proteins.

Weiterhin bevorzugt ist jede der bislang beschriebenen Alkalischen Proteasen, bei denen die Homologiewerte ab mindestens 90% Identität jeweils für den Bereich gelten, der den Aminosäurepositionen 33 bis 470 gemäß SEQ ID NO. 5 oder 33 bis 470 gemäß SEQ ID NO. 8 entspricht.

Denn wie in den Beispielen beschrieben ist, konnten sowohl von HP70 als auch von HP53 die darin genannten und hiermit aus dem bevorzugten Schutzbreich ausgeschlossenen C-terminalen Bereiche deletiert werden, ohne daß die Deletionsvarianten ihre Proteaseaktivität, insbesondere die während des Wasch- oder Reinigungsvorgangs benötigte proteolytische Aktivität verloren. Der Vorteil in dieser drastischen Deletion besteht in der Ersparnis von Aufwand und Kosten bei der biotechnologischen Herstellung der betreffenden Proteine. Denn damit werden in kürzerer Zeit mehr erfindungsgemäß besonders für den Einsatz in Wasch- und Reinigungsmitteln verwendbare Enzyme erhalten, womit beispielsweise auch eine bessere Ausnutzung der für die Fermentation der produzierenden Mikroorganismen notwendigen Medienbestandteile einhergeht.

Ein wie in Beispiel 4 durchgeführter Sequenzvergleich dieser maturen und C-terminal deletierten Enzyme mit den nächstähnlichen aus dem Stand der Technik hat folgendes Ergebnis geliefert: die (vermutlich) mature und C-terminal deletierte Protease HP70 (SEQ ID NO. 5, Positionen 33 bis 470) ist zu dem homolgen Bereich von SEQ ID NO. 66 aus WO 2004/033668 A2 zu 85,2% identisch, zu dem von SEQ ID NO. 70 aus WO 2004/033668 A2 zu 88, 1 % und zu dem der Protease STmPr2 zu 87,7%. Die (vermutlich) mature und C-terminal deletierte Protease HP53 (SEQ ID NO. 8, Positionen 33 bis 470) ist zu dem homologen Bereich von SEQ ID NO. 66 aus WO 2004/033668 A2 zu 85,4% identisch, zu dem von SEQ ID NO. 70 aus WO 2004/033668 A2 zu 87,0% und zu dem der Protease STmPr2 ebenfalls zu 87,0%.

Weiterhin bevorzugt ist jede der bislang beschriebenen Alkalischen Proteasen mit einer Aminosäuresequenz gemäß der Consensus-Sequenz von SEQ ID NO. 9, vorzugsweise im Bereich der Aminosäurepositionen 39 bis 587, besonders bevorzugt im Bereich der Aminosäurepositionen 39 bis 476.

SEQ ID NO. 9 stellt die aus den beiden Aminosäuresequenzen SEQ ID NO. 4 und 7 erhältliche Consensussequenz dar, wie sie beispielsweise über das Alignment der Figur 4 aufgestellt werden kann. Sie umfaßt diejenigen Proteasen, deren Aminosäuresequenzen in jeder ihrer Position entweder auf SEQ ID NO. 4 oder SEQ ID NO. 7 zurückgeführt werden können. Diese beiden Sequenzen eröffnen somit einen Sequenzraum an miteinander verwandten oder ähnlichen Subtilisin-Proteasen. Sie besitzen die in SEQ ID NO. 9 angegebene allgemeine Sequenz, wobei in folgenden 35 Positionen jeweils zwei verschiedene Aminosäuren vorliegen können beziehungsweise eine bestimmte (angegeben in Dreibuchstabencode) oder keine (-); und zwar sind das folgende Möglichkeiten (in Sequenzprotokoll jeweils als "Variante" definiert): Position 2: - oder lle, Position 3: Ser oder Thr, Position 4: His oder Asn, Position 5: Asp oder Ser, Position 7: - oder Ser, Position 8: - oder Val, Position 9: - oder Pro, Position 10: - oder Gly, Position 11: - oder Asp, Position 12: Gln oder Pro, Position 13: Pro oder Gln, Position 25: Ala oder Gly, Position 48: Ser oder Ala, Position 65: Asn oder Thr, Position 66: Leu oder Asp, Position 82: Ser oder Gin, Position 149: Ala oder Ser, Position 234: Ser oder Ala, Position 236: Ile oder Tyr, Position 259: Ser oder Thr, Position 267: Phe oder Tyr, Position 321: Thr oder Ser, Position 386: Ile oder Val, Position 406: Thr oder Ala, Position 438: Thr oder Ser, Position 487: Thr oder -, Position 488: Val oder Thr, Position 501: Ala oder Ser, Position 507: Ser oder Ala, Position 511: Val oder Ala, Position 522: Ser oder Thr, Position 527: Ser oder Thr, Position 546: Asn oder Thr, Position 562: Ser oder Ala und, schließlich, Position 574: Gly oder Ala.

Da die beiden Enzyme HP70 und HP53 in den mit den vorliegenden Beispielen dokumentierten Untersuchungen erfindungsgemäße Vorteile aufweisen und zudem zu 93,9% übereinstimmen, ist zu erwarten, daß jedes weitere Enzym, das dieser Protease-Unterfamilie angehört, über vergleichbar günstige Eigenschaften verfügt.

Das gilt entsprechend dem oben Gesagten insbesondere für die Teile des jeweils maturen, das heißt aktiven Enzyms und ganz besonders für diejenigen DeletionsVarianten, bei denen ohne nennenswerten Verlust der Proteaseaktivität große Teile des C-Terminus entfernt sind.

Weiterhin bevorzugt ist jede der bislang beschriebenen Alkalischen Proteasen, die von einer Nukleotidsequenz codiert wird, die zu der in SEQ ID NO. 3 angegebenen Nukleotidsequenz mindestens zu 85% und zunehmend bevorzugt zu mindestens 90%, 95%, 96%, 97%, 98%, 99% und ganz besonders bevorzugt zu 100% identisch ist, insbesondere für den Bereich, der den Nukleotidpositionen 97 bis 1746 gemäß SEQ ID NO. 3 entspricht, ganz besonders für den Bereich, der den Nukleotidpositionen 97 bis 1410 gemäß SEQ ID NO. 3 entspricht, oder die von einer Nukleotidsequenz codiert wird, die zu der in SEQ ID NO. 6 angegebenen Nukleotidsequenz mindestens zu 85% und zunehmend bevorzugt zu mindestens 90%, 95%, 96%, 97%, 98%, 99% und ganz besonders bevorzugt zu 100% identisch ist, insbesondere für den Bereich, der den Nukleotidpositionen 115 bis 1761 gemäß SEQ ID NO. 6 entspricht, ganz besonders für den Bereich, der den Nukleotidpositionen 115 bis 1428 gemäß SEQ ID NO. 6 entspricht, wobei jeweils alle ganzzahligen oder gebrochenen Zwischenwerte entsprechend eingeschlossen werden.

Denn wie aus dem oben Gesagten und insbesondere den Beispielen hervorgeht, sind die besonders bevorzugten Proteasen nicht an sich oder über einen entsprechenden Mikroorganismus detektiert worden sondern werden von in Zusammenhang mit der vorliegenden Erfindung entdeckten Nukleinsäuren codiert.

Wie in den Beispielen 3 und 4 erläutert ist, weist das zu HP70 und HP53 auf Nukleotidebene nächstähnliche Enzym, eine extrazelluläre Serin-Protease (E.C. 3.4.21.-) aus *Xanthomonas campestris pv. campestris* (ATCC 33913; NP_636242), wie über das Computer-Programm Vector NTI^{®} Suite 7.0, erhältlich von der Firma InforMax, Inc., Bethesda, USA, mit den vorgegebenen Default-Parametern ermittelt werden kann, auf Nukleotidebene eine Homologie von 74,4 beziehungsweise 75,0% Identität auf. Dementsprechend werden alle Alkalischen Proteasen und Proteine, die von signifikant ähnlicheren Nukleinsäuren codiert werden, in den Schutzbereich eingeschlossen.

Diese nach den Beispielen erarbeiteten Angaben können folgendermaßen aktualisiert werden: Die Protease StmPr2 aus *St. maltophilia,* (GenBank: AY253983) weist auf DNA-Ebene zu der erfindungsgemäßen Protease HP70 (SEQ ID NO. 3) in dem homologisierbaren Bereich eine Sequenzhomologie von 80,8 und zu der DNA-Sequenz von HP53 (SEQ ID NO. 6) von 81,2% Identität auf. Die unter SEQ ID NO. 65 in WO 2004/033668 A2 offenbarte Protease-DNA-Sequenz ist zu der von HP70 zu 79,6% und zu der von HP53 zu 79,9% identisch. Im Vergleich zu der unter SEQ ID NO. 69 in WO 2004/033668 A2 offenbarten Protease-codierenden DNA-Sequenz ergeben sich Homologiewerte von 81,3% Identität zu der HP70-DNA und von 81,1 % Identität zu der HP53-DNA.

Die bisherigen Ausführungen gelten entsprechend besonders für die Nukleinsäuresequenzen, die für die maturen Proteine und ganz beonders für die C-terminal deletierten, proteolytisch aktiven Varianten davon codieren. Denn das sind die Enzyme, deren proteolytische Aktivitäten und insbesondere deren Beiträge zur Waschbeziehungsweise Reinigungsleistung entsprechender Rezepturen in den Beispielen der vorliegenden Anmeldung belegt sind.

Ein wie in Beispiel 4 durchgeführter Sequenzvergleich dieser für die maturen Enzyme codierenden DNA-Abschnitte mit den nächstähnlichen aus dem Stand der Technik hat folgendes Ergebnis geliefert: das Gen für die (vermutlich) mature Protease HP70 (SEQ ID NO. 3, Positionen 97 bis 1746) ist zu dem homologen Bereich von SEQ ID NO. 65 aus WO 2004/033668 A2 zu 80,0% identisch, zu dem von SEQ ID NO. 69 aus WO 2004/033668 A2 zu 81,8% und zu dem der Protease STmPr2 zu 81,3%. Das Gen für die (vermutlich) mature Protease HP53 (SEQ ID NO. 6, Positionen 115 bis 1761) ist zu dem homologen Bereich von SEQ ID NO. 65 aus WO 2004/033668 A2 zu 81,0% identisch, zu dem von SEQ ID NO. 69 aus WO 2004/033668 A2 zu 82,2% und zu dem der Protease STmPr2 zu 82,9%.

Die hiervon abgeleiteten Alkalischen Proteasen sind dementsprechend bevorzugt.

Ein weiterer wie in Beispiel 4 durchgeführter Sequenzvergleich der für diese maturen und C-terminal deletierten Enzyme codierenden DNA-Abschnitte mit den nächstähnlichen aus dem Stand der Technik hat folgendes Ergebnis geliefert: das Gen für die (vermutlich) mature und C-terminal deletierte Protease HP70 (SEQ ID NO. 3, Positionen 97 bis 1410) ist zu dem homolgen Bereich von SEQ ID NO. 65 aus WO 2004/033668 A2 zu 81,4% identisch, zu dem von SEQ ID NO. 69 aus WO 2004/033668 A2 zu 83,7% und zu dem der Protease STmPr2 zu 83,2%. Die für die (vermutlich) mature und C-terminal deletierte Protease HP53 codierende Nukleinsäure (SEQ ID NO. 6, Positionen 115 bis 1428) ist zu dem homologen Bereich von SEQ ID NO. 65 aus WO 2004/033668 A2 zu 82,1% identisch, zu dem von SEQ ID NO. 69 aus WO 2004/033668 A2 zu 83,6% und zu dem der Protease STmPr2 zu 83,9%.

Die von diesen DNA-Abschnitten abgeleiteten Alkalischen Proteasen sind dementsprechend besonders bevorzugt.

Weiterhin bevorzugt ist jede der bislang beschriebenen erfindungsgemäßen Alkalischen Proteasen, die aus einem natürlichen Habitat isolierbar ist oder die sich von einer aus einem natürlichen Habitat isolierbaren Nukleinsäure ableitet.

Denn es ist anzunehmen, daß die mit dem in den Beispielen beschriebenen Verfahren isolierte DNA von natürlichen Organismen gebildet worden sind und auch *in vivo* für funktionelle Proteine codieren. Somit müssen über analoge Verfahren auch die zugehörigen Enzyme selbst gefunden werden können, insbesondere dann, wenn es sich nicht um Pseudogene sondern um tatsächlich gebildete Proteine handelt. Demgegenüber führt die Isolierung der Nukleinsäuren unmittelbar zu einem Gen, das in molekularbiologische Charakterisierungen eingebracht und produziert werden kann. Zudem kann nicht immer erwartet werden, daß die betreffenden Gene unter allen Bedingungen exprimiert werden, so daß über die Nukleinsäure-Isolierung auch augenblicklich nicht translatierte Gene zugänglich sind.

Weiterhin bevorzugt ist jede der bislang beschriebenen erfindungsgemäßen Alkalischen Proteasen, die selbst oder deren zugehörige Nukleinsäure aus einem Organismus stammt, der aus einem natürlichen Habitat isolierbar ist.

Diese Ausführungsform ist deshalb besonders vorteilhaft, weil dann der zugehörige Organismus selbst in Kultur genommen werden kann. Vorteilhafterweise lassen sich dann aus dessen Zellextrakten oder Kulturüberständen die erfindungsgemäßen Proteasen isolieren und herstellen.

Hierunter sind jene Alkalischen Proteasen bevorzugt, wobei es sich um einen Mikroorganismus handelt, vorzugsweise um einen Pilz oder ein Bakterium, hierunter bevorzugt um ein grampositives Bakterium und besonders bevorzugt um eines der Gattung *Bacillus.*

Denn besonders für diese Organismen sind im Stand der Technik Kultivierungsmethoden bekannt und etabliert. Das gilt insbesondere für *Bacilli,* die in der technischen Enzymherstellung eine herausragende Rolle einnehmen. Eine weitere Ausführungsform stellen solche Alkalischen Proteasen und Proteine dar, die aus Xanthomonas-Spezies stammen. Denn aus einer dieser gramnegativen Spezies stammen die als nächstähnlich ermittelten, bekannten Enzyme (siehe oben); auch in der biotechnologischen Fermentation von Xanthomonaden hat man bereits Erfahrungen.

Weiterhin bevorzugt sind von einer der bislang beschriebenen erfindungsgemäßen Alkalischen Proteasen durch Fragmentierung oder Deletionsmutagenese abgeleitete Alkalische Proteasen oder Proteine mit mindestens 100 und zunehmend bevorzugt mindestens 150, 200, 250 und ganz besonders bevorzugt mindestens 300 bereits im Ausgangsmolekül zusammenhängenden Aminosäuren.

So ist es beispielsweise möglich, an den Termini oder in den Loops des Enzyms einzelne Aminosäuren zu deletieren, ohne daß dadurch die proteolytische Aktivität verlorengeht. Solche Mutationen werden beispielsweise in WO 99/49057 A1 beschrieben. WO 01/07575 A2 lehrt, daß durch derartige Deletionen die Allergenizität betreffender Proteasen gesenkt und somit insgesamt ihre Einsetzbarkeit verbessert werden kann. Die Fragmentierung kommt dem später ausgeführten Aspekt der Insertions- oder Substitutionsmutagenese und/oder Fusion mit anderen Enzymen zugute. Hinsichtlich des beabsichtigten Einsatzes dieser Enzyme ist es bevorzugt, wenn sie auch nach der Fragmentierung oder Deletionsmutagenese eine proteolytische Aktivität besitzen; besonders bevorzugt ist es, wenn sie eine hierdurch zusätzlich gesteigerte Aktivität aufweisen.

Weiterhin bevorzugt sind Alkalische Proteasen oder Proteine, wie sie bisher als erfindungsgemäß beschrieben worden sind und von einer der bislang beschriebenen Alkalischen Proteasen oder Proteinen durch Insertionsmutagenese, durch Substitutionsmutagenese und/oder durch Fusion mit mindestens einem anderen Protein abgeleitet sind.

Denn zahlreiche Dokumente des Stands der Technik offenbaren vorteilhafte Wirkungen von Insertionen und Substitionen in Proteasen; darunter auch die genannten Publikationen WO 99149057 A1 und WO 01/07575 A2. Prinzipiell gehören hierzu auch Einzelaustausche von Aminosäuren, es können aber auch mehrere zusammenhängende Aminosäuren gegen andere ausgetauscht werden. Hierzu gehören auch Neukombinationen von größeren Enzymabschnitten, so den oben genannten Fragmenten, mit anderen Proteasen oder Proteinen anderer Funktion. So ist es beispielsweise in Anlehnung an WO 99/57254 A1 möglich, ein erfindungsgemäßes Protein oder Teile davon über peptidische Linker oder direkt als Fusionsprotein mit Bindungsdomänen aus anderen Proteinen, etwa der Cellulose-Bindungsdomäne, zu versehen und dadurch die Hydrolyse des Substrats effektiver zu gestalten. Ebenso können erfindungsgemäße Proteine beispielsweise auch mit Amylasen oder Cellulasen verknüpft werden, um eine Doppelfunktion auszuüben.

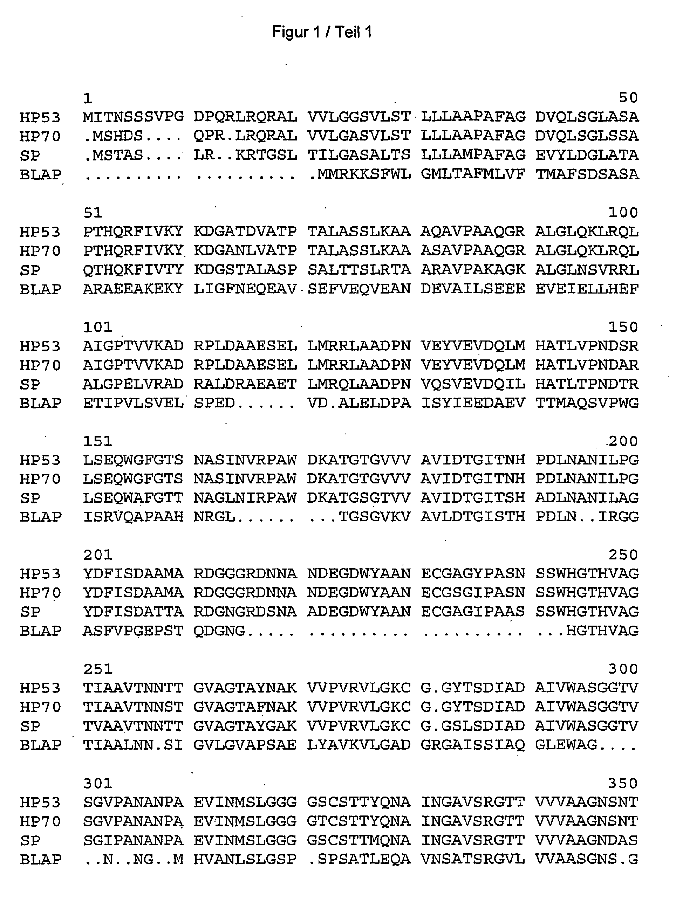

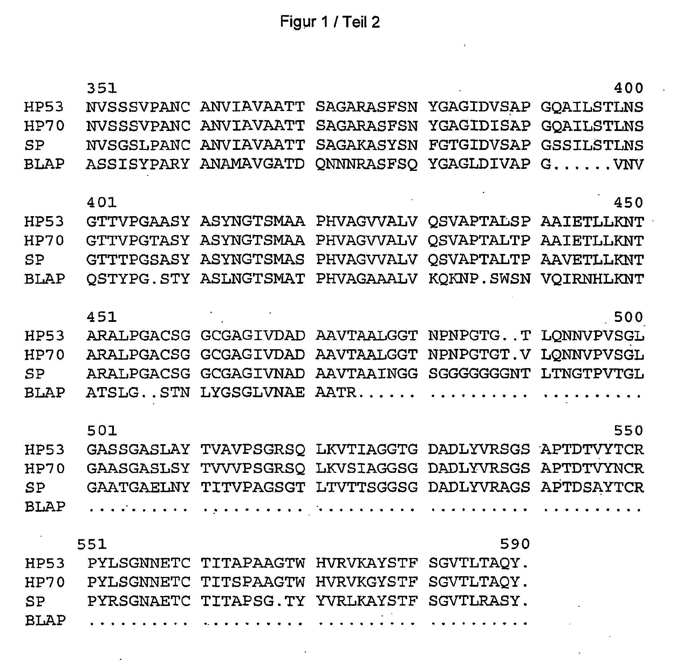

Hierunter sind die Alkalischen Proteasen oder Proteine mit einem oder mehreren Aminosäureaustauschen in den Positionen 3, 4, 36, 42, 47, 56, 61, 69, 87, 96, 99, 101, 102, 104, 114, 118, 120, 130, 139, 141, 142, 154, 157, 188, 193, 199, 205, 211, 224, 229, 236, 237, 242, 243, 255 und 268 in der Zählung der Alkalischen Protease aus *Bacillus lentus,* bevorzugt, wobei diese Positionen über das Alignment in Figur 1 zuzuordnen sind.

Hier liegen in dem Wildtypmolekül der B. lentus-Alkalischen Protease folgende Aminosäurereste: S3, V4, S36, N42, A47, T56, G61, T69, E87, A96, R99, A101, 1102, S104, N114, H118, A120, S130, S139, T141, S142, S154, S157, A188, V193, V199, G205, L211, A224, K229, S236, N237, N242, H243, N255 beziehungsweise T268.

Da neben der Alkalischen Protease aus *Bacillus licheniformis* die B. lentus-Alkalische Protease im Stand der Technik ein wichtiges Referenzmolekül zur Beschreibung neuer Proteasen und von Punktmutationen darstellt, die hier beschriebene neue Protease und somit auch ihre Sequenz bislang unbekannt sind, erscheint es vorteilhaft, in der Zuordnung der Punktmutationen auf diese Zählung Bezug zu nehmen. Zum anderen richtet sich die Zählung im allgemeinen nach dem maturen Protein, und wie oben ausgeführt steht zum gegenwärtigen Zeitpunkt noch nicht fest, mit welcher Aminosäure das mature Protein beginnt. In der Zählung der SEQ ID NO. 4 (HP70) entsprechen diese Positionen - wie über Figur 1 nachvollzogen werden kann - folgenden Positionsnummern: P140, N141, T182, N188, Y195, A204, G209, T245, K264, K273, (-), Y277, T278, D280, V296, E304, 1306, S317, G326, V328, S329, S341, V345, A376, S381, S393, G399, Y406, V419, Q424, T432, P433, T438, L439, G453 und V466.

Das sind in der Zählung von SEQ ID NO. 7, das heißt HP53 folgende Positionen: P146, N 147, T188, N 194, Y201, A210, G215, T251, K270, K279, (-), Y283, T284, D286, V302, E310, 1312, S323, G332, V334, S335, S347, V351, A382, S387, S399, G405, Y412, V425, Q430, S438, P439, T444, L445, G459 und V472.

So gehen aus der Anmeldung WO 92/21760 A1 Einfach- und Mehrfachvarianten des Subtilisins aus *Bacillus lentus* DSM 5483 in folgenden Positionen hervor: 3, 4, 36, 42, 47, 56, 69, 87, 96, 101, 102, 104, 114, 118, 120, 130, 139, 141, 142, 157, 188, 193, 199, 205, 224, 229, 236, 237, 242, 243, 255 und 268. Die Anmeldung WO 95/23221 A1 offenbart zusätzlich Austausche an diesem Molekül in den Positionen 99, 154 und 211, insbesondere R99G, R99A, R99S, S154D, S154E, L211 D und L211 E. Solche Varianten eignen sich der Anmeldung WO 95/07770 A1 zufolge besonders auch für den Einsatz in Kosmetika. Neben anderen Austauschen ist in der Anmeldung WO 02/088340 A2 auch der Austausch L211G beschrieben, und in WO 03/038082 A2 der Austausch G61A.

Hierunter sind dementsprechend solche bevorzugt, bei denen die weiteren Aminosäureaustausche in einer oder mehreren der Positionen 3, 4, 61, 188, 193, 199 und 211 vorliegen. Dem entsprechen bei HP70 die Positionen P140, N141, G209, A376, S381, S393 und Y406 beziehungsweise bei HP53 die Positionen P146, N147, G215, A382, S387, S399 und Y412.

Hierunter sind entsprechend dem oben Gesagten wiederum solche bevorzugt, bei denen es sich um einen oder mehrere der Aminosäureaustausche 3T, 41, 61A, 188P, 193M, 1991 und 211 D oder 211G handelt, sofern die entsprechend homologen Positionen nicht schon natürlicherweise von einer dieser bevorzugten Aminosäuren eingenommen werden.

Die Austausche S3T und V41 führen, wie insbesondere in WO 02/088340 A2 erläutert ist, vermutlich über einen Stabilisierungeffekt auf das Molekül zu einer Verbesserung dessen Beitrags zur Waschleistung eines Wasch- oder Reinigungsmittels. Die Austausche S3T, V41, A188P, V193M, V1991 und L21 1 D kennzeichnen die gemäß WO 95/23221 A1 als F49 bezeichnete Protease, die in den Beispielen 7 und 8 der vorliegenden Anmeldung als leistungsfähiges, im Stand der Technik etabliertes Vergleichsenzym herangezogen worden ist. Demgegenüber handelt es sich bei den Proteasen HP70 und HP53 noch um unveränderte Wildtyp-Moleküle, deren Aktivität, insbesondere deren Beitrag zur Waschleistung durch ebendiese Austausche verbessert werden dürfte.

Weiter bevorzugt ist eine zuvor beschriebene erfindungsgemäße Alkalische Protease oder ein solches Protein, die beziehungsweise das zusätzlich stabilisiert ist.

Denn eine Erhöhung der Stabilität bei der Lagerung und/oder während des Einsatzes, beispielsweise beim Waschprozeß führt dazu, daß ihre Aktivität länger anhält und damit in der Wirkung verstärkt wird. Als Stabilisierungsmöglichkeiten kommen alle im Stand der Technik beschriebenen und zweckmäßigen Strategien in Betracht, bespielsweise gemäß US 5230891 die kovalente Kopplung an ein Polymer.

Bevorzugt sind Stabilisierungen, die über Punktmutagenese des Moleküls selbst möglich sind. Denn diese erfordern im Anschluß an die Proteingewinnung keine weiteren Arbeitsschritte. Einige hierfür geeignete Punktmutationen sind an sich aus dem Stand der Technik bekannt. So können gemäß US 6087315 und US 6110884 Proteasen dadurch stabilisiert werden, daß man bestimmte Tyrosin-Reste gegen andere austauscht.

Weitere Möglichkeiten sind beispielsweise:
- Veränderung der Bindung von Metallionen, insbesondere der Calcium-Bindungsstellen; beispielsweise gemäß der Lehre der Anmeldungen WO 88/08028 A1 und WO 88/08033 A1; gemäß der ersten dieser Schriften müßten eine oder mehrere der an der Calcium-Bindung beteiligten Aminosäure-Reste gegen negativ geladene Aminosäuren ausgetauscht werden; gemäß der Lehre der Anmeldung WO 88/08033 müßten zur Stabilisierung über die Calcium-Bindung gleichzeitig in mindestens einer der Folgen der beiden Reste Arginin/Glycin Punktmutationen eingeführt werden;
- Gemäß dem Patent US 5453372 können Proteine durch bestimmte Mutationen auf der Oberfläche gegen den Einfluß von denaturierenden Agentien wie Tensiden geschützt werden.

Eine andere Möglichkeit zur Stabilisierung gegenüber erhöhter Temperatur und dem Einwirken von Tensiden wäre in Anwendung der Lehre aus WO 92/21760 A1, WO 02/088340 A2 und WO 03/038082 A2 die Stabilisierung über den Austausch von Aminosäuren, die nahe dem N-Terminus liegen, gegen solche, die vermutlich über nichtkovalente Wechselwirkungen mit dem Rest des Moleküls in Kontakt treten und somit einen Beitrag zur Aufrechterhaltung der globulären Struktur leisten. Dies ist insbesondere für Alkalische Proteasen empfehlenswert, die ursprünglich as *B. lentus* erhalten worden sind. Entsprechende Mutanten sind mit den Varianten gemäß SEQ ID NO. 12 und 16 in den Beispielen der vorliegenden Anmeldung beschrieben.

Bevorzugte Ausführungsformen sind solche, bei denen das Molekül auf mehrere Arten stabilisiert wird. Denn beispielsweise nach WO 89/09819 A1 kann davon ausgegangen werden, daß mehrere stabilisierende Mutationen additiv wirken.

Weiter bevorzugt ist eine zuvor beschriebene erfindungsgemäße Alkalische Protease oder ein solches Protein, die beziehungsweise das zusätzlich derivatisiert ist.

Unter Derivaten werden solche Proteine verstanden, die sich über eine zusätzliche Modifikation von den ausgeführten Proteinen ableiten. Derartige Modifikationen können beispielsweise die Stabilität, Substratspezifität oder die Bindungsstärke an das Substrat oder die enzymatische Aktivität beeinflussen. Sie können auch dazu dienen, um die Allergenizität und/oder lmmunogenizität des Proteins herabzusetzen und damit beispielsweise dessen Hautverträglichkeit zu erhöhen.

Solche Derivatisierungen können beispielsweise biologisch erfolgen, etwa im Zusammenhang mit der Proteinbiosynthese durch den produzierenden Wirtsorganismus. Hier sind Kopplungen niedrigmolekularer Verbindungen wie von Lipiden oder Oligosacchariden besonders hervorzuheben.

Derivatisierungen können aber auch chemisch durchgeführt werden, etwa durch die chemische Umwandlung einer Seitenkette oder durch kovalente Bindung einer anderen, beispielsweise makromolekularen Verbindung an das Protein. Eine chemische Modifikation wird beispielsweise in der Anmeldung DE 4013142 A1 beschrieben. Beispielsweise die Kopplung von Aminen an Carboxylgruppen eines Enzyms zur Veränderung des isoelektrischen Punkts geht aus WO 95/26398 A1 hervor. Es können beispielsweise Makromoleküle wie Proteine, etwa über bifunktionelle chemische Verbindungen an erfindungsgemäße Proteine gebunden werden. So ist es beispielsweise in Anwendung der Lehre von WO 99/57154 A1 möglich, ein erfindungsgemäßes Protein auch über einen Nichtprotein-Linker mit einer spezifischen Bindungsdomäne zu versehen. Solche Derivate eignen sich besonders für den Einsatz in in Wasch- oder Reinigungsmitteln. Analog WO 00/01831 A2 können auch ProteaseInhibitoren über Linker, insbesondere Aminosäure-Linker an die erfindungsgemäßen Proteine gebunden werden. Kopplungen mit sonstigen makromolekularen Verbindungen, wie etwa Polyethylenglykol verbessern das Molekül hinsichtlich weiterer Eigenschaften wie Stabilität oder Hautverträglichkeit; das wurde bereits erläutert.

Unter Derivaten erfindungsgemäßer Proteine können im weitesten Sinne auch Präparationen dieser Enzyme verstanden werden. Je nach Gewinnung, Aufarbeitung oder Präparation kann ein Protein mit diversen anderen Stoffen vergesellschaftet sein, beispielsweise aus der Kultur der produzierenden Mikroorganismen. Ein Protein kann auch, beispielsweise zur Erhöhung seiner Lagerstabilität, mit bestimmten anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, daß es bei der Lagerung keine oder nur geringe Aktivität besitzt, und erst zum Zeitpunkt der Verwendung seine proteolytische Funktion entfaltet. Dies kann beispielsweise über entsprechende Begleitstoffe gesteuert werden. Insbesondere die gemeinsame Präparation von Proteasen mit ProteaseInhibitoren ist vorteilhaft und aus dem Stand der Technik bekannt (WO 00/01826 A2).

Weiter bevorzugt ist eine zuvor beschriebene erfindungsgemäße Alkalische Protease oder ein solches Protein, die beziehungsweise das wenigstens eine antigene Determinante mit einem der in den zuvor bezeichneten Alkalischen Proteasen oder Proteinen gemeinsam hat, insbesondere über mindestens eine der Epitop-Regionen, innerhalb derer die Positionen 3, 4, 36, 42, 47, 56, 61, 69, 87, 96, 99, 101, 102, 104, 114, 118, 120, 130, 139, 141, 142, 154, 157, 188, 193, 199, 205, 211, 224, 229, 236, 237, 242, 243, 255 und 268 in der Zählung der Alkalischen Protease aus *Bacillus lentus* liegen, zuzuordnen über das Alignment in Figur 1.

Das gilt insbesondere für die oben beschriebenen Varianten in diesen Positionen, da sie zum einen an sich bevorzugt sind und zum anderen über Antikörper, die spezifisch gegen diese Regionen gebildet worden sind, von den Proteasen unterschieden werden können, die in diesen Positionen mit dem Wildtypmolekül übereinstimmen.

Die Lösung einer Teilaufgabe und somit einen eigenständigen Erfindungsgegenstand stellen Nukleinsäuren mit einer Nukleotidsequenz dar, die zu der in SEQ ID NO. 3 angegebenen Nukleotidsequenz mindestens zu 85% oder zu der in SEQ ID NO. 6 angegebenen Nukleotidsequenz mindestens zu 85% identisch ist.

Denn zum einen beruht die Detektion der in den Beispielen beschriebenen Protease auf der Isolierung der zugehörigen DNA. Zum anderen können die Nukleinsäuren unmittelbar kloniert und somit in die gentechnische Produktion der angeleiteten Enzyme eingebracht werden.

Hierunter sind diejenigen zunehmend bevorzugt, die zu einer der angegebenen Nukleotidsequenzen zunehmend bevorzugt zu mindestens 87,5%, 90%, 91%, 92%, 93%, 94%, 95%, 96%, 97%, 98%, 99% und ganz besonders bevorzugt zu 100% identisch sind, wobei jeweils alle ganzzahligen oder gebrochenen Zwischenwerte eingeschlossen werden.

Denn entsprechend den oben gemachten Ausführungen und wie in den Beispielen beschrieben ist, konnten im Stand der Technik als zu der SEQ ID NO. 3 und SEQ ID NO. 6 nächstähnliche Nukleotidsequenzen nur solche mit 74,4% beziehungsweise 75,0% Identität gefunden werden.

Die weiteren, aus WO 2004/033668 A2 und dem GenBank-Eintrag AY253983 hervorgehenden DNA-Sequenzen sind bereits oben diskutiert worden und sind zu den hier beschriebenen erfindungsgemäßen Nukleotidsequenzen hinreichend unterschiedlich.

Weiterhin bevorzugt sind solche erfindungsgemäßen Nukleinsäuren, bei denen die Homologiewerte jeweils für den Bereich gelten, der den Nukleotidpositionen 97 bis 1746 gemäß SEQ ID NO. 3 beziehungsweise den Nukleotidpositionen 115 bis 1761 gemäß SEQ ID NO. 6 entspricht.

Hiermit ist entsprechend dem oben Gesagten der Bereich gemeint, der für das jeweils mature, das heißt aktive Protein codiert. Auch das Stop-Codon ist einbezogen, denn dessen Existenz sorgt dafür, daß nicht ein größeres evtl. nicht mehr funktionsfähiges, ungewolltes Fusionsprotein gebildet wird. Somit ist bei der Klonierung darauf zu achten, daß an dieser Stelle ebenfalls ein Stop-Codon liegt, wenn nicht gezielt über den C-Terminus eine Proteinfusion herbeigeführt werden soll. Sollte sich später herausstellen, daß das mature Protein von einem anderen Teil dieser Sequenz gebildet wird, so gilt der Schutzbereich entsprechend für diesen Teil.

Weiterhin bevorzugt sind solche erfindungsgemäßen Nukleinsäuren, bei denen die Homologiewerte jeweils für den Bereich gelten, der den Nukleotidpositionen 97 bis 1410 gemäß SEQ ID NO. 3 beziehungsweise den Nukleotidpositionen 115 bis 1428 gemäß SEQ ID NO. 6 entspricht.

Denn wie in den Beispielen beschrieben ist, reicht es bei den Proteinen HP70 und HP53 aus, den N-terminalen Teil des Gesamtproteins zu verwenden; eine erhebliche C-terminale Deletion führte in beiden Fällen zu einem in Wasch- und Reinigungsmitteln proteolytisch aktiven Enzym, welches einen entsprechenden Beitrag zur Gesamtreinigungsleistung des betreffenden Mittels geliefert hat. Nukleinsäuren, die für derartige Varianten codieren, stellen deshalb bevorzugte Ausführungsformen dar, weil sie eine kosteneffizientere biotechnologische Herstellung der betreffenden Proteine ermöglichen.

Weiterhin und den bisherigen Ausführungen entsprechend bevorzugt sind solche erfindungsgemäßen Nukleinsäuren, die für eine Alkalische Protease oder ein Protein des ersten Erfindungsgegenstands codieren.

Denn ebensolche Proteine sollten mit der vorliegenden Anmeldung zur Verfügung gestellt werden, so daß Nukleinsäuren, die für lediglich inaktive Proteine codieren, keine erfindungsgemäße Lösung darstellen. Bevorzugt sind solche Nukleinsäuren die für mature Proteine codieren, und zunehmend besonders solche, die für zunehmend aktivere Varianten codieren.

Weiterhin bevorzugt sind solche der genannten erfindungsgemäßen Nukleinsäuren, von denen ein eines oder vorzugsweise mehrere Codons durch synonyme Codons ersetzt sind.

Dieser Aspekt bezieht sich insbesondere auf die heterologe Expression der betreffenden Proteasen. So besitzt jeder Organismus, insbesondere jeder Produktionsstamm über eine gewisse Codon-Usage. Hierbei kann es zu Engpässen in der Proteinbiosynthese kommen, wenn die auf der transgenen Nukleinsäure liegenden Codons in der Wirtszelle einer vergleichsweise geringen Zahl von beladenen tRNAs gegenüberstehen. Synonyme Codons codieren dagegen für dieselben Aminosäuren und können in Abhängigkeit vom Wirt besser translatiert werden. Dieses gegebenenfalls notwendige Umschreiben hängt somit von der Wahl des Expressionssystems ab. Insbesondere bei Proben aus unbekannten, eventuell nicht kultivierbaren Organismen kann eine entsprechende Anpassung notwendig sein.

Entsprechend den oben gemachten Ausführungen werden weiterhin die Zellen eines Organismus in den Schutzbreich einbezogen und stellen einen eigenen Erfindungsgegenstand dar, der natürlicherweise eine erfindungsgemäße Nukleinsäure enthält.

Denn über deren Kultivierung können die gewünschten Enzyme direkt zugänglich sein.

Besonders bevorzugt sind hierunter solche Zellen, die natürlicherweise eine Protease oder ein Protein des ersten Erfindungsgegenstands exprimieren und vorzugsweise sekretieren.

Denn hierüber können erfindungsgemäße Proteasen hinsichtlich ihres beabsichtigten Anwendungsgebiets sofort getestet und möglicherweise durch sofortige Kultivierung dieses Organismus in größeren Mengen gewonnen werden.

Hierunter sind wiederum solche Zellen bevorzugt, bei denen es sich um Mikroorganismen handelt, vorzugsweise um Pilze oder Bakterien, hierunter bevorzugt um grampositive Bakterien und besonders bevorzugt um solche der Gattung *Bacillus* oder um gramnegative Bakterien der Gattung *Xanthomonas.*

Denn mit Mikroorganismen hat man im Stand der Technik reichhaltige Erfahrungen hinsichtlich der molekularbiologischen Techniken und der Produktion sammeln können. Das gilt besonders für grampositive Bakterien, von denen solche der Gattung *Bacillus* zu den geläufigsten Produktionsstämmen gehören. Nicht minder bevorzugt sind jedoch gramnegative Bakterien der Gattung *Xanthomonas,* die bislang insbesondere für die Produktion des extrazellulären Polysaccharids Xanthan genutzt werden. Aufgrund der bereits diskutierten und in den Beispielen gezeigten Homologievergleiche erscheint es darüber hinaus möglich, daß natürlicherweise Stämme dieser Gattung die erfindungsgemäß besonders bevorzugten Proteasen HP70 und HP53 produzieren. Zumindest sollte deren Produktion in nahe verwandten Stämmen besonders vorteilhaft realisierbar sein, beipielsweise was deren Codon-Usage betrifft.

Einen weiteren eigenständigen Erfindungsgegenstand stellen Verfahren zur Identifizierung einer Alkalischen Protease des ersten Erfindungsgegenstands dar, welche auf der Isolierung einer Nukleinsäure aus einem natürlich besiedelten Habitat beruhen.

Denn wie mit der vorliegenden Erfindung belegt ist, ist es zur Identifizierung neuer Proteasen nicht unbedingt notwendig, auch die betreffenden Proteasen und Mikroorganismen aus der Natur zu isolieren. Insbesondere über Schrotschußklonierungen oder alternativ über PCR-Primer zu bekannten Sequenzmotiven ist es möglich, direkt die betreffenden Nukleinsäuren aufzufinden.
Solch ein Verfahren ist in den Beispielen 1 bis 3 der vorliegenden Anmeldung dargestellt. Demnach ist es beispielsweise möglich, die Mikroorganismenflora von Bodenproben zu kultivieren, daraus DNA zu isolieren und über eine Klonierung in einen Expressionsvektor auf Proteaseexpression zu testen.

Unter den genannten Verfahren sind diejenigen bevorzugt, bei denen ein, vorzugsweise zwei einander entsprechende Oligonukleotide eingesetzt werden, die als PCR-Primer dienen können und von einer der beiden Sequenzen SEQ ID NO. 3 oder 6 abgeleitet sind.

Ein vergleichbarer, auf einer PCR mit geeigneten Primern beruhender Ansatz geht beispielsweise aus der Anmeldung WO 03/002711 A2 am Beispiel von α-Amylasen hervor. So ist es möglich, anstatt die Mikroorganismen zu kultivieren und daraus die DNA zu präparieren, direkt die in einer Bodenprobe enthaltenen Nukleinsäuren zu amplifizieren. Hierfür eignen sich PCR-basierte Ansätze. Dabei können die unter SEQ ID NO. 3 und 6 angegebenen Nukleotidsequenzen als Vorlage zum Entwerfen von entsprechenden PCR-Primern dienen. Dabei ist es vorteilhaft, nach an sich bekannten Methoden Primer zu entwerfen, die ausschließlich oder insbesondere N-terminal nur wenig mehr als das mature Protein umfassen; zudem kann man die an den dc-Varianten (Beispiel 6) gewonnenen Erkenntnisse dahingehend nutzen, daß man über die PCR lediglich für entsprechend verkürzte Proteine codierende Nukleinsäuren amplifiziert.

Weiterhin sind solche Verfahren bevorzugt, bei denen die isolierte Nukleinsäure kloniert, vorzugsweise exprimiert und besonders bevorzugt über die Protease-Aktivität des Expressionsprodukts als Protease identifiziert wird.

Denn die Klonierung stellt meist, auch wenn nicht zuvor eine PCR durchgeführt worden ist, den wesentlichen molekularbiologischen Schritt dar, mit dem die Gewinnung des zugehörigen Enzyms eingeleitet wird. Die Expression dient der biochemischen Charakterisierung des von der Nukleinsäure abgeleiteten Proteins. Insbesondere, wenn der Test auf Proteaseaktivität, beispielsweise über den Abbau eines Protein-Substrats (vergleiche Beispiele) erfolgreich ist, kann man sicher sein, eine Protease gefunden zu haben, welche in nachfolgenden Tests hinsichtlich ihrer technischen Verwendbarkeit untersucht werden kann.

Einen weiteren eigenständigen Erfindungsgegenstand stellen Vektoren dar, die einen zuvor bezeichneten erfindungsgemäßen Nukleinsäurebereich enthalten.

Denn um mit den erfindungsrelevanten Nukleinsäuren umzugehen, und damit insbesondere die Produktion erfindungsgemäßer Proteine vorzubereiten, werden sie geeigneterweise in Vektoren ligiert. Solche Vektoren sowie die zugehörigen Arbeitsmethoden sind im Stand der Technik ausführlich beschrieben. Vektoren sind in großer Zahl und Variationsbreite, sowohl für die Klonierung als auch für die Expression kommerziell erhältlich. Dazu gehören beispielsweise Vektoren, die sich von bakteriellen Plasmiden, von Bacteriophagen oder von Viren ableiten, oder überwiegend synthetische Vektoren. Ferner werden sie nach der Art der Zelltypen, in denen sie sich zu etablieren vermögen, beispielsweise nach Vektoren für gramnegative, für grampositive Bakterien, für Hefen oder für höhere Eukaryonten unterschieden. Sie bilden geeignete Ausgangspunkte beispielsweise für molekularbiologische und biochemische Untersuchungen sowie für die Expression des betreffenden Gens oder zugehörigen Proteins.

In einer Ausführungsform handelt es sich bei erfindungsgemäßen Vektoren um Klonierungsvektoren.

Denn Klonierungsvektoren eignen sich neben der Lagerung, der biologischen Amplifikation oder der Selektion des interessierenden Gens für dessen molekularbiologische Charakterisierung. Gleichzeitig stellen sie transportierbare und lagerfähige Formen der beanspruchten Nukleinsäuren dar und sind auch Ausgangspunkte für molekularbiologische Techniken, die nicht an Zellen gebunden sind, wie beispielsweise die PCR oder *In-vitro*-Mutagenese-Verfahren.

Vorzugsweise handelt es sich bei erfindungsgemäßen Vektoren um Expressionsvektoren.

Denn derartige Expressionsvektoren sind die Basis dafür, die entsprechenden Nukleinsäuren in biologischen Produktionssystemen zu realisieren und damit die zugehörigen Proteine zu produzieren. Bevorzugte Ausführungsformen dieses Erfindungsgegenstands sind Expressionsvektoren, die die zur Expression notwendigen genetischen Elemente tragen, beispielsweise den natürlichen, ursprünglich vor diesem Gen lokalisierten Promotor oder einen Promotor aus einem anderen Organismus. Diese Elemente können beispielsweise in Form einer sogenannten Expressionskassette angeordnet sein. Alternativ können einzelne oder alle Regulationselemente auch von der jeweiligen Wirtszelle bereitgestellt werden. Besonders bevorzugt sind die Expressionsvektoren hinsichtlich weiterer Eigenschaften, wie beispielsweise die optimale Kopienzahl, auf das gewählte Expressionssystem, insbesondere die Wirtszelle (siehe unten) abgestimmt.

Einen eigenen Erfindungsgegenstand bilden Zellen, die nach gentechnischer Modifizierung einen der zuvor bezeichneten, erfindungsgemäßen Nukleinsäurebereiche enthalten.

Denn diese Zellen enthalten die genetische Information zur Synthese eines erfindungsgemäßen Proteins. Hierunter sind im Gegensatz zu den oben beschriebenen, ebenfalls beanspruchten natürlichen Produzenten diejenigen Zellen gemeint, die nach an sich bekannten Verfahren mit den erfindungsgemäßen Nukleinsäuren versehen worden sind, beziehungsweise die sich von solchen Zellen ableiten. Dafür werden geeigneterweise solche Wirtszellen ausgewählt, die sich vergleichsweise einfach kultivieren lassen und/oder hohe Produktausbeuten liefern.

Sie ermöglichen beispielsweise die Amplifikation der entsprechenden Gene, aber auch deren Mutagenese oder Transkription und Translation und letztlich die biotechnologische Produktion der betreffenden Proteine. Diese genetische Information kann entweder extrachromosomal als eigenes genetisches Element, das heißt bei Bakterien in plasmidaler Lokalisation vorliegen oder in ein Chromosom integriert sein. Die Wahl eines geeigneten Systems hängt von Fragestellungen, wie beispielsweise die Art und Dauer der Lagerung des Gens, beziehungsweise des Organismus oder die Art der Mutagenese oder Selektion ab. So sind im Stand der Technik beispielsweise auf Bakteriophagen - und deren spezifischen Wirtszellen - beruhende Mutagenese- und Selektionsverfahren zur Entwicklung von Waschmittelenzymen beschrieben (WO 97/09446 A1).

In den Ländern, wo entsprechende nationale Gesetze verlangen, daß von solch einem Anmeldungsgegenstand menschliche embryonale Stammzellen ausgenommen werden, wird die vorliegende Erfindung nur für einen entsprechend eingeschränkten Gegenstand beansprucht.

Vorzugsweise liegt der genannte Nukleinsäurebereich auf einem der oben bezeichneten, erfindungsgemäßen Vektoren, insbesondere auf einem Klonierungs- oder Expressionsvektor.

Denn hierdurch werden sie zur Realisierung der vorliegenden Erfindung relevant.

Weiterhin sind solche Zellen bevorzugt, die eine Alkalische Protease oder ein Protein des ersten Erfindungsgegenstands exprimieren, vorzugsweise sekretieren.

Denn Protein-bildende Wirtszellen ermöglichen deren biotechnologische Produktion. Als Wirtszellen zur Proteinexpression eignen sich prinzipiell alle Organismen, das heißt Prokaryonten, Eukaryonten oder Cyanophyta. Bevorzugt sind solche Wirtszellen, die sich genetisch gut handhaben lassen, was beispielsweise die Transformation mit dem Expressionsvektor, dessen stabile Etablierung und die Regulation der Expression angeht, beispielsweise einzellige Pilze oder Bakterien. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Vorzugsweise werden Laborstämme gewählt, die auf die Expression ausgerichtet sind. Solche sind kommerziell oder über allgemein zugängliche Stammsammlungen erhältlich. Jedes erfindungsgemäße Protein kann auf diese Weise theoretisch aus einer Vielzahl von Wirtsorganismen gewonnen werden. Aus der Fülle an verschiedenen nach dem Stand der Technik zur Verfügung stehenden Systeme müssen die optimalen Expressionssysteme für den Einzelfall experimentell ermitteln werden.

Besonders vorteilhaft sind Wirtszellen, die selbst Protease-negativ sind und somit gebildete Proteine nicht abbauen.

Bevorzugte Ausführungsformen stellen solche Wirtszellen dar, die aufgrund entsprechender genetischer Elemente in ihrer Aktivität regulierbar sind, beispielsweise durch kontrollierte Zugabe von chemischen Verbindungen, durch Änderung der Kultivierungsbedingungen oder in Abhängigkeit von der jeweiligen Zelldichte. Diese kontrollierbare Expression ermöglicht eine sehr wirtschaftliche Produktion der interessierenden Proteine; sie ist beispielsweise über ein entsprechendes Element auf dem betreffenden Vektor realisierbar. Geeigneterweise sind Gen, Expressionsvektor und Wirtszelle aufeinander abgestimmt, was beispielsweise die zur Expression notwendigen genetischen Elemente (Ribosomen-Bindungsstelle, Promotoren, Terminatoren) oder die Codon-Usage betrifft.

Bevorzugt sind darunter Wirtszellen, die dadurch gekennzeichnet sind, daß sie Bakterien sind.

Denn Bakterien zeichnen sich durch kurze Generationszeiten und geringe Ansprüche an die Kultivierungsbedingungen aus. Dadurch können kostengünstige Verfahren etabliert werden. Zudem verfügt man bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf etc. gramnegative oder grampositive Bakterien geeignet sein.

In einer bevorzugten Ausführungsform handelt es sich um gramnegative Bakterien, insbesondere der Gattungen *Escherichia coli, Klebsiella, Pseudomonas* oder *Xanthomonas,* insbesondere um Stämme von *E. coli* K12, E. *coli* B oder *Klebsiella planticola,* und ganz besonders um Derivate der Stämme *Escherichia coli* BL21 (DE3), *E. coli* RV308, *E. coli* DH5α, *E. coli* JM1 09, *E. coli* XL-1 oder *Klebsiella planticola* (Rf).

Denn bei gramnegativen Bakterien, wie beispielsweise *E. coli,* wird eine Vielzahl von Proteinen in den periplasmatischen Raum sekretiert. Dies kann für spezielle Anwendungen vorteilhaft sein. In der Anmeldung WO 01/81597 A1 wird ein Verfahren offenbart, nach welchem erreicht wird, daß auch gramnegative Bakterien die exprimierten Proteine ausschleusen. Solch ein System ist auch für die Herstellung erfindungsgemäßer Proteine geeignet. Die als bevorzugt genannten gramnegativen Bakterien sind in der Regel leicht, das heißt kommerziell oder über öffentliche Stammsammlungen zugänglich und im Zusammenspiel mit ebenfalklks in großer Zahl zur Verfügung stehenden übrigen Komponenten wie etwa Vektoren auf spezifische Herstellbedingungen hin optimierbar.

Wie oben erwähnt stellen *Xanthomonas,* aber auch *Pseudomonas* aufgrund ihrer vermuteten Verwandtschaft zu den *in vivo* HP70 und/oder HP53 produzierenden Stämmen erfolgversprechende Wirtszellen dar; nicht zuletzt auch wegen einer vermutlich ähnlichen Codon-Usage.

In einer alternativen, nicht minder bevorzugten Ausführungsform handelt es sich um ein grampositives Bakterium, insbesondere eines der Gattungen *Bacillus, Staphylococcus* oder *Corynebakterium,* ganz besonders der Species *Bacillus lentus, B. licheniformis, B. amyloliquefaciens, B. subtilis, B. globigii* oder *B. alcalophilus, Staphylococcus carnosus* oder *Corynebacterium glutamicum.*

Denn grampositive Bakterien besitzen den gramnegativen gegenüber den grundsätzlichen Unterschied, sekretierte Proteine sogleich in das die Zellen umgebende Nährmedium abzugeben, aus welchem sich, wenn das gewünscht ist, die exprimierten erfindungsgemäßen Proteine direkt aus dem Nährmedium aufreinigen lassen. Zudem sind sie mit den meisten Herkunftsorganismen für technisch wichtige Subtilisine verwandt oder identisch und bilden meist selbst vergleichbare Subtilisine, so daß sie über eine ähnliche Codon-Usage verfügen und ihr Protein-Syntheseapparat naturgemäß entsprechend ausgerichtet ist. Ein weiterer Vorteil kann darin bestehen, daß über dieses Verfahren eine Mischung erfindungsmäßer Proteine mit den endogen von den Wirtsstämmen gebildeten Subtilisinen erhalten werden kann. Solch eine Coexpression geht ebenfalls aus der Anmeldung WO 91102792 hervor. Sollte sie nicht gewünscht sein, müßten die in der Wirtszelle natürlicherweise vorhandenen Proteasegene dauerhaft oder vorübergehend inaktiviert werden.

Weiter bevorzugt sind Wirtszellen, bei denen es sich um eukaryontische Zellen, vorzugsweise der Gattung Saccharomyces handelt.

Beispiele hierfür sind Pilze wie *Actinomyceten* oder eben Hefen wie *Saccharomyces* oder *Kluyveromyces.* Thermophile pilzliche Expressionssysteme werden beispielsweise in WO 96/02653 A1 vorgestellt. Solche eignen sich besonders zur Expression temperaturbeständiger Varianten. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität wünschenswert sein. Auch eine Coexpression mit den natürlicherweise von derartigen Zellen gebildeten Enzymen, wie beispielsweise Cellulasen, kann vorteilhaft sein.

Einen eigenständigen Erfindungsgegenstand stellen Verfahren zur Herstellung einer Alkalischen Proteasen oder eines Proteins nach dem ersten Erfindungsgegenstand dar.

Dazu gehört jedes Verfahren zur Herstellung eines oben beschriebenen, erfindungsgemäßen Proteins, beispielsweise chemische Syntheseverfahren.

Demgegenüber bevorzugt sind jedoch alle im Stand der Technik etablierten, oben in einzelnen Aspekten bereits angesprochenen molekularbiologischen, mikrobiologischen, beziehungsweise biotechnologischen Herstellverfahren.

Vorzugsweise handelt es sich dabei um Verfahren, die unter Einsatz einer oben bezeichneten erfindungsgemäßen Nukleinsäuren erfolgen, vorzugsweise unter Einsatz eines zuvor bezeichneten Vektors und besonders bevorzugt unter Einsatz einer zuvor bezeichneten Zelle erfolgen.

Durch die genannten Nukleinsäuren, insbesondere den im Sequenzprotokoll unter SEQ ID NO. 3 oder 6 angegebenen Nukleinsäuren wird die entsprechend bevorzugte genetische Information in mikrobiologisch verwertbarer Form, das heißt für gentechnische Produktionsverfahren zur Verfügung gestellt. Zunehmend bevorzugt ist die Bereitstellung auf einem von der Wirtszelle besonders erfolgreich verwertbaren Vektor beziehungsweise von solchen Zellen selbst. Die betreffenden Produktionsverfahren sind dem Fachmann an sich bekannt.

Ausführungsformen der vorliegenden Erfindung können auf der Grundlage der zugehörigen Nukleinsäuresequenzen auch zellfreie Expressionssysteme sein, bei denen die Proteinbiosynthese *in vitro* nachvollzogen wird. Alle bereits oben ausgeführten Elemente können auch zu neuen Verfahren kombiniert werden, um erfindungsgemäße Proteine herzustellen. Es ist dabei für jedes erfindungsgemäße Protein eine Vielzahl von Kombinationsmöglichkeiten an Verfahrensschritten denkbar, so daß optimale Verfahren für jeden konkreten Einzelfall experimentell ermittelt werden müssen.

Entsprechend dem oben Gesagten sind unter den zellgebundenen Verfahren solche bevorzugt, bei denen die Nukleotidsequenz in einem oder vorzugsweise mehreren Codons an die Codon-Usage des Wirtsstamms angepaßt worden ist.

Einen eigenen Erfindungsgegenstand stellen Mittel, dar, die eine oben beschriebene, erfindungsgemäße Alkalische Protease enthalten.

Hiermit werden alle Arten von Mitteln, insbesondere Gemische, Rezepturen, Lösungen etc., deren Einsetzbarkeit durch Zugabe eines oben beschriebenen, erfindungsgemäßen Proteins verbessert wird, in den Schutzbereich der vorliegenden Erfindung eingeschlossen. Es kann sich dabei je nach Einsatzgebiet beispielsweise um feste Gemische, beispielsweise Pulver mit gefriergetrockeneten oder verkapselten Proteinen, oder um gelförmige oder flüssige Mittel handeln. Bevorzugte Rezepturen enthalten beispielsweise Puffersubstanzen, Stabilisatoren, Reaktionspartner und/oder Cofaktoren der Proteasen und/oder andere mit den Proteasen synergistische Inhaltsstoffe. Insbesondere sind darunter Mittel für die weiter unten ausgeführten Einsatzgebiete zu verstehen. Weitere Einsatzgebiete gehen aus dem Stand der Technik hervor und werden beispielsweise in dem Handbuch "Industrial enyzmes and their applications" von H. Uhlig, Wiley-Verlag, New York, 1998 dargestellt.

Diesem Erfindungsgegenstand werden als bevorzugte Ausführungsform Mittel zugerechnet, bei denen es sich um Wasch- oder Reinigungsmittel handelt.

Denn wie in den Ausführungsbeispielen der vorliegenden Anmeldung gezeigt ist, konnte für Wasch- und Reinigungsmittel mit einer erfindungsgemäß bevorzugten Protease überraschenderweise eine Leistungssteigerung gegenüber dem proteasefreien Mittel festgestellt werden.

Zu diesem Erfindungsgegenstand zählen alle denkbaren Reinigungsmittelarten, sowohl Konzentrate als auch unverdünnt anzuwendende Mittel, zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet.

Ausführungsformen der vorliegenden Erfindung umfassen alle nach dem Stand der Technik etablierten und/oder alle zweckmäßigen Darreichungsformen der erfindungsgemäßen Wasch- oder Reinigungsmittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

Neben einer erfindungsgemäßen Alkalischen Protease vom Subtilisin-Typ enthält ein erfindungsgemäßes Wasch- oder Reinigungsmittel entsprechend seinem Einsatzgebiet gegebenenfalls weitere Inhaltsstoffe wie weitere Enzyme, Enzymstabilisatoren, Tenside, zum Beispiel nichtionische, anionische und/oder amphotere Tenside, und/oder Bleichmittel, und/oder Builder, sowie gegebenenfalls weitere übliche Inhaltsstoffe, die unten detaillierter ausgeführt werden.

In einer bevorzugten Ausführungsform enthalten die erfindungsgemäßen Wasch- oder Reinigungsmittel die oben beschriebenen, erfindungsgemäßen Alkalischen Proteasen vom Subtilisin-Typ in einer Menge von 2 µg bis 20 mg, vorzugsweise von 5 µg bis 17,5 mg, besonders bevorzugt von 20 µg bis 15 mg, ganz besonders bevorzugt von 50 µg bis 10 mg pro Gramm des Mittels. Eingeschlossen werden alle ganzzahligen und nichtganzzahligen jeweils zwischen diesen Zahlen liegenden Werte.

Die Proteaseaktivität in derartigen Mitteln kann nach der in Tenside, Band 7 (1970), Seite 125-132 beschriebenen Methode ermittelt werden. Sie wird dementsprechend in PE (Protease-Einheiten) angegeben.

Bei dem Vergleich der Leistungen zweier Waschmittelenzyme, wie etwa in den Beispielen der vorliegenden Anmeldung, muß zwischen proteingleichem und aktivitätsgleichem Einsatz unterschieden werden. Insbesondere bei gentechnisch erhaltenen, weitgehend nebenaktivitätsfreien Präparationen ist der proteingleiche Einsatz angebracht. Denn damit ist eine Aussage darüber möglich, ob dieselben Proteinmengen - als Maß für den Ertrag der fermentativen Produktion - zu vergleichbaren Ergebnissen führen. Klaffen die jeweiligen Verhältnisse von Aktivsubstanz zu Gesamtprotein (die Werte der spezifischen Aktivität) auseinander, so ist ein aktivitätsgleicher Vergleich zu empfehlen, weil hierüber die jeweiligen enzymatischen Eigenschaften verglichen werden. Generell gilt, daß eine niedrige spezifische Aktivität durch Zugabe einer größeren Proteinmenge ausgeglichen werden kann. Hierbei handelt es sich letztlich um eine ökonomische Erwägung.

Nun folgt eine nicht erschöpfende Zusammenstellung wichtiger, für Wasch- und Reinigungsmittel üblicher Inhaltsstoffe. Ersatzweise oder ergänzend können weitere für den jeweiligen Zweck geeignete Inhaltsstoffe hinzukommen.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann, beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die vorteilhafterweise eingesetzt werden kann, sind die Alkylpolyglycoside (APG). Einsetzbare Alkypolyglycoside genügen der allgemeinen Formel RO(G)_{z}, in der R einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosylierungsgrad z liegt dabei zwischen 1,0 und 4,0, vorzugsweise zwischen 1,0 und 2,0 und insbesondere zwischen 1,1 und 1,4. Bevorzugt eingesetzt werden lineare Alkylpolyglucoside, also Alkylpolyglycoside, in denen der Polyglycosylrest ein Glucoserest und der Alkylrest ein n-Alkylrest ist.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Der Anteil dieser nichtionischen Tenside liegt vorzugsweise nicht über dem der ethoxylierten Fettalkohole, insbesondere bei nicht mehr als der Hälfte davon.

Weitere geeignete Tenside sind Polyhydroxyfettsäureamide der Formel (II), 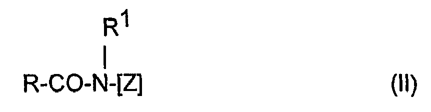 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (III), 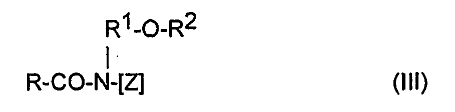 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierenden Zuckers erhalten, beispielsweise Glucose, Fructose, Maltose, Lactose, Galactose, Mannose oder Xylose. Die N-Alkoxy- oder N-Aryloxy-substituierten Verbindungen können beispielsweise durch Umsetzung mit Fettsäuremethylestern in Gegenwart eines Alkoxids als Katalysator in die gewünschten Polyhydroxyfettsäureamide überführt werden.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, das heißt Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende Alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse beziehungsweise Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂C₁₅Alkylsulfate sowie C₁₄-C₁₅-Alkylsulfate bevorzugt. Auch 2,3-Alkylsulfate sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen bis 5 Gew.-%, üblicherweise von 1 bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe oben). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, zum Beispiel Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Die Tenside können in den erfindungsgemäßen Reinigungs- oder Waschmitteln insgesamt in einer Menge von vorzugsweise 5 Gew.-% bis 50 Gew.-%, insbesondere von 8 Gew.-% bis 30 Gew.-%, bezogen auf das fertige Mittel, enthalten sein.

Erfindungsgemäße Wasch- oder Reinigungsmittel können Bleichmittel enthalten. Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie H₂O₂ liefernde persaure Salze oder Persäuren, wie Persulfate beziehungsweise Perschwefelsäure. Brauchbar ist auch das Harnstoffperoxohydrat Percarbamid, das durch die Formel H₂N-CO-NH₂·H₂O₂ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-α-Naphthoesäure und Magnesiummonoperphthalat, die aliphatischen oder substituiert aliphatischen Peroxysäuren, wie Peroxylaurinsäure, Peroxystearinsäure, ε-Phthatimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyl-di(6-aminopercapronsäure) können eingesetzt werden.

Der Gehalt der Wasch- oder Reinigungsmittel an Bleichmittel kann 1 bis 40 Gew.-% und insbesondere 10 bis 20 Gew.-%, betragen, wobei vorteilhafterweise Perboratmonohydrat oder Percarbonat eingesetzt wird.

Um beim Waschen bei Temperaturen von 60 °C und darunter, und insbesondere bei der Wäschevorbehandlung eine verbesserte Bleichwirkung zu erreichen, können die Mittel auch Bleichaktivatoren enthalten. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O-und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glycolurile, insbesondere 1,3,4,6-Tetraacetylglycoluril (TAGU), N-Acylimide, insbesondere N-Nonanoylsuccinimid (NOSl), acylierte Phenolsulfonate, insbesondere n-Nonanoyl- oder Isononanoyloxybenzolsulfonat (n- beziehungsweise iso-NOBS), acylierte Hydroxycarbonsäuren, wie Triethyl-O-acetylcitrat (TEOC), Carbonsäureanhydride, insbesondere Phthalsäureanhydrid, Isatosäureanhydrid und/oder Bernsteinsäureanhydrid, Carbonsäureamide, wie N-Methyldiacetamid, Glycolid, acylierte mehrwertige Alkohole, insbesondere Triacetin, Ethylenglycoldiacetat, Isopropenylacetat, 2,5-Diacetoxy-2,5-dihydrofuran und die aus den deutschen Patentanmeldungen DE 196 16 693 und DE 196 16 767 bekannten Enolester sowie acetyliertes Sorbitol und Mannitol beziehungsweise deren in der europäischen Patentanmeldung EP 0 525 239 beschriebene Mischungen (SORMAN), acylierte Zuckerderivate, insbesondere Pentaacetylglucose (PAG), Pentaacetylfructose, Tetraacetylxylose und Octaacetyllactose sowie acetyliertes, gegebenenfalls N-alkyliertes Glucamin beziehungsweise Gluconolacton, Triazol beziehungsweise Triazolderivate und/oder teilchenförmige Caprolactame und/oder Caprolactamderivate, bevorzugt N-acylierte Lactame, beispielsweise N-Benzoylcaprolactam und N-Acetylcaprolactam, die aus den internationalen Patentanmeldungen WO 94/27970, WO 94/28102, WO 94/28103, WO 95/00626, WO 95/14759 und WO 95/17498 bekannt sind. Die aus der deutschen Patentanmeldung DE 196 16 769 bekannten hydrophil substituierten Acylacetale und die in der deutschen Patentanmeldung DE 196 16 770 sowie der internationalen Patentanmeldung WO 95/14075 beschriebenen Acyllactame werden ebenfalls bevorzugt eingesetzt. Auch die aus der deutschen Patentanmeldung DE 44 43 177 bekannten Kombinationen konventioneller Bleichaktivatoren können eingesetzt werden. Ebenso können Nitrilderivate wie Cyanopyridine, Nitrilquats, zum Beispiel N-Alkylammoniumacetonitrile, und/oder Cyanamidderivate eingesetzt werden. Bevorzugte Bleichaktivatoren sind Natrium-4-(octanoyloxy)-benzolsulfonat, n-Nonanoyl- oder Isononanoyloxybenzolsulfonat (n- beziehungsweise iso-NOBS), Undecenoyloxybenzolsulfonat (UDOBS), Natriumdodecanoyloxybenzolsulfonat (DOBS), Decanoyloxybenzoesäure (DOBA, OBC 10) und/oder Dodecanoyloxybenzolsulfonat (OBS 12), sowie N-Methylmorpholinum-acetonitril (MMA). Derartige Bleichaktivatoren können im üblichen Mengenbereich von 0,01 bis 20 Gew.-%, vorzugsweise in Mengen von 0,1 bis 15 Gew.-%, insbesondere 1 Gew.-% bis 10 Gew.-%, bezogen auf die gesamte Zusammensetzung, enthalten sein.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze beziehungsweise Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren geeignet, wobei solche Verbindungen bevorzugt eingesetzt werden, die in der DE 19709284 A1 beschrieben sind.

Erfindungsgemäße Wasch- oder Reinigungsmittel enthalten in der Regel einen oder mehrere Builder, insbesondere Zeolithe, Silikate, Carbonate, organische Cobuilder und - wo keine ökologischen Gründe gegen ihren Einsatz sprechen - auch die Phosphate. Letztere sind insbesondere in Reinigungsmitteln für das maschinelle Geschirrspülen bevorzugt einzusetzende Gerüststoffe.

Zu nennen sind hier kristalline, schichtförmige Natriumsilicate der allgemeinen Formel NaMSiₓO₂ₓ₊₁·yH₂O, wobei M Natrium oder Wasserstoff bedeutet, x eine Zahl von 1,6 bis 4, vorzugsweise 1,9 bis 4,0 und y eine Zahl von 0 bis 20 ist und bevorzugte Werte für x 2, 3 oder 4 sind. Derartige kristalline Schichtsilicate werden beispielsweise in der europäischen Patentanmeldung EP 164514 beschrieben. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅·yH₂O bevorzugt. Im Handel befinden sich derartige Verbindungen beispielsweise unter der Bezeichnung SKS^{®} (Firma Clariant). So handelt es sich bei SKS-6^{®} vorwiegend um ein δ-Natriumdisilicat mit der Formel Na₂Si₂O₅·yH₂O, bei SKS-7^{®} vorwiegend um das β-Natriumdisilicat. Durch Reaktion mit Säuren (zum Beispiel Citronensäure oder Kohlensäure) entsteht aus dem δ-Natriumdisilicat Kanemit NaHSi₂O₅·yH₂O, im Handel unter den Bezeichnungen SKS-9^{®} beziehungsweise SKS-10^{®} (Firma Clariant). Von Vorteil kann es auch sein, chemische Modifikationen dieser Schichtsilicate einzusetzen. So kann beispielsweise die Alkalität der Schichtsilicate geeignet beeinflußt werden. Mit Phosphat beziehungsweise mit Carbonat dotierte Schichtsilicate weisen im Vergleich zu dem δ-Natriumdisilicat veränderte Kristallmorphologien auf, lösen sich schneller und zeigen im Vergleich zu δ-Natriumdisilicat ein erhöhtes Calciumbindevermögen. So sind Schichtsilicate der allgemeinen Summenformel x Na₂O • y SiO₂ • z P₂O₅, in der das Verhältnis x zu y einer Zahl 0,35 bis 0,6, das Verhältnis x zu z einer Zahl von 1,75 bis 1200 und das Verhältnis y zu z einer Zahl von 4 bis 2800 entsprechen, in der Patentanmeldung DE 196 01 063 beschrieben. Die Löslichkeit der Schichtsilicate kann auch erhöht werden, indem besonders feinteilige Schichtsilicate eingesetzt werden. Auch Compounds aus den kristallinen Schichtsilicaten mit anderen Inhaltsstoffen können eingesetzt werden. Dabei sind insbesondere Compounds mit Cellulosederivaten, die Vorteile in der desintegrierenden Wirkung aufweisen und insbesondere in Waschmitteltabletten eingesetzt werden, sowie Compounds mit Polycarboxylaten, zum Beispiel Citronensäure, beziehungsweise polymeren Polycarboxylaten, zum Beispiel Copolymeren der Acrylsäure, zu nennen.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Obertlächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Insbesondere bevorzugt sind verdichtete/kompaktierte amorphe Silikate, compoundierte amorphe Silikate und übertrocknete röntgenamorphe Silikate.

Ein gegebenenfalls einsetzbarer, feinkristalliner, synthetischer und gebundenes Wasser enthaltender Zeolith ist vorzugsweise Zeolith A und/oder P. Als Zeolith P wird Zeolith MAP^{®} (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX^{®} vertrieben wird und durch die Formel

nNa₂O · (1-n)K₂O · Al₂O₃ · (2 - 2,5)SiO₂ · (3,5 - 5,5) H₂O

beschrieben werden kann. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- beziehungsweise Pentakaliumtriphosphat (Natrium- beziehungsweise Kaliumtripolyphosphat) in der Wasch- und Reinigungsmittel-Industrie die größte Bedeutung.

Alkalimetallphosphate ist dabei die summarische Bezeichnung für die Alkalimetall-(insbesondere Natrium- und Kalium-) -Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen beziehungsweise Kalkinkrustationen in Geweben und tragen überdies zur Reinigungsleistung bei.

Natriumdihydrogenphosphat, NaH₂PO₄, existiert als Dihydrat (Dichte 1,91 gcm⁻³, Schmelzpunkt 60°) und als Monohydrat (Dichte 2,04 gcm⁻³). Beide Salze sind weiße, in Wasser sehr leicht lösliche Pulver, die beim Erhitzen das Kristallwasser verlieren und bei 200°C in das schwach saure Diphosphat (Dinatriumhydrogendiphosphat, Na₂H₂P₂O₇), bei höherer Temperatur in Natiumtrimetaphosphat (Na₃P₃O₉) und Maddrellsches Salz (siehe unten), übergehen. NaH₂PO₄ reagiert sauer; es entsteht, wenn Phosphorsäure mit Natronlauge auf einen pH-Wert von 4,5 eingestellt und die Maische versprüht wird. Kaliumdihydrogenphosphat (primäres oder einbasiges Kaliumphosphat, Kaliumbiphosphat, KDP), KH₂PO₄, ist ein weißes Salz der Dichte 2,33 gcm⁻³, hat einen Schmelzpunkt von 253°C [Zersetzung unter Bildung von Kaliumpolyphosphat (KPO₃)ₓ] und ist leicht löslich in Wasser.

Dinatriumhydrogenphosphat (sekundäres Natriumphosphat), Na₂HPO₄, ist ein farbloses, sehr leicht wasserlösliches kristallines Salz. Es existiert wasserfrei und mit 2 Mol. (Dichte 2,066 gcm⁻³, Wasserverlust bei 95°), 7 Mol. (Dichte 1,68 gcm⁻³, Schmelzpunkt 48°C unter Verlust von 5 H₂O) und 12 Mol. Wasser (Dichte 1,52 gcm⁻³, Schmelzpunkt 35°C unter Verlust von 5 H₂O) wird bei 100°C wasserfrei und geht bei stärkerem Erhitzen in das Diphosphat Na₄P₂O₇ über. Dinatriumhydrogenphosphat wird durch Neutralisation von Phosphorsäure mit Sodalösung unter Verwendung von Phenolphthalein als Indikator hergestellt. Dikaliumhydrogenphosphat (sekundäres od. zweibasiges Kaliumphosphat), K₂HPO₄, ist ein amorphes, weißes Salz, das in Wasser leicht löslich ist.

Trinatriumphosphat, tertiäres Natriumphosphat, Na₃PO₄, sind farblose Kristalle, die als Dodecahydrat eine Dichte von 1,62 gcm⁻³ und einen Schmelzpunkt von 73-76°C (Zersetzung), als Decahydrat (entsprechend 19-20% P₂O₅) einen Schmelzpunkt von 100°C und in wasserfreier Form (entsprechend 39-40% P₂O₅) eine Dichte von 2,536 gcm⁻³ aufweisen. Trinatriumphosphat ist in Wasser unter Alkalischer Reaktion leicht löslich und wird durch Eindampfen einer Lösung aus genau 1 Mol Dinatriumphosphat und 1 Mol NaOH hergestellt. Trikaliumphosphat (tertiäres oder dreibasiges Kaliumphosphat), K₃PO₄, ist ein weißes, zerfließliches, körniges Pulver der Dichte 2,56 gcm⁻³, hat einen Schmelzpunkt von 1340° und ist in Wasser mit Alkalischer Reaktion leicht löslich. Es entsteht zum Beispiel beim Erhitzen von Thomasschlacke mit Kohle und Kaliumsulfat. Trotz des höheren Preises werden in der Reinigungsmittel-Industrie die leichter löslichen, daher hochwirksamen, Kaliumphosphate gegenüber entsprechenden Natrium-Verbindungen vielfach bevorzugt.

Tetranatriumdiphosphat (Natriumpyrophosphat), Na₄P₂O₇, existiert in wasserfreier Form (Dichte 2,534 gcm⁻³, Schmelzpunkt 988°C, auch 880°C angegeben) und als Decahydrat (Dichte 1,815-1,836 gcm⁻³, Schmelzpunkt 94°C unter Wasserverlust). Beide Substanzen sind farblose, in Wasser mit Alkalischer Reaktion lösliche Kristalle. Na₄P₂O₇ entsteht beim Erhitzen von Dinatriumphosphat auf >200°C oder indem man Phosphorsäure mit Soda im stöchiometrischem Verhältnis umsetzt und die Lösung durch Versprühen entwässert. Das Decahydrat komplexiert Schwermetall-Salze und Härtebildner und verringert daher die Härte des Wassers. Kaliumdiphosphat (Kaliumpyrophosphat), K₄P₂O₇, existiert in Form des Trihydrats und stellt ein farbloses, hygroskopisches Pulver mit der Dichte 2,33 gcm⁻³ dar, das in Wasser löslich ist, wobei der pH-Wert der 1 %igen Lösung bei 25°C 10,4 beträgt.

Durch Kondensation des NaH₂PO₄ beziehungsweise des KH₂PO₄ entstehen höhermolekulare Natrium- und Kaliumphosphate, bei denen man cyclische Vertreter, die Natrium- beziehungsweise Kaliummetaphosphate und kettenförmige Typen, die Natriumbeziehungsweise Kaliumpolyphosphate, unterscheiden kann. Insbesondere für letztere sind eine Vielzahl von Bezeichnungen in Gebrauch: Schmelz- oder Glühphosphate, Grahamsches Salz, Kurrolsches und Maddrellsches Salz. Alle höheren Natrium- und Kaliumphosphate werden gemeinsam als kondensierte Phosphate bezeichnet.

Das technisch wichtige Pentanatriumtriphosphat (Na₅P₃O₁₀; Natriumtripolyphosphat) ist ein wasserfrei oder mit 6 H₂O kristallisierendes, nicht hygroskopisches, weißes, wasserlösliches Salz der allgemeinen Formel NaO-[P(O)(ONa)-O]ₙ-Na mit n=3. In 100 g Wasser lösen sich bei Zimmertemperatur etwa 17 g, bei 60°C ca. 20 g, bei 100°C rund 32 g des kristallwasserfreien Salzes; nach zweistündigem Erhitzen der Lösung auf 100°C entstehen durch Hydrolyse etwa 8% Orthophosphat und 15% Diphosphat. Bei der Herstellung von Pentanatriumtriphosphat wird Phosphorsäure mit Sodalösung oder Natronlauge im stöchiometrischen Verhältnis zur Reaktion gebracht und die Lösung durch Versprühen entwässert. Ähnlich wie Grahamsches Salz und Natriumdiphosphat löst Pentanatriumtriphosphat viele unlösliche Metall-Verbindungen (auch Kalkseifen usw.). Pentakaliumtriphosphat, K₅P₃O₁₀ (Kaliumtripolyphosphat), kommt beispielsweise in Form einer 50 Gew.-%-igen Lösung (> 23% P₂O₅, 25% K₂O) in den Handel. Die Kaliumpolyphosphate finden in der Wasch- und Reinigungsmittel-Industrie breite Verwendung. Weiter existieren auch Natriumkaliumtripolyphosphate, welche ebenfalls im Rahmen der vorliegenden Erfindung einsetzbar sind. Diese entstehen beispielsweise, wenn man Natriumtrimetaphosphat mit KOH hydrolysiert:

(NaPO₃)₃ + 2 KOH → Na₃K₂P₃O₁₀ + H₂O

Diese sind erfindungsgemäß genau wie Natriumtripolyphosphat, Kaliumtripolyphosphat oder Mischungen aus diesen beiden einsetzbar; auch Mischungen aus Natriumtripolyphosphat und Natriumkaliumtripolyphosphat oder Mischungen aus Kaliumtripolyphosphat und Natriumkaliumtripolyphosphat oder Gemische aus Natriumtripolyphosphat und Kaliumtripolyphosphat und Natriumkaliumtripolyphosphat sind erfindungsgemäß einsetzbar.

Als organische Cobuilder können in den erfindungsgemäßen Wasch- und Reinigungsmitteln insbesondere Polycarboxylate oder Polycarbonsäuren, polymere Polycarboxylate, Polyasparaginsäure, Polyacetale, gegebenenfalls oxidierte Dextrine, weitere organische Cobuilder (siehe unten) sowie Phosphonate eingesetzt werden. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen.

Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu vermeiden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Sie besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln, sofern nicht der sich durch die Mischung der übrigen Komponenten ergebende pH-Wert gewünscht ist. Insbesondere sind hierbei system- und umweltverträgliche Säuren wie Citronensäure, Essigsäure, Weinsäure, Äpfelsäure, Milchsäure, Glykolsäure, Bernsteinsäure, Glutarsäure, Adipinsäure, Gluconsäure und beliebige Mischungen aus diesen zu nennen. Aber auch Mineralsäuren, insbesondere Schwefelsäure oder Basen, insbesondere Ammonium- oder Alkalihydroxide können als pH-Regulatoren dienen. Derartige Regulatoren sind in den erfindungemäßen Mitteln in Mengen von vorzugsweise nicht über 20 Gew.-%, insbesondere von 1,2 Gew.-% bis 17 Gew.-%, enthalten.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70 000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2 000 bis 20 000 g/mol aufweisen. Aufgrund ihrer überlegenen Löslichkeit können aus dieser Gruppe wiederum die kurzkettigen Polyacrylate, die Molmassen von 2 000 bis 10 000 g/mol, und besonders bevorzugt von 3 000 bis 5 000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2 000 bis 70 000 g/mol, vorzugsweise 20 000 bis 50 000 g/mol und insbesondere 30 000 bis 40 000 g/mol. Die (co-) polymeren Polycarboxylate können entweder als Pulver oder als wässerige Lösung eingesetzt werden. Der Gehalt der Mittel an (co-)polymeren Polycarboxylaten kann von 0,5 bis 20 Gew.-%, insbesondere 1 bis 10 Gew.-%, betragen.

Zur Verbesserung der Wasserlöslichkeit können die Polymere auch Allylsulfonsäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol beziehungsweise Vinylalkohol-Derivate oder die als Monomere Salze der Acrylsäure und der 2-Alkylallylsulfonsäure sowie Zucker-Derivate enthalten.

Weitere bevorzugte Copolymere sind solche, die als Monomere vorzugsweise Acrolein und Acrylsäure/Acrylsäuresalze beziehungsweise Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren beziehungsweise deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere beziehungsweise Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500 000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose ist, welche ein DE von 100 besitzt. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2 000 bis 30 000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Besonders bevorzugte organische Builder für erfindungsgemäße Mittel sind oxidierte Stärken, beziehungsweise deren Derivate aus den Anmeldungen EP 472042, WO 97/25399, und EP 755944.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolith-, carbonat- und/oder silicathaltigen Formulierungen zwischen 3 und 15 Gew.-%.

Weitere brauchbare organische Cobuilder sind beispielsweise acetylierte Hydroxycarbonsäuren beziehungsweise deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- beziehungsweise Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP beziehungsweise als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüberhinaus können alle Verbindungen, die in der Lage sind, Komplexe mit Erdalkaliionen auszubilden, als Cobuilder eingesetzt werden.

Buildersubstanzen können in den erfindungsgemäßen Wasch- oder Reinigungsmitteln gegebenenfalls in Mengen bis zu 90 Gew.-% enthalten sein. Sie sind vorzugsweise in Mengen bis zu 75 Gew.-% enthalten. Erfindungsgemäße Waschmittel weisen Buildergehalte von insbesondere 5 Gew.-% bis 50 Gew.-% auf. In erfindungsgemäßen Mitteln für die Reinigung harter Oberflächen, insbesondere zur maschinellen Reinigung von Geschirr, beträgt der Gehalt an Buildersubstanzen insbesondere 5 Gew.-% bis 88 Gew.-%, wobei in derartigen Mitteln vorzugsweise keine wasserunlöslichen Buildermaterialien eingesetzt werden. In einer bevorzugten Ausführungsform erfindungsgemäßer Mittel zur insbesondere maschinellen Reinigung von Geschirr sind 20 Gew.-% bis 40 Gew.-% wasserlöslicher organischer Builder, insbesondere Alkalicitrat, 5 Gew.-% bis 15 Gew.-% Alkalicarbonat und 20 Gew.-% bis 40 Gew.-% Alkalidisilikat enthalten.

Lösungsmittel, die in den flüssigen bis gelförmigen Zusammensetzungen von Wasch- und Reinigungsmitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propyl-ether, Dipropylenglykolmonomethyl-, oder -ethylether, Di-isopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykolt-butylether sowie Mischungen dieser Lösungsmittel.

Lösungsmittel können in den erfindungsgemäßen flüssigen bis gelförmigen Wasch- und Reinigungsmitteln in Mengen zwischen 0,1 und 20 Gew.-%, bevorzugt aber unter 15 Gew.-% und insbesondere unterhalb von 10 Gew.-% eingesetzt werden.

Zur Einstellung der Viskosität können der erfindungsgemäßen Zusammensetzung ein oder mehrere Verdicker, beziehungsweise Verdickungssysteme zugesetzt werden. Diese hochmolekularen Stoffe, die auch Quell(ungs)mittel genannt werden, saugen meist die Flüssigkeiten auf und quellen dabei auf, um schließlich in zähflüssige echte oder kolloide Lösungen überzugehen.

Geeignete Verdicker sind anorganische oder polymere organische Verbindungen. Zu den anorganischen Verdickern zählen beispielsweise Polykieselsäuren, Tonmineralien wie Montmorillonite, Zeolithe, Kieselsäuern und Bentonite. Die organischen Verdicker stammen aus den Gruppen der natürlichen Polymere, der abgewandelten natürlichen Polymere und der vollsynthetischen Polymere. Solche aus der Natur stammenden Polymere sind beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine und Casein. Abgewandelte Naturstoffe, die als Verdicker verwendet werden, stammen vor allem aus der Gruppe der modifizierten Stärken und Cellulosen. Beispielhaft seien hier Carboxymethylcellulose und andere Celluloseether, Hydroxyethyl- und -propylcellulose sowie Kernmehlether genannt. Vollsynthetische Verdicker sind Polymere wie Polyacryl- und Polymethacryl-Verbindungen, Vinylpolymere, Polycarbonsäuren, Polyether, Polyimine, Polyamide und Polyurethane.

Die Verdicker können in einer Menge bis zu 5 Gew.-%, vorzugsweise von 0,05 bis 2 Gew.-%, und besonders bevorzugt von 0,1 bis 1,5 Gew.-%, bezogen auf die fertige Zusammensetzung, enthalten sein.

Das erfindungsgemäße Wasch- und Reinigungsmittel kann gegebenenfalls als weitere übliche Inhaltsstoffe Sequestrierungsmittel, Elektrolyte und weitere Hilfsstoffe, wie optische Aufheller, Vergrauungsinhibitoren, Silberkorrosionsinhibitoren, Farbübertragungsinhibitoren, Schauminhibitoren, Abrasivstoffe, Farb- und/oder Duftstoffe, sowie mikrobielle Wirkstoffe, UV-Absorbenzien und/oder Enzymstabilisatoren enthalten.

Erfindungsgemäße Textilwaschmittel können als optische Aufheller Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Um einen Silberkorrosionsschutz zu bewirken, können in erfindungsgemäßen Reinigungsmitteln für Geschirr Silberkorrosionsinhibitoren eingesetzt werden. Solche sind aus dem Stand der Technik bekannt, beispielsweise Benzotriazole, Eisen(III)-chlorid oder CoSO₄. Wie beispielsweise aus der europäischen Patentschrift EP 0 736 084 B1 bekannt ist, sind für die gemeinsame Verwendung mit Enzymen besonders geeignete Silberkorrosionsinhibitoren Mangan-, Titan-, Zirkonium-, Hafnium-, Vanadium-, Cobalt- oder Cersalze und/oder -komplexe, in denen die genannten Metalle in einer der Oxidationsstufen II, III, IV, V oder VI vorliegen. Beispiele für derartige Verbindungen sind MnSO₄, V₂O₅, V₂O₄, VO₂ TiOSO₄ K₂TiF₆, K₂ZrF₆, Co(NO₃)₂, Co(NO₃)₃, sowie deren Gemische.

"Soil-Release"-Wirkstoffe oder "Soil-Repellents" sind zumeist Polymere, die bei der Verwendung in einem Waschmittel der Wäschefaser schmutzabstoßende Eigenschaften verleihen und/oder das Schmutzablösevermögen der übrigen Waschmittelbestandteile unterstützen. Ein vergleichbarer Effekt kann auch bei deren Einsatz in Reinigungsmitteln für harte Oberflächen beobachtet werden.

Besonders wirksame und seit langer Zeit bekannte Soil-Release-Wirkstoffe sind Copolyester mit Dicarbonsäure-, Alkylenglykol- und Polyalkylenglykoleinheiten. Beispiele dafür sind Copolymere oder Mischpolymere aus Polyethylenterephthalat und Polyoxyethylenglykol (DT 16 17 141, beziehungsweise DT 22 00 911). In der deutschen Offenlegungsschrift DT 22 53 063 sind saure Mittel genannt, die unter anderem ein Copolymer aus einer dibasigen Carbonsäure und einem Alkylen- oder Cycloalkylenpolyglykol enthalten. Polymere aus Ethylenterephthalat und Polyethylenoxid-terephthalat und deren Einsatz in Waschmitteln sind in den deutschen Schriften DE 28 57 292 und DE 33 24 258 und der Europäischen Patentschrift EP 0 253 567 beschrieben. Das europäische Patent EP 066 944 betrifft Mittel, die einen Copolyester aus Ethylenglykol, Polyethylenglykol, aromatischer Dicarbonsäure und sulfonierter aromatischer Dicarbonsäure in bestimmten Molverhältnissen enthalten. Aus dem europäischen Patent EP 0 185 427 sind Methyl- oder Ethylgruppenendverschlossene Polyester mit Ethylen- und/oder Propylen-terephthalat- und Polyethylenoxid-terephthalat-Einheiten und Waschmitel, die derartiges Soil-release-Polymer enthalten, bekannt. Das europäische Patent EP 0 241 984 betrifft einen Polyester, der neben Oxyethylen-Gruppen und Terephthalsäureeinheiten auch substituierte Ethyleneinheiten sowie Glycerineinheiten enthält. Aus dem europäischen Patent EP 0 241 985 sind Polyester bekannt, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind. Aus der europäischen Patentanmeldung EP 0 272 033 sind zumindest anteilig durch C₁₋₄-Alkyl- oder Acylreste endgruppenverschlossene Polyester mit Poly-propylenterephthalat- und Polyoxyethylenterephthalat-Einheiten bekannt. Das europäische Patent EP 0 274 907 beschreibt sulfoethylendgruppenverschlossene terephthalathaltige Soil-release-Polyester. Gemäß der europäischen Patentanmeldung EP 0 357 280 werden durch Sulfonierung ungesättigter Endgruppen Soil-Release-Polyester mit Terephthalat-, Alkylenglykol- und Poly-C₂₋₄-Glykol-Einheiten hergestellt. Die internationale Patentanmeldung WO 95/32232 betrifft saure, aromatische schmutzablösevermögende Polyester. Aus der internationalen Patentanmeldung WO 97/31085 sind nicht polymere soil-repellent-Wirkstoffe für Materialien aus Baumwolle mit mehreren funktionellen Einheiten bekannt: Eine erste Einheit, die beispielsweise kationisch sein kann, ist zur Adsorption auf die Baumwolloberfläche durch elektrostatische Wechselwirkung befähigt, und eine zweite Einheit, die hydrophob ausgebildet ist, ist verantwortlich für das Verbleiben des Wirkstoffs an der Wasser/ Baumwolle-Grenzfläche.

Zu den für den Einsatz in erfindungsgemäßen Textilwaschmitteln in Frage kommenden Farbübertragungsinhibitoren gehören insbesondere Polyvinylpyrrolidone, Polyvinylimidazole, polymere N-Oxide wie Poly-(vinylpyridin-N-oxid) und Copolymere von Vinylpyrrolidon mit Vinylimidazol.

Beim Einsatz in maschinellen Reinigungsverfahren kann es von Vorteil sein, den betreffenden Mitteln Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche, beziehungsweise dispergierbare Trägersubstanz gebunden. Insbesondere sind dabei Mischungen aus Paraffinen und Bistearylethylendiamiden bevorzugt.

Ein erfindungsgemäßes Reinigungsmittel für harte Oberflächen kann darüber hinaus abrasiv wirkende Bestandteile, insbesondere aus der Gruppe umfassend Quarzmehle, Holzmehle, Kunststoffmehle, Kreiden und Mikroglaskugeln sowie deren Gemische, enthalten. Abrasivstoffe sind in den erfindungsgemäßen Reinigungsmitteln vorzugsweise nicht über 20 Gew.-%, insbesondere von 5 Gew.-% bis 15 Gew.-%, enthalten.

Farb- und Duftstoffe werden Wasch- und Reinigungsmitteln zugesetzt, um den ästhetischen Eindruck der Produkte zu verbessern und dem Verbraucher neben der Wasch- und Reinigungsleistung ein visuell und sensorisch "typisches und unverwechselbares" Produkt zur Verfügung zu stellen. Als Parfümöle beziehungsweise Duftstoffe können einzelne Riechstoffverbindungen, zum Beispiel die synthetischen Produkte vom Typ der Ester, Ether, Aldehyde, Ketone, Alkohole und Kohlenwasserstoffe verwendet werden. Riechstoffverbindungen vom Typ der Ester sind zum Beispiel Benzylacetat, Phenoxyethylisobutyrat, p-tert.-Butylcyclohexylacetat, Linalylacetat, Dimethylbenzyl-carbinylacetat, Phenylethylacetat, Linalylbenzoat, Benzylformiat, Ethylmethylphenyl-glycinat, Allylcyclohexylpropionat, Styrallylpropionat und Benzylsalicylat. Zu den Ethern zählen beispielsweise Benzylethylether, zu den Aldehyden zum Beispiel die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen zum Beispiel die Jonone, α-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, zum Beispiel Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelhotzöl. Üblicherweise liegt der Gehalt von Wasch- und Reinigungsmitteln an Farbstoffen unter 0,01 Gew.-%, während Duftstoffe bis zu 2 Gew.-% der gesamten Formulierung ausmachen können.

Die Duftstoffe können direkt in die Wasch- oder Reinigungsmittel eingearbeitet werden, es kann aber auch vorteilhaft sein, die Duftstoffe auf Träger aufzubringen, die die Haftung des Parfüms auf dem Reinigungsgut verstärken und durch eine langsamere Duftfreisetzung für langanhaltenden Duft, insbesondere von behandelten Textilien sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können. Ein weiter bevorzugter Träger für Duftstoffe ist der beschriebene Zeolith X, der anstelle von oder in Mischung mit Tensiden auch Duftstoffe aufnehmen kann. Bevorzugt sind daher Wasch- und Reinigungsmittel, die den beschriebenen Zeolith X und Duftstoffe, die vorzugsweise zumindest teilweise an dem Zeolithen absorbiert sind, enthalten.

Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Zur Bekämpfung von Mikroorganismen können Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung, die beispielsweise von K. H. Wallhäußer in "Praxis der Sterilisation, Desinfektion - Konservierung : Keimidentifizierung - Betriebshygiene" (5. Aufl. - Stuttgart; New York : Thieme, 1995) wiedergegeben wird, wobei alle dort beschriebenen Substanzen mit antimikrobieller Wirkung eingesetzt werden können. Geeignete antimikrobielle Wirkstoffe sind vorzugsweise ausgewählt aus den Gruppen der Alkohole, Amine, Aldehyde, antimikrobiellen Säuren beziehungsweise deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, lsothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propyl-butyl-carbamat, lod, lodophore, Peroxoverbindungen, Halogenverbindungen sowie beliebigen Gemischen der voranstehenden. Der antimikrobielle Wirkstoff kann dabei ausgewählt sein aus Ethanol, n-Propanol, i-Propanol, 1,3-Butandiol, Phenoxyethanol, 1,2-Propylenglykol, Glycerin, Undecylensäure, Benzoesäure, Salicylsäure, Dihydracetsäure, o-Phenylphenol, N-Methylmorpholinacetonitril (MMA), 2-Benzyl-4-chlorphenol, 2,2'-Methylen-bis-(6-brom-4-chlorphenol), 4,4'-Dichlor-2'-hydroxydiphenylether (Dichlosan), 2,4,4'-Trichlor-2'-hydroxydiphenylether (Trichlosan), Chlorhexidin, N-(4-Chlorphenyl)-N-(3,4-dichlorphenyl)-harnstoff, N,N'-(1,10-decan-diyldi-1-pyridinyl-4-yliden)-bis-(1-octanamin)-dihydrochlorid, N,N'-Bis-(4-chlorphenyl)-3, 12-diimino-2,4,11, 13-tetraaza-tetradecandiimidamid, Glucoprotaminen, antimikrobiellen oberflächenaktiven quaternären Verbindungen, Guanidinen einschl. den Bi- und Polyguanidinen, wie beispielsweise 1,6-Bis-(2-ethylhexyl-biguanido-hexan)-dihydrochlorid, 1,6-Di-(N₁, N₁'-phenyldiguanido-N₅, N₅')-hexan-tetrahydochlorid, 1,6-Di-(N₁,N₁'-phenyl-N₁,N₁-methyldiguanido-N₅,N₅')-hexan-dihydrochlorid, 1,6-Di-(N₁,N₁'-o-chlorophenyldiguanido- N₅,N₅')-hexan-dihydrochlorid, 1,6-Di-(N₁,N₁'-2,6-dichlorophenyldiguanido-N₅,N₅')hexan-dihydrochlorid, 1,6-Di-[N₁,N₁'-beta-(p-methoxyphenyl) diguanido-N₅,N₅']-hexane-dihydrochlorid, 1,6-Di-(N₁,N₁'-alpha-methyl-.beta.-phenyldiguanido-N₅,N₅')-hexan-dihydrochlorid, 1,6-Di-(N₁,N₁'-p-nitrophenyldiguanido-N₅,N₅')hexan-dihydrochlorid, omega:omega-Di-(N₁,N₁'-phenyldiguanido-N₅,N₅')-di-n-propylether-dihydrochlorid, omega:omega'-Di-(N₁,N₁'-p-chlorophenyldiguanido-N₅,N₅')-di-n-propylether-tetrahydrochlorid, 1,6-Di-(N₁,N₁'-2,4-dichlorophenyldiguanido-N₅,N₅')hexan-tetrahydrochlorid, 1,6-Di-(N₁,N₁'-p-methylphenyldiguanido- N₅,N₅')hexan-dihydrochlorid, 1,6-Di-(N₁,N₁'-2,4,5-trichlorophenyldiguanido-N₅,N₅')hexan-tetrahydrochlorid, 1 ,6-Di-[N₁,N₁'-alpha-(p-chlorophenyl) ethyldiguanido-N₅,N₅'] hexan-dihydrochlorid, omega:omega-Di-(N₁,N₁'-p-chlorophenyldiguanido-N₅,N₅')m-xylene-dihydrochlorid, 1,12-Di-(N₁,N₁'-p-chlorophenyldiguanido-N₅,N₅') dodecan-dihydrochlorid, 1,10-Di-(N₁,N₁'-phenyldiguanido-N₅,N₅')-decan-tetrahydrochlorid, 1,12-Di-(N₁,N₁'-phenyldiguanido- N₅,N₅') dodecantetrahydrochlorid, 1,6-Di-(N₁,N₁'-o-chlorophenyldiguanido- N₅,N₅') hexan-dihydrochlorid, 1,6-Di-(N₁,N₁'-o-chlorophenyldiguanido- N₅,N₅') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbiguanid), Ethylen-bis-(N-butylphenylbiguanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylen bis (o-tolylbiguanid), N-Butyl-trimethyle- bis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlormeta-xylol, sowie natürliche antimikrobielle Wirkstoffe pflanzlicher Herkunft (zum Beispiel aus Gewürzen oder Kräutern), tierischer sowie mikrobieller Herkunft. Vorzugsweise können antimikrobiell wirkende oberflächenaktive quaternäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/oder mindestens ein natürlicher antimikrobieller Wirkstoff tierischer Herkunft aus der Gruppe, umfassend Enzyme wie Eiweiß aus Milch, Lysozym und Lactoperoxidase, und/oder mindestens eine antimikrobiell wirkende oberflächenaktive quaternäre Verbindung mit einer Ammonium-, Sulfonium-, Phosphonium-, lodonium- oder Arsoniumgruppe, Peroxoverbindungen und Chlorverbindungen eingesetzt werden. Auch Stoffe mikrobieller Herkunft, sogenannte Bakteriozine, können eingesetzt werden.

Die als antimikrobielle Wirkstoffe geeigneten quaternären Ammoniumverbindungen (QAV) weisen die allgemeine Formel (R¹)(R²) (R³)(R⁴) N⁺ X⁻ auf, in der R¹ bis R⁴ gleiche oder verschiedene C₁-C₂₂-Alkylreste, C₇-C₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, zum Beispiel eine Pyridinium- oder lmidazoliniumverbindung, bilden, darstellen und X- Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

QAV sind durch Umsetzung tertiärer Amine mit Alkylierungsmitteln, wie zum Beispiel Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielweise Benzalkoniumchlorid (N-Alkyl-N,N-dimethyl-benzyl-ammoniumchlorid, CAS No. 8001-54-5), Benzalkon B (m,p-Dichlorbenzyl-dimethyl-C12-alkylammoniumchlorid, CAS No. 58390-78-6), Benzoxoniumchlorid (Benzyl-dodecyl-bis-(2-hydroxyethyl)-ammonium-chlorid), Cetrimoniumbromid (N-Hexadecyl-N,N-trimethylammoniumbromid, CAS No. 57-09-0), Benzetoniumchlorid (N,N-Dimethyl-N-[2-[2-[p-(1,1,3,3-tetramethylbutyl)-pheno-xy]ethoxy]ethyl]-benzylammoniumchlorid, CAS No. 121-54-0), Dialkyldimethylammonium-chloride wie Di-n-decyl-dimethyl-ammoniumchlorid (CAS No. 7173-51-5-5), Didecyldi-methylammoniumbromid (CAS No. 2390-68-3), Dioctyl-dimethyl-ammoniumchloric, 1-Cetylpyridiniumchlorid (CAS No. 123-03-5) und Thiazoliniodid (CAS No. 15764-48-1) sowie deren Mischungen. Besonders bevorzugte QAV sind die Benzalkoniumchloride mit C₈-C₁₈-Alkylresten, insbesondere C₁₂-C₁₄-Aklylbenzyl-dimethyl-ammoniumchlorid.

Benzalkoniumhalogenide und/oder substituierte Benzalkoniumhalogenide sind beispielsweise kommerziell erhältlich als Barquat^{®} ex Lonza, Marquat^{®} ex Mason, Variquat^{®} ex Witco/ Sherex und Hyamine^{®} ex Lonza, sowie Bardac^{®} ex Lonza. Weitere kommerziell erhältliche antimikrobielle Wirkstoffe sind N-(3-Chlorallyl)-hexaminiumchlorid wie Dowicide^{®} und Dowicil^{®} ex Dow, Benzethoniumchlorid wie Hyamine^{®} 1622 ex Rohm & Haas, Methylbenzethoniumchlorid wie Hyamine^{®} 10X ex Rohm & Haas, Cetylpyridiniumchlorid wie Cepacolchlorid ex Merrell Labs.

Die antimikrobiellen Wirkstoffe werden in Mengen von 0,0001 Gew.-% bis 1 Gew.-%, bevorzugt von 0,001 Gew.-% bis 0,8 Gew.-%, besonders bevorzugt von 0,005 Gew.-% bis 0,3 Gew.-% und insbesondere von 0,01 bis 0,2 Gew.-% eingesetzt.

Die erfindungsgemäßen Wasch- oder Reinigungsmittel können UV-Absorbenzien (UV-Absorber) enthalten, die auf die behandelten Textilien aufziehen und die Lichtbeständigkeit der Fasern und/oder die Lichtbeständigkeit sonstiger Rezepturbestandteile verbessern. Unter UV-Absorber sind organische Substanzen (Lichtschutzfilter) zu verstehen, die in der Lage sind, ultraviolette Strahlen zu absorbieren und die aufgenommene Energie in Form längerwelliger Strahlung, zum Beispiel Wärme wieder abzugeben.

Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate, gegebenenfalls mit Cyanogruppen in 2-Stellung), Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise in der EP 0728749 A beschrieben werden und kommerziell als Tinosorb^{®} FD oder Tinosorb^{®} FR ex Ciba erhältlich sind. Als UV-B-Absorber sind zu nennen: 3-Benzylidencampher beziehungsweise 3-Benzylidennorcampher und dessen Derivate, zum Beispiel 3-(4-Methylbenzyliden)campher, wie in der EP 0693471 B1 beschrieben; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie zum Beispiel 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB); Propan-1,3-dione, wie zum Beispiel 1-(4-tert.Butylphenyl)-3-(4'methoxyphenyl)propan-1,3-dion; Ketotricyclo(5.2.1.0)decan-Derivate, wie in der EP 0694521 B1 beschrieben. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie zum Beispiel 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide beziehungsweise Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, das heißt hydrophilisiert oder hydrophobiert vorliegen. Typische Beispiele sind ummantelte Titandioxide, wie zum Beispiel Titandioxid T 805 (Degussa) oder Eusolex® T2000 (Merck; als hydrophobe Coatingmittel kommen dafür bevorzugt Silicone und besonders bevorzugt Trialkoxyoctylsilane oder Simethicone in Frage. Vorzugsweise wird mikronisiertes Zinkoxid verwendet. Weitere geeignete UV-Lichtschutzfilter sind der Übersicht von P. Finkel in SÖFW-Journal 122 (1996), S. 543 zu entnehmen.

Die UV-Absorbenzien werden üblicherweise in Mengen von 0,01 Gew.-% bis 5 Gew.-%, vorzugsweise von 0,03 Gew.-% bis 1 Gew.-%, eingesetzt.

Erfindungsgemäße Mittel können zur Steigerung der Wasch-, beziehungsweise Reinigungsleistung weitere Enzyme enthalten, wobei prinzipiell alle im Stand der Technik für diese Zwecke etablierten Enzyme einsetzbar sind. Hierzu gehören insbesondere weitere Proteasen, Amylasen, Lipasen, Hemicellulasen, Cellulasen oder Oxidoreduktasen, sowie vorzugsweise deren Gemische. Diese Enzyme sind im Prinzip natürlichen Ursprungs; ausgehend von den natürlichen Molekülen stehen für den Einsatz in Wasch- und Reinigungsmitteln verbesserte Varianten zur Verfügung, die entsprechend bevorzugt eingesetzt werden. Erfindungsgemäße Mittel enthalten Enzyme vorzugsweise in Gesamtmengen von 1 x 10⁻⁸ bis 5 Gewichts-Prozent bezogen auf aktives Protein.

Unter den weiteren Proteasen sind solche vom Subtilisin-Typ bevorzugt. Beispiele hierfür sind die Subtilisine BPN' und Carlsberg, die Protease PB92, die Subtilisine 147 und 309, die Alkalische Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase^{®} von der Firma Novozymes A/S, Bagsvaerd, Dänemark, erhältlich. Die Subtilisine 147 und 309 werden unter den Handelsnamen Esperase^{®}, beziehungsweise Savinase^{®} von der Firma Novozymes vertrieben. Von der Protease aus *Bacillus lentus* DSM 5483 (WO 91/02792 A1) leiten sich die unter der Bezeichnung BLAP^{®} geführten Varianten ab, die insbesondere in WO 92/21760 A1, WO 95/23221 A1, WO 02/088340 A2 und WO 03/038082 A2 beschrieben werden. Weitere verwendbare Proteasen aus verschiedenen *Bacillus sp.* und *B. gibsonii* gehen aus den einleitend bereits erwähnten Patentanmeldungen WO 03/054185 A1, WO 03/056017 A2, WO 03/055974 A2 und WO 03/054184 A1 hervor.

Weitere brauchbare Proteasen sind beispielsweise die unter den Handelsnamen Durazym^{®}, Relase^{®}, Everlase^{®}, Nafizym, Natalase^{®}, Kannase^{®} und Ovozymes^{®} von der Firma Novozymes, die unter den Handelsnamen, Purafect^{®}, Purafect^{®} OxP und Properase^{®} von der Firma Genencor, das unter dem Handelsnamen Protosol^{®} von der Firma Advanced Biochemicals Ltd., Thane, Indien, das unter dem Handelsnamen Wuxi^{®} von der Firma Wuxi Snyder Bioproducts Ltd., China, die unter den Handelsnamen Proleather^{®} und Protease P^{®} von der Firma Amano Pharmaceuticals Ltd., Nagoya, Japan, und das unter der Bezeichnung Proteinase K-16 von der Firma Kao Corp., Tokyo, Japan, erhältlichen Enzyme.

Beispiele für erfindungsgemäß einsetzbare Amylasen sind die α-Amylasen aus *Bacillus licheniformis,* aus *B. amyloliquefaciens* oder aus *B. stearothermophilus* sowie deren für den Einsatz in Wasch- und Reinigungsmitteln verbesserte Weiterentwicklungen. Das Enzym aus *B. licheniformis* ist von der Firma Novozymes unter dem Namen Termamyl^{®} und von der Firma Genencor unter dem Namen Purastar^{®}ST erhältlich.

Weiterentwicklungsprodukte dieser α-Amylase sind von der Firma Novozymes unter den Handelsnamen Duramyl^{®} und Termamyl^{®}ultra, von der Firma Genencor unter dem Namen Purastar^{®}OxAm und von der Firma Daiwa Seiko Inc., Tokyo, Japan, als Keistase^{®} erhältlich. Die α-Amylase von *B. amyloliquefaciens* wird von der Firma Novozymes unter dem Namen BAN^{®} vertrieben, und abgeleitete Varianten von der α-Amylase aus *B. stearothermophilus* unter den Namen BSG^{®} und Novamyl^{®}, ebenfalls von der Firma Novozymes.

Desweiteren sind für diesen Zweck die in der Anmeldung WO 02/10356 A2 offenbarte α-Amylase aus *Bacillus sp.* A 7-7 (DSM 12368) und die in der Anmeldung WO 02/44350 A2 beschriebene Cyclodextrin-Glucanotransferase (CGTase) aus *B. agaradherens* (DSM 9948) hervorzuheben. Ferner sind die amylolytischen Enzyme einsetzbar, die dem Sequenzraum von α-Amylasen angehören, der in der Anmeldung WO 03/002711 A2 definiert wird, und die, die in der Anmeldung WO 03/054177 A2 beschrieben werden. Ebenso sind Fusionsprodukte der genannten Moleküle einsetzbar, beispielsweise die aus der Anmeldung DE 10138753 A1.

Darüber hinaus sind die unter den Handelsnamen Fungamyl^{®} von der Firma Novozymes erhältlichen Weiterentwicklungen der α-Amylase aus *Aspergillus niger* und A. *oryzae* geeignet. Ein weiteres Handelsprodukt ist beispielsweise die Amylase-LT^{®}.

Erfindungsgemäße Mittel können Lipasen oder Cutinasen, insbesondere wegen ihrer Triglycerid-spaltenden Aktivitäten enthalten, aber auch, um aus geeigneten Vorstufen in situ Persäuren zu erzeugen. Hierzu gehören beispielsweise die ursprünglich aus *Humicola lanuginosa (Thermomyces lanuginosus)* erhältlichen, beziehungsweise weiterentwickelten Lipasen, insbesondere solche mit dem Aminosäureaustausch D96L. Sie werden beispielsweise von der Firma Novozymes unter den Handelsnamen Lipolase^{®}, Lipolase^{®}Ultra, LipoPrime^{®}, Lipozyme^{®} und Lipex^{®} vertrieben. Desweiteren sind beispielsweise die Cutinasen einsetzbar, die ursprünglich aus *Fusarium solani pisi* und *Humicola insolens* isoliert worden sind. Ebenso brauchbare Lipasen sind von der Firma Amano unter den Bezeichnungen Lipase CE^{®}, Lipase P^{®}, Lipase B^{®}, beziehungsweise Lipase CES^{®}, Lipase AKG^{®}, Bacillis sp. Lipase^{®}, Lipase AP^{®}, Lipase M-AP^{®} und Lipase AML^{®} erhältlich. Von der Firma Genencor sind beispielsweise die Lipasen, beziehungsweise Cutinasen einsetzbar, deren Ausgangsenzyme ursprünglich aus *Pseudomonas mendocina* und *Fusarium solanii* isoliert worden sind. Als weitere wichtige Handelsprodukte sind die ursprünglich von der Firma Gist-Brocades vertriebenen Präparationen M1 Lipase^{®} und Lipomax^{®} und die von der Firma Meito Sangyo KK, Japan, unter den Namen Lipase MY-30^{®}, Lipase OF^{®} und Lipase PL^{®} vertriebenen Enzyme zu erwähnen, ferner das Produkt Lumafast^{®} von der Firma Genencor.

Erfindungsgemäße Mittel können, insbesondere wenn sie für die Behandlung von Textilien gedacht sind, Cellulasen enthalten, je nach Zweck als reine Enzyme, als Enzympräparationen oder in Form von Mischungen, in denen sich die einzelnen Komponenten vorteilhafterweise hinsichtlich ihrer verschiedenen Leistungsaspekte ergänzen. Zu diesen Leistungsaspekten zählen insbesondere Beiträge zur Primärwaschleistung, zur Sekundärwaschleistung des Mittels (Antiredepositionswirkung oder Vergrauungsinhibition) und Avivage (Gewebewirkung), bis hin zum Ausüben eines "stone washed"-Effekts.

Eine brauchbare pilzliche, Endoglucanase(EG)-reiche Cellulase-Präparation, beziehungsweise deren Weiterentwicklungen werden von der Firma Novozymes unter dem Handelsnamen Celluzyme^{®} angeboten. Die ebenfalls von der Firma Novozymes erhältlichen Produkte Endolase^{®} und Carezyme^{®} basieren auf der 50 kD-EG, beziehungsweise der 43 kD-EG aus *H. insolens* DSM 1800. Weitere einsetzbare Handelsprodukte dieser Firma sind Cellusoft^{®} und Renozyme^{®}. Letzteres basiert auf der Anmeldung WO 96/29397 A1. Leistungsverbesserte Cellulase-Varianten gehen beispielsweise aus der Anmeldung WO 98112307 A1 hervor. Ebenso sind die in der Anmeldung WO 97/14804 A1 offenbarten Cellulasen einsetzbar; beispielsweise die darin offenbarte 20 kD-EG aus *Melanocarpus,* die von der Firma AB Enzymes, Finnland, unter den Handelsnamen Ecostone^{®} und Biotouch^{®} erhältlich ist. Weitere Handelprodukte der Firma AB Enzymes sind Econase^{®} und Ecopulp^{®}. Weitere geeignete Cellulasen aus *Bacillus sp.* CBS 670.93 und CBS 669.93 werden in WO 96/34092 A2 offenbart, wobei die aus *Bacillus sp.* CBS 670.93 von der Firma Genencor unter dem Handelsnamen Puradax^{®} erhältlich ist. Weitere Handelsprodukte der Firma Genencor sind "Genencor detergent cellulase L" und IndiAge^{®}Neutra.

Erfindungsgemäße Mittel können insbesondere zur Entfernung bestimmter Problemanschmutzungen weitere Enzyme enthalten, die unter dem Begriff Hemicellulasen zusammengefaßt werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullulanasen und β-Glucanasen. Geeignete Mannanasen sind beispielsweise unter den Namen Gamanase^{®} und Pektinex AR^{®} von der Firma Novozymes, unter dem Namen Rohapec^{®} B1L von der Firma AB Enzymes und unter dem Namen Pyrolase^{®} von der Firma Diversa Corp., San Diego, CA, USA erhältlich. Eine geeignete β-Glucanase aus einem *B. alcalophilus* geht beispielsweise aus der Anmeldung WO 99/06573 A1 hervor. Die aus *B. subtilis* gewonnene β-Glucanase ist unter dem Namen Cereflo^{®} von der Firma Novozymes erhältlich.

Zur Erhöhung der bleichenden Wirkung können erfindungsgemäße Wasch- und Reinigungsmittel Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) enthalten. Als geeignete Handelsprodukte sind Denilite^{®} 1 und 2 der Firma Novozymes zu nennen. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluß zu gewährleisten (Mediatoren).

Die in erfindungsgemäßen Mitteln eingesetzten Enzyme stammen entweder ursprünglich aus Mikroorganismen, etwa der Gattungen *Bacillus, Streptomyces, Humicola,* oder *Pseudomonas,* und/oder werden nach an sich bekannten biotechnologischen Verfahren durch geeignete Mikroorganismen produziert, etwa durch transgene Expressionswirte der Gattungen *Bacillus* oder filamentöse Fungi.

Die Aufreinigung der betreffenden Enzyme erfolgt günstigerweise über an sich etablierte Verfahren, beispielsweise über Ausfällung, Sedimentation, Konzentrierung, Filtration der flüssigen Phasen, Mikrofiltration, Ultrafiltration, Einwirken von Chemikalien, Desodorierung oder geeignete Kombinationen dieser Schritte.

Erfindungsgemäßen Mitteln können die Enzyme in jeder nach dem Stand der Technik etablierten Form zugesetzt werden. Hierzu gehören beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem, vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so daß ein einzelnes Granulat mehrere Enzymaktivitäten aufweist.

Ein in einem erfindungsgemäßen Mittel enthaltenes Protein und/oder Enzym kann besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung der Proteine und/oder Enzyme ist eine Inhibierung der Proteolyse besonders bevorzugt, insbesondere wenn auch die Mittel Proteasen enthalten. Bevorzugte erfindungsgemäße Mittel enthalten zu diesem Zweck Stabilisatoren.

Eine Gruppe von Stabilisatoren sind reversible Proteaseinhibitoren. Häufig werden hierfür Benzamidin-Hydrochlorid, Borax, Borsäuren, Boronsäuren oder deren Salze oder Ester eingesetzt, darunter vor allem Derivate mit aromatischen Gruppen, etwa ortho-, meta- oder para-substituierte Phenylboronsäuren, insbesondere 4-Formylphenyl-Boronsäure, beziehungsweise die Salze oder Ester der genannten Verbindungen. Auch Peptidaldehyde, das heißt Oligopeptide mit reduziertem C-Terminus, insbesondere solche aus 2 bis 50 Monomeren werden zu diesem Zweck eingesetzt. Zu den peptidischen reversiblen Proteaseinhibitoren gehören unter anderem Ovomucoid und Leupeptin. Auch spezifische, reversible Peptid-Inhibitoren für die Protease Subtilisin sowie Fusionsproteine aus Proteasen und spezifischen Peptid-Inhibitoren sind hierfür geeignet.

Weitere Enzymstabilisatoren sind Aminoalkohole wie Mono-, Di-, Triethanol- und -Propanolamin und deren Mischungen, aliphatische Carbonsäuren bis zu C₁₂, wie beispielsweise Bernsteinsäure, andere Dicarbonsäuren oder Salze der genannten Säuren. Auch endgruppenverschlossene Fettsäureamidalkoxylate sind für diesen Zweck geeignet. Bestimmte als Builder eingesetzte organische Säuren vermögen, wie in WO 97/18287 offenbart, zusätzlich ein enthaltenes Enzym zu stabilisieren.

Niedere aliphatische Alkohole, vor allem aber Polyole, wie beispielsweise Glycerin, Ethylenglykol, Propylenglykol oder Sorbit sind weitere häufig eingesetzte Enzym-stabilisatoren. Auch Di-Glycerinphosphat schützt gegen Denaturierung durch physikalische Einflüsse. Ebenso werden Calcium- und/oder Magnesiumsalze eingesetzt, wie beispielsweise Calciumacetat oder Calcium-Formiat.

Polyamid-Oligomere oder polymere Verbindungen wie Lignin, wasserlösliche Vinyl-Copolymere oder Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere wirken gleichzeitig als Enzymstabilisatoren und als Farbübertragungsinhibitoren. Andere polymere Stabilisatoren sind lineare C₈-C₁₈ Polyoxyalkylene. Auch Alkylpolyglycoside können die enzymatischen Komponenten des erfindungsgemäßen Mittels stabilisieren und vermögen vorzugsweise, diese zusätzlich in ihrer Leistung zu steigern. Vernetzte N-haltige Verbindungen erfüllen vorzugsweise eine Doppelfunktion als Soil-release-Agentien und als Enzym-Stabilisatoren. Hydrophobes, nichtionisches Polymer stabilisiert insbesondere eine gegebenenfalls enthaltene Cellulase.

Reduktionsmittel und Antioxidantien erhöhen die Stabilität der Enzyme gegenüber oxidativem Zerfall; hierfür sind beispielsweise schwefelhaltige Reduktionsmittel geläufig. Andere Beispiele sind Natrium-Sulfit und reduzierende Zucker.

Besonders bevorzugt werden Kombinationen von Stabilisatoren eingesetzt, beispielsweise aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen. Die Wirkung von Peptid-Aldehyd-Stabilisatoren wird günstigerweise durch die Kombination mit Borsäure und/oder Borsäurederivaten und Polyolen gesteigert und noch weiter durch die zusätzliche Wirkung von zweiwertigen Kationen, wie zum Beispiel Calcium-lonen.

Da erfindungsgemäße Mittel in allen denkbaren Formen angeboten werden können, stellen erfindungsgemäße Enzyme, beziehungsweise Proteine in allen für die Zugabe zu den jeweiligen Mitteln zweckmäßigen Formulierungen jeweilige Ausführungsformen der vorliegenden Erfindung dar. Dazu gehören beispielsweise flüssige Formulierungen, feste Granulate oder Kapseln.

Die verkapselte Form bietet sich an, um die Enzyme oder andere Inhaltsstoffe vor anderen Bestandteilen, wie beispielsweise Bleichmitteln, zu schützen oder um eine kontrollierte Freisetzung (controlled release) zu ermöglichen. Je nach der Größe dieser Kapseln wird nach Milli-, Mikro- und Nanokapseln unterschieden, wobei Mikrokapseln für Enzyme besonders bevorzugt sind. Solche Kapseln werden beispielsweise mit den Patentanmeldungen WO 97/24177 und DE 19918267 offenbart. Eine mögliche Verkapselungsmethode besteht darin, daß die Proteine, ausgehend von einer Mischung der Proteinlösung mit einer Lösung oder Suspension von Stärke oder einem Stärkederivat, in dieser Substanz verkapselt werden. Ein solches Verkapselungsverfahren wird mit der Anmeldung WO 01/38471 beschrieben.

Im Fall fester Mittel können die Proteine beispielsweise in getrockneter, granulierter und/oder verkapselter Form eingesetzt werden. Sie können separat, das heißt als eigene Phase, oder mit anderen Bestandteilen zusammen in derselben Phase, mit oder ohne Kompaktierung zugesetzt werden. Sollen mikroverkapselte Enzyme in fester Form verarbeitet werden, so kann das Wasser mit aus dem Stand der Technik bekannten Verfahren aus den sich aus der Aufarbeitung ergebenden wäßrigen Lösungen entfernt werden, wie Sprühtrocknung, Abzentrifugieren oder durch Umsolubilisieren. Die auf diese Weise erhaltenen Teilchen haben üblicherweise eine Teilchengröße zwischen 50 und 200 µm.

Flüssigen, gelförmigen oder pastösen erfindungsgemäßen Mitteln können die Enzyme, und auch das erfindungsgemäße Protein ausgehend von einer nach dem Stand der Technik durchgeführten Proteingewinnung und Präparation in konzentrierter wäßriger oder nichtwäßriger Lösung, Suspension oder Emulsion zugesetzt werden, aber auch in Gelform oder verkapselt oder als getrocknetes Pulver. Derartige erfindungsgemäße Wasch- oder Reinigungsmittel werden in der Regel durch einfaches Mischen der Inhaltsstoffe hergestellt, die in Substanz oder als Lösung in einen automatischen Mischer gegeben werden können.

Neben der primären Waschleistung können die in Waschmitteln enthaltenen Proteasen ferner die Funktion erfüllen, andere enzymatische Bestandteile durch proteolytische Spaltung zu aktivieren oder nach entsprechender Einwirkzeit zu inaktivieren, so wie beispielsweise in den Anmeldungen WO 94/29426 oder EP 747471 offenbart worden ist. Vergleichbare regulatorische Funktionen sind auch über das erfindungsgemäße Protein möglich. Eine Ausführungsform der vorliegenden Erfindung sind ferner solche Mittel mit Kapseln aus proteasesensitivem Material, welche beispielsweise von erfindungsgemäßen Proteinen zu einem beabsichtigten Zeitpunkt hydrolysiert werden und ihren Inhalt freisetzen. Ein vergleichbarer Effekt kann auch bei anderen mehrphasigen Mitteln erzielt werden.

Eine weitere Ausführungsform stellen Mittel zur Behandlung von Textilrohstoffen oder zur Textilpflege dar, die eine erfindungsgemäße Alkalische Protease enthalten.

Eine weitere Ausführungsform stellen Mittel zur Behandlung von Fasern oder Textilien mit natürlichen Bestandteilen dar, insbesondere von solchen mit Wolle oder Seide.

Denn insbesondere natürliche Fasern, wie beispielsweise Wolle oder Seide, zeichnen sich durch eine charakteristische, mikroskopische Oberflächenstruktur aus. Diese kann, wie am Beispiel der Wolle im Artikel von R. Breier in Melliand Textilberichte vom 1.4.2000 (Seite 263) ausgeführt worden ist, langfristig zu unerwünschten Effekten, wie etwa Verfilzung führen. Zur Vermeidung solcher Effekte werden die natürlichen Rohstoffe mit erfindungsgemäßen Mitteln behandelt, welche beispielsweise dazu beitragen, die auf Proteinstrukturen beruhende geschuppte Oberflächenstruktur zu glätten und damit einem Verfilzen entgegenwirken.

In einer bevorzugten Ausführungsform ist das Mittel mit einer erfindungsgemäßen Protease so konzipiert, daß es regelmäßig als Pflegemittel verwendet werden kann, beispielsweise indem es dem Waschprozeß zugesetzt, nach dem Waschen angewendet oder unabhängig von dem Waschen appliziert wird. Der gewünschte Effekt besteht darin, eine glatte Oberflächenstruktur des Textils über einen langen Zeitraum zu erhalten und/oder Schädigungen des Gewebes vorzubeugen und/oder zu verringern.

Einen eigenen Erfindungsgegenstand stellen Verfahren zur maschinellen Reinigung von Textilien oder von harten Oberflächen dar, bei denen wenigstens in einem der Verfahrensschritte eine erfindungsgemäße Alkalische Protease aktiv wird.

Darunter sind solche Verfahren bevorzugt, bei denen die erfindungsgemäße Alkalische Protease in einer Menge von 40 µg bis 4 g, vorzugsweise von 50 µg bis 3 g, besonders bevorzugt von 100 µg bis 2 g und ganz besonders bevorzugt von 200 µg bis 1 g pro Anwendung eingesetzt wird. Eingeschlossen werden alle ganzzahligen und nichtganzzahligen jeweils zwischen diesen Zahlen liegenden Werte.

Hierunter fallen sowohl manuelle als auch maschinelle Verfahren, wobei maschinelle Verfahren aufgrund ihrer präziseren Steuerbarkeit, was beispielsweise die eingesetzten Mengen und Einwirkzeiten angeht, bevorzugt sind.

Verfahren zur Reinigung von Textilien zeichnen sich im allgemeinen dadurch aus, daß in mehreren Verfahrensschritten verschiedene reinigungsaktive Substanzen auf das Reinigungsgut aufgebracht und nach der Einwirkzeit abgewaschen werden, oder daß das Reinigungsgut in sonstiger Weise mit einem Waschmittel oder einer Lösung dieses Mittels behandelt wird. Das gleiche gilt für Verfahren zur Reinigung von allen anderen Materialien als Textilien, welche unter dem Begriff harte Oberflächen zusammengefaßt werden. Alle denkbaren Wasch- oder Reinigungsverfahren können in wenigstens einem der Verfahrensschritte um erfindungsgemäße Proteine bereichert werden, und stellen dann Ausführungsformen der vorliegenden Erfindung dar.

Da bevorzugte erfindungsgemäße Enzyme natürlicherweise bereits eine proteinauflösende Aktivität besitzen und diese auch in Medien entfalten, die sonst keine Reinigungskraft besitzen, wie beispielsweise in bloßem Puffer, kann ein einzelner Teilschritt eines solchen Verfahrens zur maschinellen Reinigung von Textilien darin bestehen, daß gewünschtenfalls neben stabilisierenden Verbindungen, Salzen oder Puffersubstanzen als einzige reinigungsaktive Komponente ein erfindungsgemäßes Enzym aufgebracht wird. Dies stellt eine besonders bevorzugte Ausführungsform der vorliegenden Erfindung dar.

In einer weiteren bevorzugten Ausführungsform solcher Verfahren werden die betreffenden erfindungsgemäßen Alkalischen Protease im Rahmen einer der oben ausgeführten Rezepturen für erfindungsgemäße Mittel, vorzugsweise erfindungsgemäße Wasch-, beziehungsweise Reinigungsmittel bereitgestellt.

Bevorzugte Ausführungsformen dieses Erfindungsgegenstands stellen Verfahren zur Behandlung von Textilrohstoffen oder zur Textilpflege dar, bei denen in wenigstens einem der Verfahrensschritte eine erfindungsgemäße Alkalische Protease aktiv wird.

Hierunter sind Verfahren für Textilrohstoffe, Fasern oder Textilien mit natürlichen Bestandteilen bevorzugt, und ganz besonders für solche mit Wolle oder Seide.

Es kann sich dabei beispielsweise um Verfahren handeln, in denen Materialien zur Verarbeitung in Textilien vorbereitet werden, etwa zur Antifilzausrüstung, oder beispielsweise um Verfahren, welche die Reinigung getragener Textilien um eine pflegende Komponente bereichern. Wegen der oben beschriebenen Wirkung von Proteasen auf natürliche, proteinhaltige Rohstoffe handelt es sich in bevorzugten Ausführungsformen um Verfahren zur Behandlung von Textilrohstoffen, Fasern oder Textilien mit natürlichen Bestandteilen, insbesondere mit Wolle oder Seide.

Einen eigenen Erfindungsgegenstand stellt die Verwendung einer oben beschriebenen erfindungsgemäßen Alkalische Protease zur Reinigung von Textilien oder von harten Oberflächen dar.

Entsprechend bevorzugt gelten für diese Verwendungen die oben ausgeführten Konzentrationsbereiche.

Denn erfindungsgemäße Proteasen können, insbesondere entsprechend den oben beschriebenen Eigenschaften und den oben beschriebenen Verfahren dazu verwendet werden, um von Textilien oder von harten Oberflächen proteinhaltige Verunreinigungen zu beseitigen. Ausführungsformen stellen beispielsweise die Handwäsche, die manuelle Entfernung von Flecken von Textilien oder von harten Oberflächen oder die Verwendung im Zusammenhang mit einem maschinellen Verfahren dar.

In einer bevorzugten Ausführungsform dieser Verwendung werden die betreffenden erfindungsgemäßen Alkalischen Proteasen im Rahmen einer der oben ausgeführten Rezepturen für erfindungsgemäße Mittel, vorzugsweise Wasch-, beziehungsweise Reinigungsmittel bereitgestellt.

Eine weitere Ausführungsform dieses Erfindungsgegenstands stellt die Verwendung einer erfindungsgemäßen Alkalischen Protease zur Aktivierung oder Deaktivierung von Inhaltsstoffen von Wasch- oder Reinigungsmitteln dar.

Denn wie bekannt ist, können Protein-Bestandteile von Wasch- oder Reinigungsmitteln durch das Einwirken einer Protease inaktiviert werden. Diesen ansonsten eher unerwünschten Effekt gezielt einzusetzen, ist ein Gegenstand der vorliegenden Erfindung. Ebenso ist es wie oben beschrieben möglich, daß durch Proteolyse eine andere Komponente erst aktiviert wird, etwa, wenn sie ein Hybridprotein aus dem eigentlichen Enzym und dem dazu passenden Inhibitor darstellt, wie dies beispielsweise in der Anmeldung WO 00/01831 A2 offenbart worden ist. Ein anderes Beispiel für eine solche Regulation ist die, bei der eine aktive Komponente zum Schutz oder zur Kontrolle seiner Aktivität in einem Material verkapselt vorliegt, das durch Proteolyse angegriffen wird. Erfindungsgemäße Proteine können somit zu Inaktivierungs-, Aktivierungs- oder Freisetzungsreaktionen verwendet werden, insbesondere in mehrphasigen Mitteln.

Entsprechend dem oben Gesagten stellen auch folgende Verwendungen Ausführungsformen der vorliegenden Erfindung dar:
- Die Verwendung einer erfindungsgemäßen Alkalischen Protease zur Gewinnung oder Behandlung von Rohmaterialien oder Zwischenprodukten in der Textilherstellung, insbesondere zum Entfernen von Schutzschichten auf Geweben;
- die Verwendung einer erfindungsgemäßen Alkalischen Protease zur Behandlung von Textilrohstoffen oder zur Textilpflege und hierunter bevorzugt
- die entsprechende Verwendung für Textilrohstoffe, Fasern oder Textilien mit natürlichen Bestandteilen und ganz besonders für solche mit Wolle oder Seide.

Die vorliegende Erfindung wird auch in Form von solchen eine erfindungsgemäße Alkalische Protease enthaltenden Mitteln verwirklicht, bei denen es sich um Kosmetika handelt. Hierunter werden alle Arten von reinigenden und pflegenden Mitteln für menschliche Haut oder Haar verstanden, insbesondere reinigende Mittel.

Denn Proteasen spielen auch im Zellerneuerungsprozeß der menschlichen Haut (Desquamation) eine entscheidende Rolle (T. Egelrud et al., Acta Derm. Venerol., Band 71 (1991), Seiten 471-474). Dementsprechend werden Proteasen auch als bioaktive Komponenten in Hautpflegemitteln verwendet, um den Abbau der in trockener Haut vermehrten Desmosomenstrukturen zu unterstützen. Der Einsatz von Subtilisin-Proteasen mit Aminosäureaustauschen in den Positionen R99G/A/S, S154D/E und/oder L211 D/E für kosmetische Zwecke wird beispielsweise in WO 97/07770 A1 beschrieben. Entsprechend dem oben Gesagten können erfindungsgemäße Proteasen über die entsprechenden Punktmutationen weiterentwickelt werden. Somit eignen sich auch erfindungsgemäße Proteasen, insbesondere solche, die etwa nach Mutagenese oder durch Zugabe entsprechender, mit ihnen wechselwirkender Stoffe in ihrer Aktivität kontrolliert sind, als aktive Komponenten in Haut- oder Haar-Reinigungs- oder Pflegemitteln. Besonders bevorzugt sind solche Präparationen dieser Enzyme, die wie oben beschrieben, beispielsweise durch Kopplung an makromolekulare Träger (vergleiche US 5230891) stabilisiert und/oder durch Punktmutationen an hochallergenen Positionen derivatisiert sind, so daß sie für den Menschen eine höhere Hautverträglichkeit aufweisen.

Dementsprechend werden auch entsprechende kosmetische Reinigungs- und Pflegeverfahren und die Verwendung derartiger proteolytischer Enzyme zu kosmetischen Zwecken in diesen Erfindungsgegenstand einbezogen, insbesondere in entsprechenden Mitteln, wie beispielsweise Shampoos, Seifen oder Waschlotionen, oder in Pflegemitteln, die beispielsweise in Form von Cremes angeboten werden. Auch die Verwendung in einem schälenden Arzneimittel, beziehungsweise zu dessen Herstellung ist in diesen Anspruch eingeschlossen.

Neben dem Einsatz in Wasch- und Reinigungsmitteln und Kosmetika sind im Stand der Technik zahlreiche Anwendungsmöglichkeiten von Proteasaen, insbesondere Subtilasen etabliert. Einen Überblick hierüber bietet beispielsweise das Handbuch "Industrial enyzmes and their applications" von H. Uhlig, Wiley-Verlag, New York, 1998. All diese Techniken können erfindungsgemäße Alkalischen Proteasen bereichert werden. Sollte sich herausstellen, daß sie durch den Einsatz erfindungsgemäßer Proteasen weiterentwickelt werden können, so sind diese in den Schutzbereich der vorliegenden Erfindung eingeschlossen. Hierzu gehören insbesondere folgende Einsatzgebiete:
- die Verwendung einer erfindungsgemäßen Alkalischen Proteasen zur biochemischen Analyse oder zur Synthese von niedermolekularen Verbindungen oder von Proteinen;
- darunter bevorzugt die Verwendung zur Endgruppenbestimmung im Rahmen einer Peptid-Sequenzanalyse;
- die Verwendung einer erfindungsgemäßen Alkalischen Proteasen zur Präparation, Reinigung oder Synthese von Naturstoffen oder biologischen Wertstoffen, vorzugsweise im Rahmen entsprechender Mittel oder Verfahren;
- die Verwendung einer erfindungsgemäßen Alkalischen Proteasen zur Synthese von Proteinen oder anderen niedermolekularen chemischen Verbindungen;
- die Verwendung einer erfindungsgemäßen Alkalischen Proteasen zur Behandlung von natürlichen Rohstoffen, insbesondere zur Oberflächenbehandlung, ganz besonders in einem Verfahren zur Behandlung von Leder, vorzugsweise im Rahmen entsprechender Mittel oder Verfahren;
- die Verwendung einer erfindungsgemäßen Alkalischen Proteasen zur Behandlung von photographischen Filmen, insbesondere zur Entfernung von gelatinhaltigen oder ähnlichen Schutzschichten; und
- die Verwendung einer erfindungsgemäßen Alkalischen Proteasen zur Herstellung von Lebensmitteln oder von Futtermitteln.

Die nachfolgenden Beispiele erläutern die vorliegende Erfindung, ohne sie darauf einzuschränken.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme und Baukästen (Kits) wurden nach den Angaben der jeweiligen Hersteller eingesetzt.

### Beispiel 1

### Gewinnung von Zellmaterial aus Bodenhabitaten

Bodenproben wurden von verschiedenen Standorten in Deutschland genommen, in Wasser aufgenommen und Schwebstoffe durch 30minütiges Stehenlassen sedimentiert. Der Überstand wurde auf 5%igen Agarplatten mit HSP10-Festmedium (0,1 g Hefeextrakt, Fa. Difco, Heidelberg; 0,1 g Casein-Pepton, tryptisch verdaut, Fa. Difco; 0,1 g lösliche Stärke (Fa. Merck, Best.-Nr. 1.01251); 2 g Na₂CO₃; ad 1000 ml destilliertes Wasser; pH10) ausplattiert und für ca. 2 Wochen bei 30°C kultiviert. Der erhaltene Bakterien-Rasen wurde mechanisch von der Agaroberfläche gewonnen.

### Beispiel 2

### Anlage einer Expressions-Genbank

Als Expressionssystem wurde der Vektor pUC18 (GenBank, National Institutes of Health, Bethesda, MD, USA; Zugangsnummer L08752; Fig. 3) in *Escherichia coli* DH12S gewählt. Dieser Vektor trägt den durch Zugabe von IPTG induzierbaren β-Galactosidase Promotor des lac-Operons, so daß in diesen Zellen eine hierüber kontrollierte Expression der in die multiple Klonierungsstelle integrierten DNA möglich ist. Der Stamm DH12S ist wegen seines laclq-Genotyps für die IPTG-Induktion geeignet und vorteilhaft für ein Protease-Aktivitätsscreening, weil er eine hinreichend niedrige endogene proteolytische Aktivität aufweist. Vorversuche hatten gezeigt, daß auch E. *coli* JM109 dieselben Voraussetzungen erfüllt.

Die Aufarbeitung der DNA aus der nach Beispiel 1 erhaltenen Probe erfolgte nach Zhou et al. (1996), Appl. Environ. Microbiol., Band 62, Seiten 316-322. Diese gereinigte Metagenom-DNA (siehe unten) wurde einer präparativen Partialrestriktion mit dem Restriktionsenzym Alu l zur Darstellung von Fragmentgrößen im Bereich von 2 bis 10 kb unterworfen.

Dafür wurde zunächst die optimale Restriktions-Inkubationsdauer durch Aufnahme einer Enzymkinetik bestimmt. Hierzu wurden 2,8 µg der DNA-Präparation in dem von dem Hersteller von Alu I (Fa. New England Biolabs, Schwalbach, Deutschland; Katalog-Nr. R0137S) angebotenen entsprechenden Reaktionspuffer bei 37°C inkubiert. Durch Zugabe von 0,2 U Alu I pro µg DNA wurde die Reaktion in einem Gesamtvolumen von 21 µl gestartet und danach in zweiminütigen Intervallen jeweils 1,5 µl aus dem Ansatz entnommen, worin sofort jeweils die Reaktion durch Zugabe von 10 mM Tris/HCl, pH 7,0; 20% Glycerin; 0,1% SDS und Kühlen auf 0°C beendet wurde. Durch anschließende Analyse auf einem 0,7%igen Agarosegel wurde die optimale Restriktionsdauer für einen partiellen Verdau ermittelt. Sie beträgt für die Isolierung der nach Beispiel 1 isolierten DNA bei ca. 6 bis 7 min, um Fragmente im Größenbereich von 2 bis 10 kb zu erhalten.

Der präparative Partialverdau erfolgte dementsprechend in 30 parallelen Ansätzen. Nach entsprechendem Abstoppen der Reaktion wurde der Ansatz auf einem präparativen 0,7%igen Agarosegel elektrophoretisch aufgetrennt, der Gelbereich mit DNA der Größen 2 bis 10 kb ausgeschnitten und dieser mittels Elektroelution in Dialyseschläuchen bei 4°C isoliert. Die DNA wurde abschließend mit 1/10 Volumen 3 M Na-Acetat und dem 2,5-fachen Volumen Ethanol gefällt und in einem adäquaten Volumen aufgenommen. Zur weiteren Abtrennung von etwaig vorhandenen kleineren DNA-Bruchstücken wurden die Gelelektrophorese, Elektroporation und Fällung wiederholt.

440 ng der so erhaltenen fragmentierten Metagenom-DNA wurden über Nacht bei 16°C in einem Gesamtvolumen von 15 µl mit 150 ng des Vektors pUC18 unter Zugabe von 400 NEB-Units T4-DNA-Ligase in 1xLigase-Puffer ligiert. Dieser Vektor war zuvor mit Sma 1 linearisiert und mit Alkalischer Phosphatase aus Kalbsthymus dephosphoryliert worden.

Die Transformation kompetenter *E. coli* DH12S-Zellen (Fa. Gibco Life Technologies, Karlsruhe, Katalog-Nummer 18312017) erfolgte über Elektro-Transformation. Hierfür wurden 1 µl Ligationsansatz und 25 µl Zellen gemischt, in einer Elektroporationsküvette 1 min lang auf Eis inkubiert und im Elektroporator (BTX^{®} ECM630, Genetronics Inc. San Diego, USA) nach Herstellerangaben behandelt. Nach sofortiger Überführung in 1 ml SOC-Medium (2% Bacto-Trypton; 0,5% Hefeextrakt; 10 mM NaCl; 2,5 mM KCl; pH 7,0, eingestellt mit NaOH; autoklaviert; supplementiert mit 10 mM MgSO₄ und MgCl₂ sowie 20 mM D(+)Glucose) folgten eine Erholungsphase von 1 h bei 37°C und wie in Beispiel 1 eine Plattierung auf Agarplatten mit HSP10-Festmedium.

### Beispiel 3

### Screening auf proteolytische Aktivität

Zur Untersuchung der Qualität der nach Beispiel 2 hergestellten Genbank in *E*. *coli* DH12S wurde die Anzahl der insgesamt erzeugten Primärtransformanten und die Anzahl Insert-tragender Klone über BlauANeiß-Selektion in einer Testplattierung bestimmt. Hierfür wurden je 1 und 10 µl des Transformationsansatzes auf 5%igen Agarplatten mit LB-Medium (10 g Trypton, 5 g Hefe-Extrakt, 5 g NaCl, 1 ml 1 N NaOH pro I), welches zusätzlich mit 100 µg/ml Ampicillin, 0,2 mM (oder 4 µg/ml) IPTG und 0,2 mM (oder 1 µg/ml) X-Gal versetzt war, ausplattiert und über Nacht bei 37°C inkubiert. Aus 10 weißen Kolonien, das heißt Transformanten, wurden die Plasmide über Minipräparation (Kit der Fa. Qiagen, Hilden, Deutschland) isoliert, ein Restriktionsverdau mit den Restriktionsenzymen Sac 1 und Hind III zur Excision des Inserts (vergleiche Figur 3) durchgeführt und die Fragmente auf einem 0,7%igen Agarosegel aufgetrennt. Tatsächlich enthielten alle Vektoren Inserts von ca. 2 bis 10 kb Größe.

Das Screening der nach Beispiel 2 erzeugten Genbank erfolgte auf 5%igen Agarplatten mit 14 cm Durchmesser mit LB-Medium Ampicillin/IPTG/X-Gal (siehe oben) und zusätzlich 2% Magermilchpulver (Skim Milk, Fa. Difco, Best.-Nr. 232100): Auf 10 dieser Selektionsagar-Platten wurden dem Titer der Bank entsprechend Volumina des Transformationsansatzes von jeweils ca. 10.000 cfu mittels Glaskugeln gleichmäßig ausplattiert (primäre Plattierung).

Nach 16stündiger Inkubation bei 37°C wurden die Platten bis zu zwei Wochen bei 28°C inkubiert. Während dieser Zeit gaben sich proteasebildende Klone durch Klärungshöfe in dem trüben Substrat zu erkennen. Eine gesonderte Zell-Lyse zum Nachweis nicht exportierter Proteasen war nicht notwendig. Die Validierung der Plasmid-vermittelten Proteasebildung erfolgte durch erneute Vereinzelung der Primärklone und anschließend durch Isolierung der betreffenden inserthaltigen pUC18-Vektoren, Retransformation und erneutes Screening (wie oben; sekundäre Plattierung). Die hieraus hervorgegangenen Tansformanten zeigten ebenfalls Hofbildung auf Magermilch-Medium und bestätigten somit die Lokalisation eines Proteasegens auf dem jeweils klonierten DNA-Fragment.

### Beispiel 4

### Sequenzanalyse eines proteolytisch aktiven Klons (HP70Pa_2)

Aus einem nach Beispiel 3 erhaltenen, Protease-positiven Klon mit der Bezeichnung HP70Pa_2 wurde die Plasmid-DNA nach Standardmethoden isoliert, das Insert über Sac I/Hind III-Verdau präpariert (siehe oben) und nach Standardmethoden sequenziert. Hierbei kamen zunächst die für den Vektor spezifischen, das Insert flankierenden Primer gemäß SEQ ID NO. 1 und 2 mit den Bezeichnungen M13f beziehungsweise M13r zum Einsatz, gefolgt von dem sogenannten Primer-Walking, wie es aus dem Stand der Technik (R.J.Kaiser et al. (1989): "Specific primer-directed DNA sequencing using automated fluorescence detection"; Nucl. Acids Res., 17 (15), S. 6087-6102) bekannt ist.

Die Sequenzierung dieses Klons ergab einen Bereich mit einem offenen Leserahmen, dessen DNA-Sequenz in SEQ ID NO. 3 angegeben ist. Aufgrund seiner Herkunft wird dort als Organismus "unbekannt" vermerkt und zusätzlich angegeben, daß diese Sequenz auf ein DNA-Isolat zurückzuführen ist. Ferner muß aufgrund der vorliegenden Daten (insbesondere über Sequenzvergleiche, siehe unten) davon ausgegangen werden, daß die Nukleotidpositionen 1 bis 96 für das Signalpeptid codieren und sich insgesamt der codierende Bereich von 1 bis 1746 erstreckt. Die hiervon abgeleitete Aminosäuresequenz offenbart SEQ ID NO. 4, mit den gleichen Angaben bezüglich der Herkunft.

Als nächstähnliches beschriebenes Enzym wurde eine extrazelluläre Serin-Protease (E.C. 3.4.21.-) aus *Xanthomonas campestris pv. campestris* (ATCC 33913) gefunden, die bei GenBank (National Center for Biotechnology Information NCBI, National Institutes of Health, Bethesda, MD, USA) die Zugangsnummer NP_636242 trägt. Die über das Computer-Programm Vector NTI^{®} Suite 7.0, erhältlich von der Firma InforMax, Inc., Bethesda, USA, mit den vorgegebenen Default-Parametern ermittelte Homologie auf Aminosäureebene beträgt zu HP70 75,0% Identität. Weitere bei dieser Suche gefundene Proteine, die auf Aminosäure-Ebene noch am ähnlichsten erscheinen, sind in folgender Tabelle 1 zusammengestellt.

**Tabelle 1: Auf Aminosäure-Ebene zu HP70 nächstähnliche gefundene Sequenzen**

| Zugangsnummer | Beschreibung | Identität [%] |
|---|---|---|
| NP_636242 | extrazelluläre Protease aus *Xanthomonas campestris pv. campestris,* Stamm ATCC 33913 | 75,0 |
| NP_641280 | extrazelluläre Protease aus *Xanthomonas axonopodis pv. citri* Stamm 306 | 73,5 |
| NP_641281 | extrazelluläre Protease aus *Xanthomonas axonopodis pv. citri* Stamm 306 | 60,8 |
| NP_641282 | extrazelluläre Protease aus *Xanthomonas axonopodis pv. citri* Stamm 306 | 60,8 |
| NP_636245 | extrazelluläre Protease aus *Xanthomonas campestris pv. campestris* Stamm ATCC 33913 | 59,2 |

Auf DNA-Ebene ergibt sich zu dem Gen der extrazellulären Serin-Protease (E.C. 3.4.21.-) aus *Xanthomonas campestris pv. campestris* (Gen ID XCC0851) eine Identität von 74,4%.

Somit handelt es sich bei der gefundenen Protease höchstwahrscheinlich ebenfalls um eine Serin-Protease. Zu der etablierten B. *lentus-*Alkalischen Protease (WO 92/21760 A1) ergeben sich auf Aminosäureebene eine Homologie von 26,2% Identität und auf Nukleinsäureebene eine Identität von 33,6%.

Der zugehörige Vektor mit der Bezeichnung 70-pUC(AWB403) wurde am 2.10.2003 bei der Deutschen Sammlung von Mikroorganismen und Zellkulturen GmbH, Mascheroder Weg 1b, 38124 Braunschweig (http://www.dsmz.de) hinterlegt und trägt dort die Hinterlegungsnummer DSM 15977. Die hiervon codierte Protease wird als HP70 bezeichnet.

### Beispiel 5

### Sequenzanalyse eines proteolytisch aktiven Klons (HP53Pa_2)

Aus einem weiteren nach Beispiel 3 erhaltenen Protease-positiven Klon mit der Bezeichnung HP53Pa_2 wurde wie in Beispiel 4 das Insert präpariert und sequenziert.

Die erhaltenen Sequenzen sind in SEQ ID NO. 6 und 7 dargestellt. Aufgrund seiner Herkunft wird dort als Organismus "unbekannt" vermerkt und zusätzlich angegeben, daß diese Sequenzen auf ein DNA-Isolat zurückzuführen sind. Ferner muß aufgrund der vorliegenden Daten (insbesondere über Sequenzvergleiche, siehe unten) davon ausgegangen werden, daß die Nukleotidpositionen 1 bis 114 für das Signalpeptid codieren und sich insgesamt der codierende Bereich von 1 bis 1761 erstreckt.

Es handelt sich wiederum um eine Subtilisin-Protease, die auf Aminosäure-Ebene zu der auch in diesem Fall als nächstähnlichen ermittelten extrazellulären Serin-Protease (E.C. 3.4.21.-) aus *Xanthomonas campestris pv. campestris* (ATCC 33913; siehe oben) eine Homologie von 75,4% Identität aufweist. Weitere bei dieser Suche gefundene Proteine, die auf Aminosäure-Ebene noch am ähnlichsten erscheinen, sind in folgender Tabelle 2 zusammengestellt.

**Tabelle 2: Auf Aminosäure-Ebene zu HP53 nächstähnliche gefundene Sequenzen**

| Zugangsnummer | Beschreibung | Identität [%] |
|---|---|---|
| NP_636242 | extrazelluläre Protease aus *Xanthomonas campestris pv. campestris* Stamm ATCC 33913 | 75,4 |
| NP_641280 | extrazelluläre Protease aus *Xanthomonas axonopodis pv. citri* Stamm 306 | 72,6 |
| NP_641281 | extrazelluläre Protease aus *Xanthomonas axonopodis pv. citri* Stamm 306 | 59,3 |
| NP_641282 | extrazelluläre Protease aus *Xanthomonas axonopodis pv. citri* Stamm 306 | 59,8 |
| NP_636245 | extrazelluläre Protease aus *Xanthomonas campestris pv. campestris* Stamm ATCC 33913 | 59,0 |

Auf DNA-Ebene ergibt sich zu dem Gen der extrazellulären Serin-Protease (E.C. 3.4.21.-) aus *Xanthomonas campestris pv. campestris* eine Identität von 75,0%.

Zu der etablierten B. lentus-Alkalischen Protease (WO 92/21760 A1) ergeben sich auf Aminosäureebene eine Homologie von 25,9% Identität und auf Nukleinsäureebene eine Identität von 33,5%.

Der zugehörige Vektor mit der Bezeichnung 53-pUC(AWB403) wurde am 2.10.2003 bei der Deutschen Sammlung von Mikroorganismen und Zellkulturen GmbH, Mascheroder Weg 1 b, 38124 Braunschweig (http://www.dsmz.de) hinterlegt und trägt dort die Hinterlegungsnummer DSM 15976. Die hiervon codierte Protease wird als HP53 bezeichnet.

### Beispiel 6

### Erzeugung C-terminaler Deletions-Mutanten von HP70 und HP53

### HP70dc

Von der nach Beispiel 3 erhaltenen und in Beispiel 4 beschriebenen Protease des Klons mit der Bezeichnung HP70Pa_2 wurden C'-terminal 332 bp deletiert. Diese Aminosäresequenz HP70_deltaC ist in SEQ ID NO. 5 dargestellt. Aufgrund seiner Konstruktion wird dort vermerkt, daß es sich um eine künstliche Sequenz handelt, konkretisiert mit der Angabe "DNA-Isolat, Delta C". Gemäß SEQ ID NO. 3 und 4 kann der Abschnitt der Positionen 1 bis 32 wiederum als Signalpeptid betrachtet werden.

Hierfür wird von der in SEQ ID NO. 3 angegebenen DNA mit den Oligonukleotiden HP70f (SEQ ID NO. 10) und HP70r (SEQ ID NO. 11) als Primern unter Standard-PCR-Bedingungen ein 1413 bp großes DNA-Stück generiert. Nach Bearbeitung des erhaltenen Fragments mit den Endonukleasen EcoRl und BamHl wird das Fragment in die entsprechenden Schnittstellen des E. coli-Expressionsvektors pUC18 kloniert und in einem geeigneten Stamm (zum Beispiel E. *coli* DH12S) transformiert. Aus den erhaltenen Transformanden kann durch Blau/weiß-Selektion der gewünschte Kandidat idientifiziert, aktiv exprimiert und so in ausreichender Menge für weitere Untersuchungen erhalten werden.

### HP53dc

Von der nach Beispiel 3 erhaltenen und in Beispiel 5 beschriebenen Protease des Klons mit der Bezeichnung HP53Pa_2 wurden C'-terminal 330 bp deletiert. Diese Aminosäresequenz HP53_deltaC ist in SEQ ID NO. 8 dargestellt.

Hierfür wird von der in SEQ ID NO. 6 angegebenen DNA mit den Oligonukleotiden HP53f (SEQ ID NO. 12) und HP53r (SEQ ID NO. 13) als Primern unter Standard-PCR-Bedingungen ein 1303 bp großes DNA-Stück generiert. Nach Bearbeitung des erhaltenen Fragments mit den Endonukleasen Blpl und BamHl wird das Fragment in die entsprechenden Schnittstellen des oben im ersten Teil des Beispiels gewonnenen E. coli-Expressionsvektors pUC18_HP70_dc kloniert, der zuvor mit denselben Endonukleasen bearbeitet worden ist.

Nach Transformation in einem geeigneten Stamm (zum Beispiel E. *coli* DH12S) kann aus den erhaltenen Transformanden durch Restriktionsanalyse mit Sacll (pUC_HP53_dc enthält eine solche Schnittstelle) der gewünschte Kandidat identifiziert, aktiv exprimiert und so in ausreichender Menge für weitere Untersuchungen erhalten werden.

Aufgrund dieser Konstruktion wird in SEQ ID NO. 8 vermerkt, daß es sich wie bei SEQ ID NO. 5 um eine künstliche Sequenz handelt, konkretisiert mit der Angabe "DNA-Isolat, Delta C". Ferner wurde dabei der Abschnitt der Positionen 1 bis 32 gemäß SEQ ID NO. 5 eingeführt, so daß dieser als Signalpeptid von HP70 betrachtet werden muß.

### Beispiel 7

### Quantitative Gewinnung der erfindungsgemäßen Proteasen und deren biochemische Charakterisierung

Die nach den Beispielen 3 bis 6 erhaltenen Expressionsklone wurden in 100 ml LB-Medium (10 g/l Trypton, 5 g/l, Hefeextrakt, 10 g/l NaCl) aufgenommen und in 500 ml-Erlenmeyer-Kolben bei 37°C und unter Schütteln bei 200 rpm kultiviert.

Anschließend wurden sie biochemisch charakterisiert. Hierbei wurde die proteolytische Aktivität über einen sogenannten MTP-Assay ermittelt, der auf einem Fluoreszenzgekoppelten Caseinsubstrat (BODIPY^{®}FL Conjugate, Fa. Molecular Probes, Göttingen, Deutschland; Best.-Nr. #6638) basiert, an welches Fluorophore (Emitter) und Dämpfer (Quencher) gekoppelt sind. Im intakten Substrat wird eine Fluoreszenz der Emitter durch die Quencher unterdrückt. Bei Hydrolyse des Caseins entfernen sich jedoch die Oligopeptide mit den an sie gekoppelten Gruppen voneinander und es kommt bei entsprechender Anregung zur Fluoreszenzemission, deren Intensität somit ein Maß für die Proteolyse darstellt.

Zur Aktivitätsbestimmung werden jeweils 5 µl einer Proteaseprobe gemäß Beispiel 5 in 100 mM Tris/HCl mit dem gewünschten pH-Wert und 4,5 µg/ml BODIPY^{®} FL Conjugate in einem Gesamtvolumen von 100 µl für 1 h bei der interessierenden Temperatur inkubiert. Alle nachfolgend angegebenen Messungen erfolgten in 96well-Mikrotiterplatten (Opaque^{®} Plates, black; Fa. Corning BV Life Sciences, Schiphol-Rijk, Niederlande; Bestell.-Nr. #3915) mit Hilfe eines FLUOstar^{®}-Fluorezenz-Meßgeräts (Fa. BMG Lab Technologies, Offenburg, Deutschland).

### pH- und Temperaturoptimum

Für die in SEQ ID NO. 7 dargestellte Protease HP53 ergeben sich folgende biochemische Parameter: pH-Optimum bei 37°C: 8,6 und Temperaturoptimum bei pH 8,6: 37°C.

### Einfluß von Komplexbildnern

Der Einfluß von Komplexbildnern wurde durch Zugabe von 1 mM EDTA bei pH 8,6 im oben angegebenen Assay untersucht, und zwar bei 37°C und 50°C. Der Meßwert ohne Zugabe von EDTA wurde auf 100% gesetzt. Demgegenüber lag die relative proteolytische Aktivität bei 50°C bei 27% und bei 37°C bei 10%.

### Stabilitätsmessung

Zur Messung der Stabilität wurde die eingesetzte Proteaseprobe zunächst für 15 min bei 50°C in 50 mM NaHCO₃-Puffer, pH 10,9 vorinkubiert und danach die Restaktivität im o.g. Assay bei 37°C und 50°C, bei jeweils pH 8,6 gemessen. Hierbei wurde die Aktivität desselben Extrakts ohne Vorinkubation aber ansonsten gleicher Behandlung jeweils auf 100% gesetzt. Auf diese Weise wurde für 37°C eine Restaktivität von 11% und für 50°C von 13% ermittelt.

Es handelt sich somit um Moleküle, welche gegenüber hohen pH-Werten relativ stabil sind, und dies sogar nahezu unabhängig von der Temperatur.

### Beispiel 8

### Beitrag der erfindungsgemäßen Protease HP70 zur Waschleistung bei niedrigerer Temperatur

Für dieses Beispiel wurden standardisiert mit Anschmutzungen versehene Textilien eingesetzt, die von der Eidgenössischen Material-Prüfungs- und -Versuchsanstalt, St. Gallen, Schweiz (EMPA), bezogen worden waren. Dabei wurden folgende Anschmutzungen und Textilien verwendet: A (Blut/Milch/Tusche auf Baumwolle), B (Blut/Milch/Tusche auf einem Polyester-Baumwolle-Mischgewebe) und C (Ei/Ruß auf Baumwolle).

Mit diesem Testmaterial wurden verschiedene Waschmittelrezepturen launderometrisch auf ihre Waschleistung hin untersucht. Dafür wurde jeweils ein Flottenverhältnis von 1:12 eingestellt und für 30 min bei einer Temperatur von 40°C gewaschen. Die Dosierung lag bei 5,9 g des jeweiligen Mittels pro I Waschflotte. Die Wasserhärte betrug 16° deutscher Härte.

Als Kontroll-Waschmittel diente eine Waschmittel-Basis-Rezeptur folgender Zusammensetzung (alle Angaben in Gewichts-Prozent): 4 % lineares Alkylbenzolsulfonat (Natrium-Salz), 4 % C₁₂-C₁₈-Fettalkoholsulfat (Natrium-Salz), 5,5 % C₁₂-C₁₈-Fettalkohol mit 7 EO, 1 % Natrium-Seife, 11 % Natriumcarbonat, 2,5 % amorphes Natriumdisilikat, 20 % Natriumperborat-Tetrahydrat, 5,5 % TAED, 25 % Zeolith A, 4,5 % Polycarboxylat, 0,5 % Phosphonat, 2,5 % Schauminhibitorgranulat, 5 % Natriumsulfat, Rest: Wasser, optischer Aufheller, Salze.

Sie wurde in parallelen Ansätzen jeweils aktivitätsgleich mit der erfindungsgemäßen und einer Kontroll-Protease versetzt. Zur Kontrolle diente die B. *lentus-*Alkalische Protease F49 (WO 95/23221 A1; Hersteller: Firma Biozym, Kundl, Österreich). Diese besaß eine (nach der in der Beschreibung angegebenen Methode bestimmbare) spezifische Aktivität von ca. 200.000 PE/g, wodurch sich mit 0,2 Gew.-% eine F49-Konzentration von ca. 40.000 PE pro 100 g des Mittels und eine Aktivität von ca. 2.400 PE pro I Waschflotte ergaben. Zusätzlich wurden Rezepturen hergestellt, die unter Verzicht auf eine entsprechende Menge an Salzen jeweils 0,5 %, also die zweieinhalbfache Protease-Menge enthielten. Die erfindungsgemäße Protease wurde derselben Basisrezeptur in denselben Aktivitäts-Konzentrationen zugesetzt. Insofern sind die in der nachfolgenden Tabelle angegebenen Gew.-%-Werte für F49 korrekt und gelten für HP70 näherungsweise.

Nach dem Waschen wurde der Weißheitsgrad der gewaschenen Textilien im Vergleich zu dem von Bariumsulfat gemessen, der auf 100 % normiert worden war. Die Messung erfolgte an einem Spektrometer Datacolor SF500-2 bei 460 nm (UV-Sperrfilter 3), 30 mm Blende, ohne Glanz, Lichtart D65, 10°, d/8°. Die erhaltenen Ergebnisse werden als Prozent Remission, das heißt als Prozentangaben im Vergleich zu Bariumsulfat zusammen mit den jeweiligen Anfangswerten in folgender Tabelle 3 zusammengestellt. Angegeben sind die Mittelwerte aus jeweils drei Messungen. Sie erlauben einen unmittelbaren Rückschluß auf den Beitrag des enthaltenen Enzyms zur Waschleistung des verwendeten Mittels.

**Tabelle 3: Beitrag der erfindungsgemäßen Protease HP70 zur Waschleistung bei einer Temperatur von 40°C**

| Basiswaschmittel mit | A | B | C |
|---|---|---|---|
| Anfangswert | 14,1 | 12,8 | 28,9 |
| Kontrolle ohne Protease | 18,9 | 16,0 | 51,3 |
| 0,2 % HP70 | 26,7 | 26,5 | 68,3 |
| 0,2 % B. lentus-Alkalische Protease F49 | 25,7 | 25,6 | 68,1 |
| 0,5 % HP70 | 33,4 | 29,8 | 68,4 |
| 0,5 % B. lentus-Alkalische Protease F49 | 30,0 | 32,2 | 70,2 |
| Standardabw. | 1,3 | 1,3 | 1,8 |

Alle drei Meßreihen belegen, daß die erfindungsgemäße Protease HP70 gegenüber proteasefreien Waschmitteln eine Verbesserung der Waschleistung an proteinhaltigen Anschmutzungen erbringt. Das heißt, sie entfaltet auch in Gegenwart denaturierender Agentien wie etwa Tensiden eine proteolytische Aktivität. Die ermittelten Werte für die B. *lentus-*Alkalische Protease F49, einem für dieses Einsatzgebiet über Punktmutagenese optimiertes Molekül (vergleiche WO 95/23221 A1) belegen die Korrektheit der Versuchsdurchführung. In der Meßreihe A und bei der niedrigeren Konzentration in Meßreihe B übertraf HP70 sogar das Ergebnis für F49; die übrigen Werte sind denen für F49 vergleichbar.

### Beispiel 9

### Beitrag der erfindungsgemäßen Protease HP53 zur Waschleistung bei niedrigerer Temperatur

Der vorangegangene Versuch wurde mit der erfindungsgemäßen Protease HP53 wiederholt. Die Bedingungen waren dieselben. Allerdings wurde die Leistung statt an der Anschmutzung C an der Anschmutzung D (Blut auf Baumwolle) ermittelt. Das Ergebnis ist in folgender Tabelle 4 zusammengestellt.

**Tabelle 4: Beitrag der erfindungsgemäßen Protease HP53 zur Waschleistung bei einer Temperatur von 40°C**

| Basiswaschmittel mit | A | B | D |
|---|---|---|---|
| Anfangswert | 13,5 | 13,9 | 18,8 |
| Kontrolle ohne Protease | 20,1 | 18,7 | 67,3 |
| 0,2 % HP53 | 48,1 | 60,7 | 73,7 |
| 0,2 % B. lentus-Alkalische Protease F49 | 25,5 | 27,9 | 69,7 |
| 0,5 % HP53 | 42,9 | 53,5 | 69,0 |
| 0,5 % B. lentus-Alkalische Protease F49 | 32,6 | 41,1 | 73,8 |
| Standardabw. | 4,5 | 1,7 | 2,4 |

Diese Meßreihen belegen, daß auch die erfindungsgemäße Protease HP53 gegenüber proteasefreien Waschmitteln eine Verbesserung der Waschleistung an proteinhaltigen Anschmutzungen erbringt. Das heißt, sie entfaltet auch in Gegenwart denaturierender Agentien wie etwa Tensiden oder Bleiche eine proteolytische Aktivität. Die ermittelten Werte sind denen für die B. *lentus-Alkalische* Protease F49 zumindest vergleichbar, in den Meßreihen A und B sogar deutlich überlegen.

### Beispiel 10

### Beitrag der erfindungsgemäßen Protease HP53dc zur Waschleistung bei niedrigerer Temperatur

Wie in den vorangegangenen beiden Beispielen wurde auch die Variante HP53dc hinsichtlich ihres Leistungsbeitrags bezüglich der Anschmutzungen A, B und C untersucht. Die Bedingungen waren wiederum dieselben. Das Ergebnis ist in folgender Tabelle 5 zusammengestellt.

**Tabelle 5: Beitrag der erfindungsgemäßen Protease HP53dc zur Waschleistung bei einer Temperatur von 40°C**

| Basiswaschmittel mit | A | B | C |
|---|---|---|---|
| Anfangswert | 14,6 | 13,2 | 28,1 |
| Kontrolle ohne Protease | 19,6 | 16,2 | 54,0 |
| 0,2 % HP53dc | 42,8 | 41,2 | 71,2 |
| 0,2 % *B. lentus*-Alkalische Protease F49 | 26,8 | 31,2 | 70,0 |
| 0,5 % HP53dc | 48,6 | 50,0 | 70,8 |
| 0,5 % *B. lentus*-Alkalische Protease F49 | 29,8 | 40,7 | 70,9 |
| Standardabw. | 1,6 | 1,9 | 2,5 |

Diese Meßreihen belegen, daß auch die erfindungsgemäße Protease HP53dc gegenüber proteasefreien Waschmitteln eine Verbesserung der Waschleistung an proteinhaltigen Anschmutzungen erbringt. Das heißt, sie entfaltet auch in Gegenwart denaturierender Agentien wie etwa Tensiden oder Bleiche eine proteolytische Aktivität. Die ermittelten Werte sind in den Meßreihen A und B denen für die B. lentus-Alkalische Protease F49 deutlich überlegen und in Reihe C zumindest vergleichbar.

### Beispiel 11

### Beitrag der erfindungsgemäßen Protease HP53dc zur Waschleistung bei höherer Temperatur

Der vorangegangene Versuch wurde mit HP53 an den beiden Anschmutzungen A und B bei einer Waschtemperatur von 60°C unter ansonsten identischen Bedingungen wiederholt. Das Ergebnis ist in folgender Tabelle 6 zusammengestellt.

**Tabelle 6: Beitrag der erfindungsgemäßen Protease HP53dc zur Waschleistung bei einer Temperatur von 60°C**

| Basiswaschmittel mit | A | B |
|---|---|---|
| Anfangswert | 14,6 | 13,2 |
| Kontrolle ohne Protease | 20,5 | 16,7 |
| 0,2 % HP53dc | 36,3 | 36,9 |
| 0,2 % *B. lentus*-Alkalische Protease F49 | 25,6 | 32,4 |
| 0,5 % HP53dc | 47,7 | 48,0 |
| 0,5 % B. lentus-Alkalische Protease F49 | 31,4 | 43,5 |
| Standardabw. | 1,7 | 1,3 |

Auch bei der Temperatur von 60°C zeigt sich in den Meßreihen A und B die Überlegenheit der erfindungsgemäßen Protease HP53dc gegenüber der *B. lentus-*Alkalischen Protease F49. Erfreulicherweise wird die erfindungsgemäße Protease HP53dc bei 60°C nicht nennenswert denaturiert, so daß sie insbesondere als Waschmittelprotease in Frage kommt.

### Beschreibung der Figuren

- Figur 1:: Alignment der erfindungsgemäßen Alkalischen Proteasen HP70 und HP53 (SEQ ID NO. 4 beziehungsweise 7) mit Alkalischen Proteasen aus dem Stand der Technik.
Darin bedeuten:
- HP70:: Erfindungsgemäße Alkalische Protease gemäß SEQ ID NO. 4;
- HP53:: Erfindungsgemäße Alkalische Protease gemäß SEQ ID NO. 7;
- SP:: Extrazelluläre Serin-Protease (E.C. 3.4.21.-) aus *Xanthomonas campestris pv. campestris* (ATCC 33913) (Zugangs-Nr. NP_636242 bei GenBank);
- BLAP:: Alkalische Protease aus *Bacillus lentus* DSM 5483 (WO 92/21760 A1).

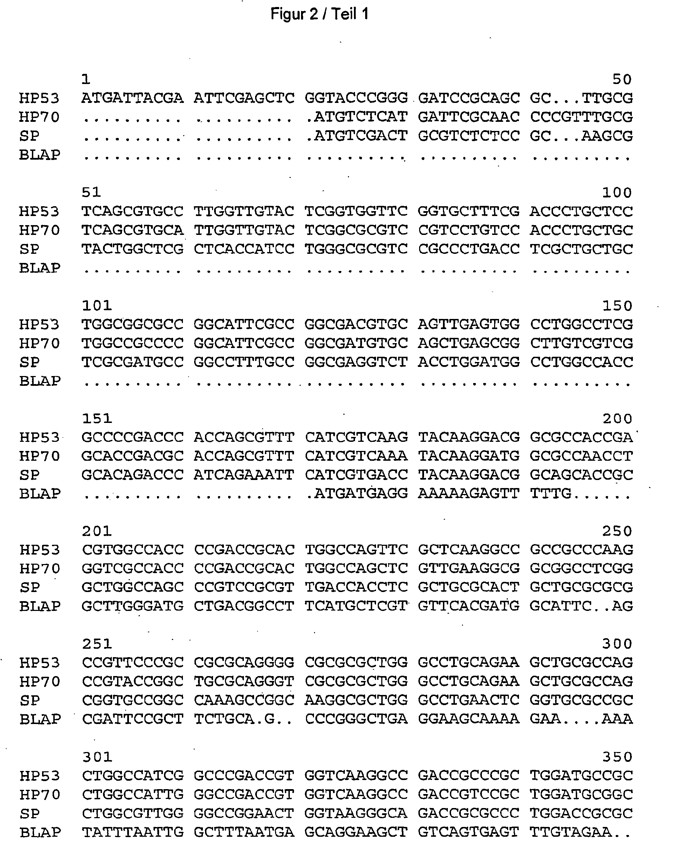

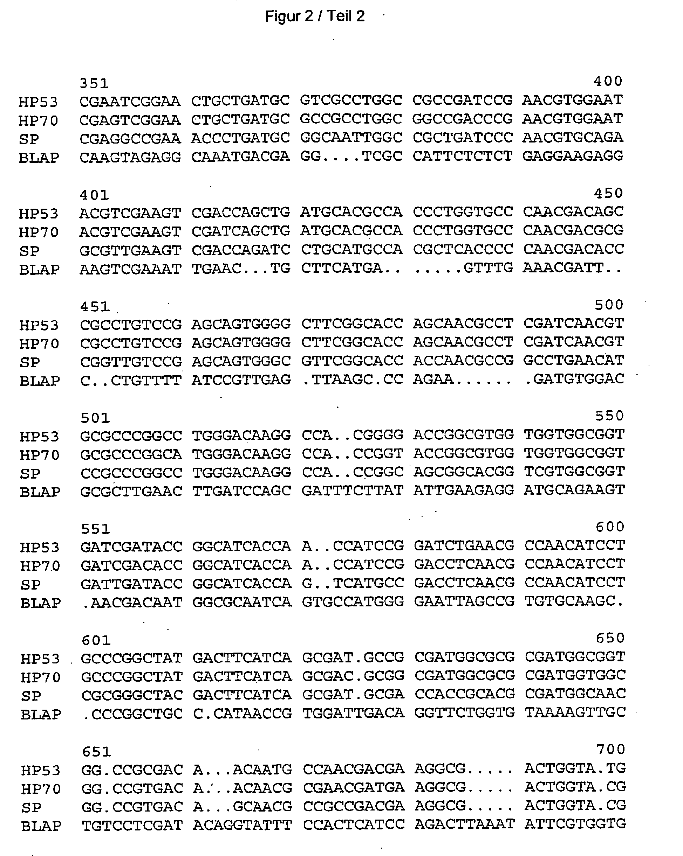

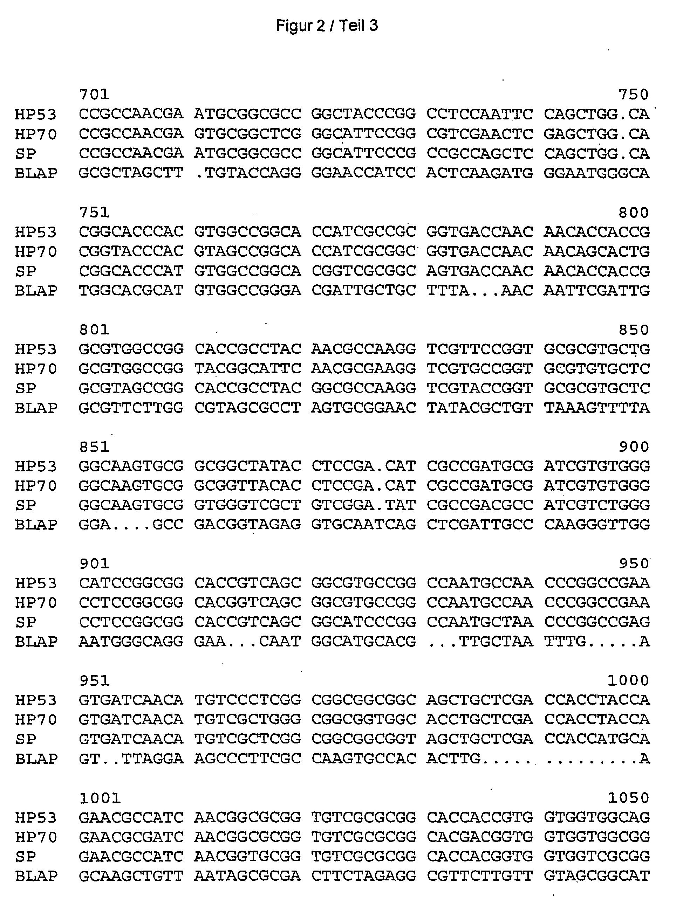

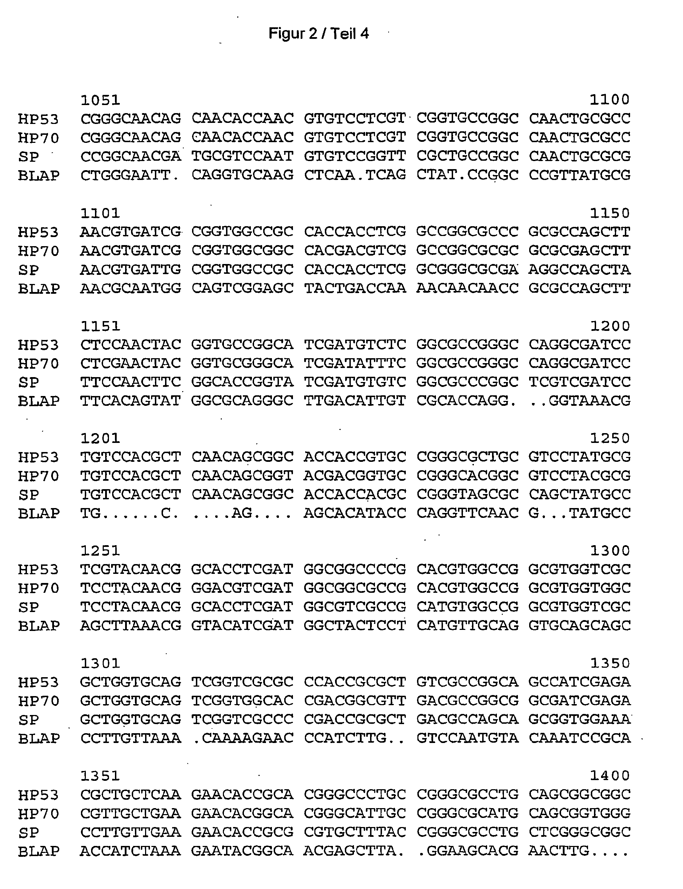

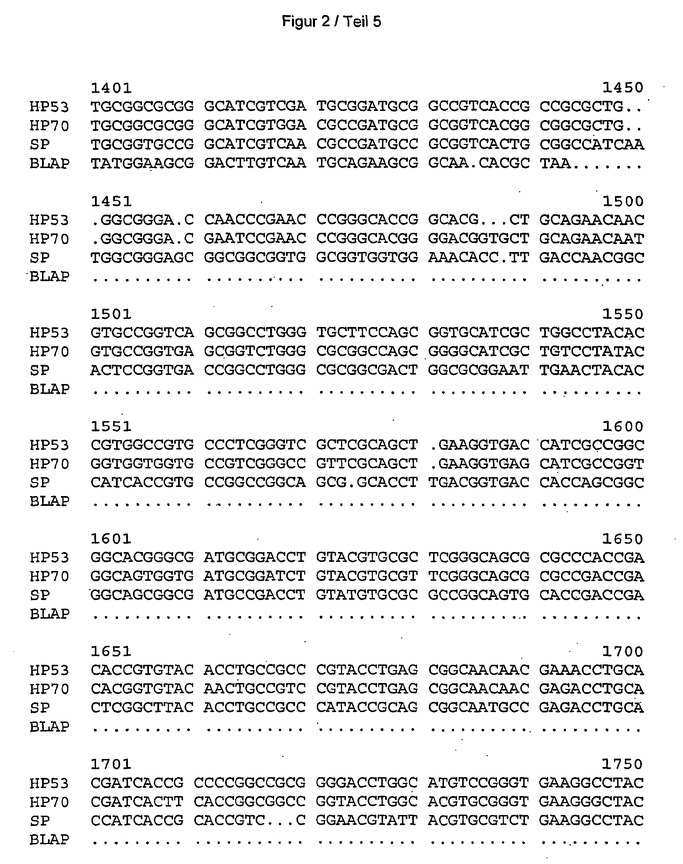

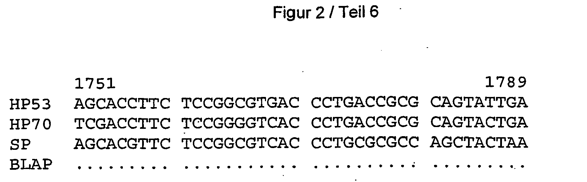

- Figur 2:: Alignment der Gene der erfindungsgemäßen Alkalische Proteasen HP70 und HP53 (SEQ ID NO. 3 beziehungsweise 6) mit denen von Alkalischen Proteasen aus dem Stand der Technik.
Darin bedeuten:
- HP70:: Gen der erfindungsgemäße Alkalischen Protease HP70 gemäß SEQ ID NO. 3;
- HP53:: Gen der erfindungsgemäßen Alkalischen Protease HP53 gemäß SEQ ID NO. 6;
- SP:: Gen der extrazellulären Serin-Protease (E.C. 3.4.21.-) aus *Xanthomonas campestris pv. campestris* (ATCC 33913) (Zugangs-Nr. NP_636242 bei GenBank);
- BLAP:: Gen der Alkalischen Protease aus *Bacillus lentus* DSM 5483 (WO 92/21760 A1).
- Figur 3:: Schematische Darstellung des zur Anlage einer Expressions-Genbank gemäß Beispiel 2 verwendeten Plasmidvektors pUC18.

Der Vektor wurde zur Aufnahme der mit Alu I verdauten Metagenom-DNA mit Sma I linearisiert.
Darin bedeuten:
- ORI:: Replikationsursprung
- Plac:: lac-Promotor
- lacZ-alpha:: Gen für das Alpha-Peptid der Beta-Glactosidase
- amp^{R}:: Ampicillinresistenz-vermittelnde Beta-Lactamase
- Figur 4:: Alignment der Aminosäuresequenzen der beiden erfindungsgemäßen Proteasen HP70 (SEQ ID NO. 4) und HP53 (SEQ ID NO. 7) zur Entwicklung der Consensus-Sequenz von SEQ ID NO. 9.

Die darin als Variablen X bezeichneten Aminosäure-Positionen lassen sich dieser Abbildung zufolge entweder auf HP70 oder auf HP53 zurückführen.

## Patentansprüche

1. Alkalische Protease, deren Aminosäuresequenz zu der in SEQ ID NO. 4 angebenen Aminosäuresequenz mindestens zu 90% oder zu der in SEQ ID NO. 7 angebenen Aminosäuresequenz mindestens zu 87,5% identisch ist.

2. Alkalische Protease nach Anspruch 1, deren Aminosäuresequenz zu der in SEQ ID NO. 4 angegebenen Aminosäuresequenz zunehmend bevorzugt zu mindestens 95%, 96%, 97%, 98%, 99% und ganz besonders bevorzugt zu 100% identisch ist oder zu der in SEQ ID NO. 7 angegebenen Aminosäuresequenz zunehmend bevorzugt zu mindestens 90%, 95%, 96%, 97%, 98%, 99% und ganz besonders bevorzugt zu 100% identisch ist.

3. Alkalische Protease nach Anspruch 1 oder 2, wobei die Homologiewerte jeweils für den Bereich gelten, der den Aminosäurepositionen 33 bis 581 gemäß SEQ ID NO. 4 oder 39 bis 586 gemäß SEQ ID NO. 7 entspricht.

4. Alkalische Protease nach einem der Ansprüche 1 bis 3, wobei die Homologiewerte ab mindestens 90% Identität jeweils für den Bereich gelten, der den Aminosäurepositionen 33 bis 470 gemäß SEQ ID NO. 5 oder 33 bis 470 gemäß SEQ ID NO. 8 entspricht.

5. Alkalische Protease nach einem der Ansprüche 1 bis 4, mit einer Aminosäuresequenz gemäß der Consensus-Sequenz von SEQ ID NO. 9, vorzugsweise im Bereich der Aminosäurepositionen 39 bis 587, besonders bevorzugt im Bereich der Aminosäurepositionen 39 bis 476.

6. Alkalische Protease nach einem der Ansprüche 1 bis 5, die von einer Nukleotidsequenz codiert wird, die zu der in SEQ ID NO. 3 angegebenen Nukleotidsequenz mindestens zu 85% und zunehmend bevorzugt zu mindestens 90%, 95%, 96%, 97%, 98%, 99% und ganz besonders bevorzugt zu 100% identisch ist, insbesondere für den Bereich, der den Nukleotidpositionen 97 bis 1746 gemäß SEQ ID NO. 3 entspricht, ganz besonders für den Bereich, der den Nukleotidpositionen 97 bis 1410 gemäß SEQ ID NO. 3 entspricht, oder die von einer Nukleotidsequenz codiert wird, die zu der in SEQ ID NO. 6 angegebenen Nukleotidsequenz mindestens zu 85% und zunehmend bevorzugt zu mindestens 90%, 95%, 96%, 97%, 98%, 99% und ganz besonders bevorzugt zu 100% identisch ist, insbesondere für den Bereich, der den Nukleotidpositionen 115 bis 1761 gemäß SEQ ID NO. 6 entspricht, ganz besonders für den Bereich, der den Nukleotidpositionen 115 bis 1428 gemäß SEQ ID NO. 6 entspricht.

7. Alkalische Protease nach einem der Ansprüche 1 bis 6, die aus einem natürlichen Habitat isolierbar ist oder die sich von einer aus einem natürlichen Habitat isolierbaren Nukleinsäure ableitet.

8. Alkalische Protease nach einem der Ansprüche 1 bis 7, die oder deren zugehörige Nukleinsäure aus einem Organismus stammt, der aus einem natürlichen Habitat isolierbar ist.

9. Alkalische Protease nach Anspruch 8, wobei es sich um einen Mikroorganismus handelt, vorzugsweise um einen Pilz oder ein Bakterium, hierunter bevorzugt um ein grampositives Bakterium und besonders bevorzugt um eines der Gattung *Bacillus.*

10. Von einer Alkalischen Protease nach einem der Ansprüche 1 bis 9 durch Fragmentierung oder Deletionsmutagenese abgeleitete Alkalische Protease oder Protein mit mindestens 100 und zunehmend bevorzugt mindestens 150, 200, 250 und ganz besonders bevorzugt mindestens 300 bereits im Ausgangsmolekül zusammenhängenden Aminosäuren.

11. Alkalische Protease oder Protein nach einem der Ansprüche 1 bis 10, abgeleitet von einer Alkalischen Protease oder einem Protein nach einem der Ansprüche 1 bis 10 durch Insertionsmutagenese, durch Substitutionsmutagenese und/oder durch Fusion mit mindestens einem anderen Protein.

12. Alkalische Protease oder Protein nach Anspruch 11 mit einem oder mehreren Aminosäureaustauschen in den Positionen 3, 4, 36, 42, 47, 56, 61, 69, 87, 96, 99, 101, 102, 104, 114, 118, 120, 130, 139, 141, 142, 154, 157, 188, 193, 199, 205, 211, 224, 229, 236, 237, 242, 243, 255 und 268 in der Zählung der Alkalischen Protease aus *Bacillus lentus,* zuzuordnen über das Alignment in Figur 1.

13. Alkalische Protease oder Protein nach einem der Ansprüche 1 bis 12, die beziehungsweise das zusätzlich stabilisiert ist.

14. Alkalische Protease oder Protein nach einem der Ansprüche 1 bis 13, die beziehungsweise das zusätzlich derivatisiert ist.

15. Alkalische Protease oder Protein nach einem der Ansprüche 1 bis 14, die beziehungsweise das wenigstens eine antigene Determinante mit einem der in den Ansprüchen 1 bis 14 bezeichneten Alkalischen Proteasen oder Proteinen gemeinsam hat, und zwar über mindestens eine der Epitop-Regionen, innerhalb derer die Positionen 3, 4, 36, 42, 47, 56, 61, 69, 87, 96, 99, 101, 102, 104, 114, 118, 120, 130, 139, 141, 142, 154, 157, 188, 193, 199, 205, 211, 224, 229, 236, 237, 242, 243, 255 und 268 in der Zählung der Alkalischen Protease aus *Bacillus lentus* liegen, zuzuordnen über das Alignment in Figur 1.

16. Nukleinsäure mit einer Nukleotidsequenz, die zu der in SEQ ID NO. 3 angegebenen Nukleotidsequenz mindestens zu 85% oder zu der in SEQ ID NO. 6 angegebenen Nukleotidsequenz mindestens zu 85% identisch ist.

17. Nukleinsäure nach Anspruch 16, deren Sequenz zu einer der angegebenen Nukleotidsequenzen zunehmend bevorzugt zu mindestens 90%, 95%, 96%, 97%, 98%, 99% und ganz besonders bevorzugt zu 100% identisch ist.

18. Nukleinsäure nach Anspruch 16 oder 17, wobei die Homologiewerte jeweils für den Bereich gelten, der den Nukleotidpositionen 97 bis 1746 gemäß SEQ ID NO. 3 beziehungsweise den Nukleotidpositionen 115 bis 1761 gemäß SEQ ID NO. 6 entspricht.

19. Nukleinsäure nach einem der Ansprüche 16 bis 18, wobei die Homologiewerte jeweils für den Bereich gelten, der den Nukleotidpositionen 97 bis 1410 gemäß SEQ ID NO. 3 beziehungsweise den Nukleotidpositionen 115 bis 1428 gemäß SEQ ID NO. 6 entspricht.

20. Nukleinsäure nach einem der Ansprüche 16 bis 19, die für eine Alkalische Protease oder ein Protein nach einem der Ansprüche 1 bis 16 codiert.

21. Nukleinsäure nach einem der Ansprüche 16 bis 20, von der eines oder vorzugsweise mehrere Codons durch synonyme Codons ersetzt sind.

22. Zelle eines Organismus, der natürlicherweise eine Nukleinsäure nach einem der Ansprüche 16 bis 21 enthält.

23. Zelle nach Anspruch 22, die natürlicherweise eine Protease oder ein Protein nach einem der Ansprüche 1 bis 15 exprimiert, vorzugsweise sekretiert.

24. Zelle nach Anspruch 22 oder 23, wobei es sich um einen Mikroorganismus handelt, vorzugsweise um einen Pilz oder ein Bakterium, hierunter bevorzugt um ein grampositives Bakterium und besonders bevorzugt um eines der Gattung *Bacillus* oder ein gramnegatives Bakterium der Gattung *Xanthomonas.*

25. Verfahren zur Identifizierung einer Alkalischen Protease nach einem der Ansprüche 1 bis 15, welches auf der Isolierung einer Nukleinsäure aus einem natürlich besiedelten Habitat beruht.

26. Verfahren nach Anspruch 25, unter Einsatz eines, vorzugsweise zwei einander entsprechenden Oligonukleotiden, die als PCR-Primer dienen können und von einer der beiden Sequenzen SEQ ID NO. 3 oder 6 abgeleitet sind.

27. Verfahren nach Anspruch 25 oder 26, wobei die isolierte Nukleinsäure kloniert, vorzugsweise exprimiert und besonders bevorzugt über die Protease-Aktivität des Expressionsprodukts als Protease identifiziert wird.

28. Vektor, der einen in den Ansprüchen 16 bis 21 bezeichneten Nukleinsäurebereich enthält.

29. Klonierungsvektor nach Anspruch 28.

30. Expressionsvektor nach Anspruch 28.

31. Zelle, die nach gentechnischer Modifizierung einen in den Ansprüchen 16 bis 21 bezeichneten Nukleinsäurebereich enthält.

32. Zelle nach Anspruch 31, wobei der genannte Nukleinsäurebereich auf einem Vektor liegt, insbesondere auf einem Vektor nach einem der Ansprüche 28 bis 30.

33. Zelle nach Anspruch 31 oder 32, die eine der in einem der Ansprüche 1 bis 15 bezeichneten Alkalischen Proteasen oder Proteine exprimiert, vorzugsweise sekretiert.

34. Zelle nach einem der Ansprüche 31 bis 33, bei der es sich um ein Bakterium handelt.

35. Zelle nach Anspruch 34, wobei es sich um ein gramnegatives Bakterium handelt, insbesondere eines der Gattungen *Escherichia coli, Klebsiella, Pseudomonas* oder *Xanthomonas,* insbesondere um Stämme von E. *coli* K12, E. coli B oder *Klebsiella* planticola, und ganz besonders um Derivate der Stämme *Escherichia coli* BL21 (DE3), *E. coli* RV308, *E. coli* DH5α, E.coli JM109, *E. coli* XL-1 oder *Klebsiella* planticola (Rf).

36. Zelle nach Anspruch 34, wobei es sich um ein grampositives Bakterium handelt, insbesondere eines der Gattungen *Bacillus,* Staphylococcus oder *Corynebacterium,* ganz besonders der Species *Bacillus lentus,* B*. licheniformis, B. amyloliquefaciens, B. subtilis,* B. globigii oder B. alcalophilus, Staphylococcus *carnosus* oder *Corynebacterium glutamicum.*

37. Zelle nach einem der Ansprüche 31 bis 33, wobei es sich um eine eukaryontische Zelle handelt, vorzugsweise eine der Gattung *Saccharomyces.*

38. Verfahren zur Herstellung einer Alkalischen Proteasen oder eines Proteins nach einem der Ansprüche 1 bis 15.

39. Verfahren nach Anspruch 38 unter Einsatz einer Nukleinsäure nach einem der Ansprüche 16 bis 21, vorzugsweise unter Einsatz eines Vektors nach einem der Ansprüche 28 bis 30, besonders bevorzugt unter Einsatz einer Zelle nach einem der Ansprüche 31 bis 37.

40. Verfahren nach Anspruch 38 oder 39, wobei die Nukleotidsequenz in einem oder vorzugsweise mehreren Codons an die Codon-Usage des Wirtsstamms angepaßt worden ist.

41. Mittel, enthaltend eine Alkalische Protease nach einem der Ansprüche 1 bis 15.

42. Mittel nach Anspruch 41, wobei es sich um ein Wasch- oder Reinigungsmittel handelt.

43. Mittel nach Anspruch 42, welches die Alkalische Protease in einer Menge von 2 µg bis 20 mg, vorzugsweise von 5 µg bis 17,5 mg, besonders bevorzugt von 20 µg bis 15 mg, ganz besonders bevorzugt von 50 µg bis 10 mg pro g des Mittels enthält.

44. Mittel nach Anspruch 42 oder 43, welches zusätzlich weitere Enzyme, insbesondere andere Proteasen, Amylasen, Cellulasen, Hemicellulasen, Oxidoreduktasen und/oder Lipasen enthält.

45. Mittel nach Anspruch 41, wobei es sich um ein Mittel zur Behandlung von Textilrohstoffen oder zur Textilpflege handelt.

46. Mittel nach Anspruch 41 zur Behandlung von Fasern oder Textilien mit natürlichen Bestandteilen, insbesondere von solchen mit Wolle oder Seide.

47. Verfahren zur maschinellen Reinigung von Textilien oder von harten Oberflächen, wobei in wenigstens einem der Verfahrensschritte eine Alkalische Protease nach einem der Ansprüche 1 bis 15 aktiv wird.

48. Verfahren nach Anspruch 47, wobei die Alkalische Protease in einer Menge von 40 µg bis 4 g, vorzugsweise von 50 µg bis 3 g, besonders bevorzugt von 100 µg bis 2 g und ganz besonders bevorzugt von 200 µg bis 1 g pro Anwendung eingesetzt wird.

49. Verfahren zur Behandlung von Textilrohstoffen oder zur Textilpflege, wobei in wenigstens einem der Verfahrensschritte eine Alkalische Protease nach einem der Ansprüche 1 bis 15 aktiv wird.

50. Verfahren nach Anspruch 49 für Textilrohstoffe, Fasern oder Textilien mit natürlichen Bestandteilen und ganz besonders für solche mit Wolle oder Seide.

51. Verwendung einer Alkalischen Protease nach einem der Ansprüche 1 bis 15 zur Reinigung von Textilien oder von harten Oberflächen.

52. Verwendung nach Anspruch 51, wobei die Alkalische Protease in einer Menge von 40 µg bis 4 g, vorzugsweise von 50 µg bis 3 g, besonders bevorzugt von 100 µg bis 2 g und ganz besonders bevorzugt von 200 µg bis 1 g pro Anwendung eingesetzt wird.

53. Mittel nach Anspruch 41, wobei es sich um ein Kosmetikum handelt.

## Claims

1. An alkaline protease whose amino acid sequence is identical to the amino acid sequence indicated in SEQ ID NO. 4 at least to 90% or is identical to the amino acid sequence indicated in SEQ ID NO. 7 at least to 87.5%.

2. The alkaline protease as claimed in claim 1, whose amino acid sequence is identical to the amino acid sequence indicated in SEQ ID NO. 4 increasingly preferably to at least 95%, 96%, 97%, 98%, 99% and very particularly preferably to 100% or is identical to the amino acid sequence indicated in SEQ ID NO. 7 increasingly preferably to at least 90%, 95%, 96%, 97%, 98%, 99% and very particularly preferably to 100%.

3. The alkaline protease as claimed in claim 1 or 2, where the homology values in each case apply for the region which corresponds to the amino acid positions 33 to 581 as in SEQ ID NO. 4 or 39 to 586 as in SEQ ID NO. 7.

4. The alkaline protease as claimed in one of claims 1 to 3, where the homology values from at least 90% identity in each case apply for the region which corresponds to the amino acid positions 33 to 470 as in SEQ ID NO. 5 or 33 to 470 as in SEQ ID NO. 8.

5. The alkaline protease as claimed in one of claims 1 to 4, having an amino acid sequence as in the consensus sequence of SEQ ID NO. 9, preferably in the range of the amino acid positions 39 to 587, particularly preferably in the range of the amino acid positions 39 to 476.

6. The alkaline protease as claimed in one of claims 1 to 5, which is encoded by a nucleotide sequence which is identical to the nucleotide sequence indicated in SEQ ID NO. 3 at least to 85% and increasingly preferably to at least 90%, 95%, 96%, 97%, 98%, 99% and very particularly preferably to 100%, in particular for the region which corresponds to the nucleotide positions 97 to 1746 as in SEQ .ID NO. 3, very particularly for the region which corresponds to the nucleotide positions 97 to 1410 as in SEQ ID NO. 3, or which is encoded by a nucleotide sequence which is identical to the nucleotide sequence indicated in SEQ ID NO. 6 at least to 85% and increasingly preferably to at least 90%, 95%, 96%, 97%, 98%, 99% and very particularly preferably to 100%, in particular for the region which corresponds to the nucleotide positions 115 to 1761 as in SEQ ID NO. 6, very particularly for the region which corresponds to the nucleotide positions 115 to 1428 as in SEQ ID NO. 6.

7. The alkaline protease as claimed in one of claims 1 to 6, which is isolable from a natural habitat or which is derived from a nucleic acid isolable from a natural habitat.

8. The alkaline protease as claimed in one of claims 1 to 7, which or whose associated nucleic acid originates from an organism which is isolable from a natural habitat

9. The alkaline protease as claimed in claim 8, where it is a microorganism, preferably a fungus or a bacterium, among these preferably a gram-positive bacterium and particularly preferably one of the genus *Bacillus.*

10. An alkaline protease or protein derived from an alkaline protease as claimed in one of claims 1 to 9 by fragmentation or deletion mutagenesis having at least 100 and increasingly preferably at least 150, 200, 250 and very particularly preferably at least 300 amino acids already connected in the starting molecule.

11. The alkaline protease or protein as claimed in one of claims 1 to 10, derived from an alkaline protease or a protein as claimed in one of claims 1 to 10 by insertion mutagenesis, by substitution mutagenesis and/or by fusion with at least one other protein.

12. The alkaline protease or protein as claimed in claim 11 having one or more amino acid replacements in the positions 3, 4, 36, 42, 47, 56, 61, 69, 87, 96, 99, 101, 102, 104, 114, 118, 120, 130, 139, 141, 142, 154, 157, 188, 193, 199, 205, 211, 224, 229, 236, 237, 242, 243, 255 and 268 in the numbering of the alkaline protease from *Bacillus lentus,* to be assigned by means of the alignment in figure 1.

13. The alkaline protease or protein as claimed in one of claims 1 to 12, which is additionally stabilized.

14. The alkaline protease or protein as claimed in one of claims 1 to 13, which is additionally derivatized.

15. The alkaline protease or protein as claimed in one of claims 1 to 14, which has at least one antigenic determinant in common with one of the alkaline proteases or proteins designated in claims 1 to 14, namely over at least one of the epitope regions within which the positions 3, 4, 36, 42, 47, 56, 61; 69, 87, 96, 99, 101, 102, 104, 114, 118, 120, 130, 139, 141, 142, 154, 157, 188, 193, 199, 205, 211, 224, 229, 236, 237, 242, 243, 255 and 268 lie in the numbering of the alkaline protease from Bacillus lentus, to be assigned by means of the alignment in figure 1.

16. A nucleic acid having a nucleotide sequence, which is identical to the nucleotide sequence indicated in SEQ ID NO. 3 at least to 85% or to the nucleotide sequence indicated in SEQ ID NO. 6 at least to 85%.

17. The nucleic acid as claimed in claim 16, whose sequence is identical increasingly preferably to at least 90%, 95%, 96%, 97%, 98%, 99% and very particularly preferably to 100% to one of the nucleotide sequences indicated.

18. The nucleic acid as claimed in claim 16 or 17, where the homology values in each case apply for the region which corresponds to the nucleotide positions 97 to 1746 as in SEQ **ID** NO, 3 or to the nucleotide positions 115 to 1761 as in SEQ ID NO. 6.

19. The nucleic acid as claimed in one of claims 16 to 18, where the homology values in each case apply for the region which corresponds to the nucleotide positions 97 to 1410 as in SEQ ID NO. 3 or to the nucleotide positions 115 to 1428 as in SEQ ID NO. 6.

20. The nucleic acid as claimed in one of claims 16 to 19, which codes for an alkaline protease or a protein as claimed in one of claims 1 to 16.

21. The nucleic acid as claimed in one of claims 16 to 20, of which one or preferably more codons are replaced by synonymous, codons.

22. A cell of an organism which naturally contains a nucleic acid as claimed in one of claims 16 to 21.

23. The cell as claimed in claim 22, which naturally expresses, preferably secretes, a protease or a protein as claimed in one of claims 1 to 15.

24. The cell as claimed in claim 22 or 23, which is a microorganism, preferably a fungus or a bacterium, among them preferably a gram-positive bacterium and particularly preferably one of the genus *Bacillus* or a gram-negative bacterium of the genus *Xanthomonas.*

25. A method for the identification of an alkaline protease as claimed in one of claims 1 to 15, which is based on the isolation of a nucleic acid from a naturally colonized habitat.

26. The method as claimed in claim 25, using one, preferably two, oligonucleotides corresponding to one another, which can serve as PCR primers and are derived from one of the two sequences SEQ ID NO. 3 or 6.

27. The method as claimed in claim 25 or 26, where the isolated nucleic acid is cloned, preferably expressed and particularly preferably identified as a protease by means of the protease activity of the expression product.

28. A vector which contains a nucleic acid region designated in claims 16 to 21.

29. A cloning vector as claimed in claim 28.

30. An expression vector as claimed in claim 28.

31. A cell which after recombinant DNA modification contains a nucleic acid region designated in claims 16 to 21.

32. The cell as claimed in claim 31, where said nucleic acid region lies on a vector, in particular on a vector as claimed in one of claims 28 to 30.

33. The cell as claimed in claim 31 or 32, which expresses, preferably secretes, one of the alkaline proteases or proteins designated in one of claims 1 to 15.

34. The cell as claimed in one of claims 31 to 33, which is a bacterium.

35. The cell as claimed in claim 34, which is a gram-negative bacterium, in particular one of the genera *Escherichia coli, Klebsiella, Pseudomonas* or *Xanthomonas,* in particular strains of *E. coli* K12, E. coli B or *Klebsiella* planticola, and very particularly derivatives of the strains *Escherichia coli* BL21 (DE3), E. *coli* RV308, E. *coli* DH5α, E.coli JM109, *E. coli* XL-1 or *Klebsiella* planticola (Rf).

36. The cell as claimed in claim 34, which is a gram-positive bacterium, in particular one of the genera *Bacillus,* Staphylococcus or *Corynebacterium,* very particularly of the species *Bacillus lentus,* B. *licheniformis, B. amyloliquefaciens, B. subtilis,* B. globigii or B. alcalophilus, Staphylococcus *carnosus* or *Corynebacterium glutamicum.*

37. The cell as claimed in one of claims 31 to 33, which is a eukaryotic cell, preferably one of the genus *Saccharomyces.*

38. A method for the production of an alkaline protease or of a protein as claimed in one of claims 1 to 15.

39. The method as claimed in claim 38 using a nucleic acid as claimed in one of claims 16 to 21, preferably using a vector as claimed in one of claims 28 to 30, particularly preferably using a cell as claimed in one of claims 31 to 37.

40. The method as claimed in claim 38 or 39, where the nucleotide sequence has been adapted in one or preferably more codons to the codon usage of the host strain.

41. A composition comprising an alkaline protease as claimed in one of claims 1 to 15.

42. The composition as claimed in claim 41, which is a detergent or cleaner.

43. The composition as claimed in claim 42, which contains the alkaline protease in an amount from 2 µg to 20 mg, preferably from 5 µg to 17.5 mg, particularly preferably from 20 µg to 15 mg, very particularly preferably from 50 µg to 10 mg per g of the composition.

44. The composition as claimed in claim 42 or 43, which additionally contains further enzymes, in particular other proteases, amylases, cellulases, hemicellulases, oxidoreductases and/or lipases.

45. The composition as claimed in claim 41, which is a composition for the treatment of textile raw materials or for textile care.

46. The composition as claimed in claim 41 for the treatment of fibers or textiles containing natural constituents, in particular of those containing wool or silk.

47. A method for the mechanical cleaning of textiles or of hard surfaces, where in at least one of the method steps an alkaline protease as claimed in one of claims 1 to 15 becomes active.

48. The method as claimed in claim 47, where the alkaline protease is employed in an amount from 40 µg to 4 g, preferably from 50 µg to 3 g, particularly preferably from 100 µg to 2 g and very particularly preferably from 200 µg to 1 g per application.

49. A method for the treatment of textile raw materials or for textile care, where in at least one of the method steps an alkaline protease as claimed in one of claims 1 to 15 becomes active.

50. The method as claimed in claim 49 for textile raw materials, fibers or textiles containing natural constituents and very particularly for those containing wool or silk.

51. The use of an alkaline protease as claimed in one of claims 1 to 15 for the cleaning of textiles or of hard surfaces.

52. The use as claimed in claim 51, where the alkaline protease is employed in an amount from 40 µg to 4 g, preferably from 50 µg to 3 g, particularly preferably from 100 µg to 2 g and very particularly preferably from 200 µg to 1 g per application.

53. The composition as claimed in claim 41, which is a cosmetic.

## Revendications

1. Protéase alcaline dont la séquence d'acides aminés est identique à raison d'au moins 90% à la séquence d'acides aminés indiquée dans la SEQ ID NO. 4 ou à raison d'au moins 87,5% à la séquence d'acides aminés indiquée dans la SEQ ID NO. 7.

2. Protéase alcaline selon la revendication 1, dont la séquence d'acides aminés est identique, de manière de plus en plus préférée, à raison d'au moins 95%, 96%, 97%, 98%, 99% et de manière tout particulièrement préférée à raison de 100% à la séquence d'acides aminés indiquée dans la SEQ ID NO. 4 ou est identique, de manière de plus en plus préférée, à raison d'au moins 90%, 95%, 96%, 97%, 98%, 99% et de manière tout particulièrement préférée à raison de 100% à la séquence d'acides aminés indiquée dans la SEQ ID NO. 7.

3. Protéase alcaline selon la revendication 1 ou 2, les valeurs d'homologie valant à chaque fois pour la plage qui correspond aux positions d'acides aminés 33 à 581 selon la SEQ ID NO. 4 ou 39 à 586 selon la SEQ ID NO. 7.

4. Protéase alcaline selon l'une quelconque des revendications 1 à 3, les valeurs d'homologie à partir d'une identité d'au moins 90% valant à chaque fois pour la plage qui correspond aux positions d'acides aminés 33 à 470 selon la SEQ ID NO. 5 ou 33 à 470 selon la SEQ ID NO. 8.

5. Protéase alcaline selon l'une quelconque des revendications 1 à 4, avec une séquence d'acides aminés selon la séquence de consensus de la SEQ ID NO. 9, de préférence dans la plage des positions d'acides aminés 39 à 587, de manière particulièrement préférée dans la plage des positions d'acides aminés 39 à 476.

6. Protéase alcaline selon l'une quelconque des revendications 1 à 5, qui est codée par une séquence de nucléotides, qui est identique à raison d'au moins 85%, de manière de plus en plus préférée à raison d'au moins 90%, 95%, 96%, 97%, 98%, 99% et de manière tout particulièrement préférée à raison de 100% à la séquence de nucléotides indiquée dans la SEQ ID NO. 3, en particulier pour la plage qui correspond aux positions de nucléotides 97 à 1746 selon la SEQ ID NO. 3, de manière tout particulièrement préférée pour la plage qui correspond aux positions de nucléotides 97 à 1410 selon la SEQ ID NO. 3, ou qui est codée par une séquence de nucléotides qui est identique, à raison d'au moins 85% et de manière de plus en plus préférée à raison d'au moins 90%, 95%, 96%, 97%, 98%, 99% et de manière tout particulièrement préférée à raison de 100% à la séquence de nucléotides indiquée dans la SEQ ID NO. 6 en particulier pour la plage qui correspond aux positions de nucléotides 115 à 1761 selon la SEQ ID NO. 6, de manière tout particulièrement préférée pour la plage qui correspond aux positions de nucléotides 115 à 1428 selon la SEQ ID NO. 6.

7. Protéase alcaline selon l'une quelconque des revendications 1 à 6, qui peut être isolée à partir d'un habitat naturel ou qui est dérivée d'un acide nucléique isolable à partir d'un habitat naturel.

8. Protéase alcaline selon l'une quelconque des revendications 1 à 7, qui provient ou dont les acides nucléiques correspondants proviennent d'un organisme qui peut être isolé à partir d'un habitat naturel.

9. Protéase alcaline selon la revendication 8, où il s'agit d'un microorganisme, de préférence d'un champignon ou d'une bactérie, parmi lesquelles de préférence une bactérie gram-positive et de manière tout particulièrement préférée de l'espèce Bacillus.

10. Protéase alcaline ou protéine dérivée d'une protéase alcaline selon l'une quelconque des revendications 1 à 9 par fragmentation ou mutagenèse de délétion comprenant au moins 100 et, de manière de plus en plus préférée, au moins 150, 200, 250 et de manière tout particulièrement préférée au moins 300 acides aminés déjà associés dans la molécule de départ.

11. Protéase alcaline ou protéine selon l'une quelconque des revendications 1 à 10, dérivée d'une protéase alcaline ou d'une protéine selon l'une quelconque des revendications 1 à 10 par mutagenèse d'insertion, par mutagenèse de substitution et/ou par fusion avec au moins une autre protéine.

12. Protéase alcaline ou protéine selon la revendication 11 présentant un ou plusieurs échanges d'acides aminés dans les positions 3, 4, 36, 42, 47, 56, 61, 69, 87, 96, 99, 101, 102, 104, 114, 118, 120, 130, 139, 141, 142, 154, 157, 188, 193, 199, 205, 211, 224, 229, 236, 237, 242, 243, 255 et 268 dans la numérotation de la protéase alcaline de Bacillus lentus, à attribuer via l'alignement dans la Figure 1.

13. Protéase alcaline ou protéine selon l'une quelconque des revendications 1 à 12, qui est en outre stabilisée.

14. Protéase alcaline ou protéine selon l'une quelconque des revendications 1 à 13, qui est en outre dérivée.

15. Protéase alcaline ou protéine selon l'une quelconque des revendications 1 à 14, qui a au moins un déterminant antigène en commun avec une des protéases alcalines ou protéines indiquées dans l'une quelconque des revendications 1 à 14 et ce via au moins une des régions d'épitope, dans lesquelles se trouvent les positions 3, 4, 36, 42, 47, 56, 61, 69, 87, 96, 99, 101, 102, 104, 114, 118, 120, 130, 139, 141, 142, 154, 157, 188, 193, 199, 205, 211, 224, 229, 236, 237, 242, 243, 255 et 268 dans la numérotation de la protéase alcaline de Bacillus lentus, à attribuer via l'alignement dans la Figure 1.

16. Acide nucléique avec une séquence de nucléotides qui est identique à raison d'au moins 85% à la séquence de nucléotides indiquée dans la SEQ ID NO. 3 ou à raison d'au moins 85% à la séquence de nucléotides indiquée dans la SEQ ID NO. 6.

17. Acide nucléique selon la revendication 16, dont la séquence est identique à une des séquences de nucléotides indiquées, de manière de plus en plus préférée à raison d'au moins 90%, 95%, 96%, 97%, 98%, 99% et de manière tout particulièrement préférée à raison de 100%.

18. Acide nucléique selon la revendication 16 ou 17, les valeurs d'homologie valant à chaque fois pour la plage qui correspond aux positions de nucléotides 97 à 1746 selon la SEQ ID NO. 3 ou aux positions de nucléotides 115 à 1761 selon la SEQ ID NO. 6.

19. Acide nucléique selon l'une quelconque des revendications 16 à 18, les valeurs d'homologie valant à chaque fois pour la plage qui correspond aux positions de nucléotides 97 à 1410 selon la SEQ ID NO. 3 ou aux positions de nucléotides 115 à 1428 selon la SEQ ID NO. 6.

20. Acide nucléique selon l'une quelconque des revendications 16 à 19, qui code pour une protéase alcaline ou une protéine selon l'une quelconque des revendications 1 à 16.

21. Acide nucléique selon l'une quelconque des revendications 16 à 20, dont un ou de préférence plusieurs codons sont remplacés par des codons synonymes.

22. Cellule d'un organisme qui contient, naturellement, un acide nucléique selon l'une quelconque des revendications 16 à 21.

23. Cellule selon la revendication 22, qui exprime, de préférence sécrète, naturellement une protéase ou une protéine selon l'une quelconque des revendications 1 à 15.

24. Cellules selon la revendication 22 ou 23, où il s'agit d'un microorganisme, de préférence d'un champignon ou d'une bactérie, parmi lesquelles de préférence une bactérie gram-positive et de manière tout particulièrement préférée de l'espèce Bacillus ou d'une bactérie gram-négative de l'espèce Xanthomonas.

25. Procédé pour identifier une protéase alcaline selon l'une quelconque des revendications 1 à 15, qui repose sur l'isolement d'un acide nucléique d'un habitat peuplé naturellement.

26. Procédé selon la revendication 25 en utilisant un oligonucléotide, de préférence deux oligonucléotides qui correspondent l'un à l'autre, qui peuvent servir d'amorce PCR et qui sont dérivés d'une des deux séquences SEQ ID NO. 3 ou 6.

27. Procédé selon la revendication 25 ou 26, l'acide nucléique isolé étant cloné, de préférence exprimé et de manière particulièrement préférée identifié via l'activité de protéase du produit d'expression comme protéase.

28. Vecteur, qui contient une plage d'acides nucléique désignée dans l'une quelconque des revendications 16 à 21.

29. Vecteur de clonage selon la revendication 28.

30. Vecteur d'expression selon la revendication 28.

31. Cellule, qui contient, après modification par génie génétique, une plage d'acides nucléiques désignée dans les revendications 16 à 21.

32. Cellule selon la revendication 31, la plage d'acides nucléiques mentionnée se trouvant sur un vecteur, en particulier sur un vecteur selon l'une quelconque des revendications 28 à 30.

33. Cellule selon la revendication 31 ou 32, qui exprime, de préférence sécrète, une des protéases alcalines ou protéines désignées dans l'une quelconque des revendications 1 à 15.

34. Cellule selon l'une quelconque des revendications 31 à 33, où il s'agit d'une bactérie.

35. Cellule selon la revendication 34, où il s'agit d'une bactérie gram-négative, en particulier d'une bactérie des espèces Escherichia coli, Klebsiella, Pseudomonas ou Xanthomonas, en particulier de la souche E. coli K12, E. coli B ou Klebsiella planticola, et de manière tout particulièrement préférée de dérivés des souches Escherichia coli BL21 (DE3), E. coli RV308, E. coli DH5α, E. coli JM 109, E. coli XL-1 ou Klebsiella planticola (Rf).

36. Cellule selon la revendication 34, où il s'agit d'une bactérie gram-positive, en particulier d'une bactérie des espèces Bacillus, Staphylococcus ou Corynebacterium, de manière tout particulièrement préférée des espèces Bacillus lentus, B. licheniformis, B. amylolique faciens, B. subtilis, B. globigii ou B. alcalophilus, Staphylococcus carnosus ou Corynebacterium glutamicum.

37. Cellule selon l'une quelconque des revendications 31 à 33, où il s'agit d'une cellule eucaryote, de préférence d'une cellule de l'espèce Saccharomyces.

38. Procédé de production d'une protéase alcaline ou d'une protéine selon l'une quelconque des revendications 1 à 15.

39. Procédé selon la revendication 38 avec utilisation d'un acide nucléique selon l'une quelconque des revendications 16 à 21, de préférence avec utilisation d'un vecteur selon l'une quelconque des revendications 28 à 30, de manière particulièrement préférée avec utilisation d'une cellule selon l'une quelconque des revendications 31 à 37.

40. Procédé selon la revendication 38 ou 39, la séquence de nucléotides ayant été adaptée dans un ou de préférence plusieurs codons au Codon-Usage de la souche hôte.

41. Agent, contenant une protéase alcaline selon l'une quelconque des revendications 1 à 15.

42. Agent selon la revendication 41, où il s'agit d'un agent de lavage ou de nettoyage.

43. Agent selon la revendication 42, qui contient la protéase alcaline en une quantité de 2 µg à 20 mg, de préférence de 5 µg à 17,5 mg, de manière particulièrement préférée de 20 µg à 15 mg, de manière tout particulièrement préférée de 50 µg à 10 mg par g de l'agent.

44. Agent selon la revendication 42 ou 43, qui contient en outre d'autres enzymes, en particulier d'autres protéases, amylases, cellulases, hémicellulases, oxydoréductases et/ou lipases.

45. Agent selon la revendication 41, où il s'agit d'un agent destiné au traitement des matières premières pour textiles ou à l'entretien du textile.

46. Agent selon la revendication 41 pour le traitement de fibres ou de textiles avec des constituants naturels, en particulier ceux avec de la laine ou de la soie.

47. Procédé pour le nettoyage en machine de textiles ou de surfaces dures, dans lequel, dans au moins une des étapes de procédé, une protéase alcaline selon l'une quelconque des revendications 1 à 15 devient active.

48. Procédé selon la revendication 47, dans lequel est utilisée la protéase alcaline en une quantité de 40 µg à 4 g, de préférence de 50 µg à 3 g, de manière particulièrement préférée de 100 µg à 2 g, de manière tout particulièrement préférée de 200 µg à 1 g par utilisation.

49. Procédé pour le traitement de matières premières pour textiles ou pour l'entretien de textiles, dans lequel, dans au moins une des étapes de procédé, une protéase alcaline selon l'une quelconque des revendications 1 à 15 devient active.

50. Procédé selon la revendication 49 pour des matières premières pour textiles, des fibres ou des textiles avec des constituants naturels et tout particulièrement ceux avec de la laine ou de la soie.

51. Utilisation d'une protéase alcaline selon l'une quelconque des revendications 1 à 15 pour le nettoyage de textiles ou de surfaces dures.

52. Utilisation selon la revendication 51, dans laquelle est utilisée la protéase alcaline en une quantité de 40 µg à 4 g, de préférence de 50 µg à 3 g, de manière particulièrement préférée de 100µg à 2 g, de manière tout particulièrement préférée de 200 µg à 1 g par utilisation.

53. Agent selon la revendication 41, où il s'agit d'un agent cosmétique.


### SEQUENZPROTOKOLL

<110> Henkel Kommanditgesellschaft auf Aktien
<120> Neue Alkalische Proteasen und Wasch- und Reinigungsmittel, enthaltend diese neuen Alkalischen Proteasen
<130> H 05925 PCT
<140>
   <141>
<150> DE102004019751.2
   <151> 2004-04-23
<160> 13
<170> PatentIn Ver. 2.1
<210> 1
   <211> 16
   <212> DNA
   <213> Künstliche Sequenz
<220>
   <223> Beschreibung der künstlichen Sequenz: Primer M13f
<400> 1
   gtaaaacgac ggccag 16
<210> 2
   <211> 17
   <212> DNA
   <213> Künstliche Sequenz
<220>
   <223> Beschreibung der künstlichen Sequenz: Primer M13r
<400> 2
   caggaaacag ctatgac 17
<210> 3
   <211> 1777
   <212> DNA
   <213> Unbekannt
<220>
   <223> Beschreibung des unbekannten Organismus:
   DNA-Isolat
<220>
   <221> sig_peptide
   <222> (1)..(96)
<220>
   <221> CDS
<222> (1)..(1746)
<400> 3
   atg tct cat gat tcg caa ccc cgt ttg cgt cag cgt gca ttg gtt gta      48
   Met Ser His Asp Ser Gln Pro Arg Leu Arg Gln Arg Ala Leu Val Val
   1               5                   10                  15
   ctc ggc gcg tcc gtc ctg tcc acc ctg ctg ctg gcc gcc ccg gca ttc      96
   Leu Gly Ala Ser Val Leu Ser Thr Leu Leu Leu Ala Ala Pro Ala Phe
               20                  25                  30
   gcc ggc gat gtg cag ctg agc ggc ttg tcg tcg gca ccg acg cac cag      144
   Ala Gly Asp Val Gln Leu Ser Gly Leu Ser Ser Ala Pro Thr His Gln
           35                  40                  45
   cgt ttc atc gtc aaa tac aag gat ggc gcc aac ctg gtc gcc acc ccg      192
   Arg Phe Ile Val Lys Tyr Lys Asp Gly Ala Asn Leu Val Ala Thr Pro
       50                  55                  60
   acc gca ctg gcc agc tcg ttg aag gcg gcg gcc tcg gcc gta ccg gct      240
   Thr Ala Leu Ala Ser Ser Leu Lys Ala Ala Ala Ser Ala Val Pro Ala
   65                  70                  75                  80
   gcg cag ggt cgc gcg ctg ggc ctg cag aag ctg cgc cag ctg gcc att      288
   Ala Gln Gly Arg Ala Leu Gly Leu Gln Lys Leu Arg Gln Leu Ala Ile
                   85                  90                  95
   ggg ccg acc gtg gtc aag gcc gac cgt ccg ctg gat gcg gcc gag tcg      336
   Gly Pro Thr Val Val Lys Ala Asp Arg Pro Leu Asp Ala Ala Glu Ser
               100                 105                 110
   gaa ctg ctg atg cgc cgc ctg gcg gcc gac ccg aac gtg gaa tac gtc      384
   Glu Leu Leu Met Arg Arg Leu Ala Ala Asp Pro Asn Val Glu Tyr Val
           115                 120                 125
   gaa gtc gat cag ctg atg cac gcc acc ctg gtg ccc aac gac gcg cgc      432
   Glu Val Asp Gln Leu Met His Ala Thr Leu Val Pro Asn Asp Ala Arg
       130                 135                 140
   ctg tcc gag cag tgg ggc ttc ggc acc agc aac gcc tcg atc aac gtg      480
   Leu Ser Glu Gln Trp Gly Phe Gly Thr Ser Asn Ala Ser Ile Asn Val
   145                 150                 155                 160
   cgc ccg gca tgg gac aag gcc acc ggt acc ggc gtg gtg gtg gcg gtg      528
   Arg Pro Ala Trp Asp Lys Ala Thr Gly Thr Gly Val Val Val Ala Val
                   165                 170                 175
   atc gac acc ggc atc acc aac cat ccg gac ctc aac gcc aac atc ctg      576
   Ile Asp Thr Gly Ile Thr Asn His Pro Asp Leu Asn Ala Asn Ile Leu
               180                 185                 190
   ccc ggc tat gac ttc atc agc gac gcg gcg atg gcg cgc gat ggt ggc      624
   Pro Gly Tyr Asp Phe Ile Ser Asp Ala Ala Met Ala Arg Asp Gly Gly
           195                 200                 205
   ggc cgt gac aac aac gcg aac gat gaa ggc gac tgg tac gcc gcc aac      672
   Gly Arg Asp Asn Asn Ala Asn Asp Glu Gly Asp Trp Tyr Ala Ala Asn
       210                 215                 220
   gag tgc ggc tcg ggc att ccg gcg tcg aac tcg agc tgg cac ggt acc    720
   Glu Cys Gly Ser Gly Ile Pro Ala Ser Asn Ser Ser Trp His Gly Thr
   225                 230                 235                 240
   cac gta gcc ggc acc atc gcg gcg gtg acc aac aac agc act ggc gtg    768
   His Val Ala Gly Thr Ile Ala Ala Val Thr Asn Asn Ser Thr Gly Val
                   245                 250                 255
   gcc ggt acg gca ttc aac gcg aag gtc gtg ccg gtg cgt gtg ctc ggc    816
   Ala Gly Thr Ala Phe Asn Ala Lys Val Val Pro Val Arg Val Leu Gly
               260                 265                 270
   aag tgc ggc ggt tac acc tcc gac atc gcc gat gcg atc gtg tgg gcc    864
   Lys Cys Gly Gly Tyr Thr Ser Asp Ile Ala Asp Ala Ile Val Trp Ala
           275                 280                 285
   tcc ggc ggc acg gtc agc ggc gtg ccg gcc aat gcc aac ccg gcc gaa    912
   Ser Gly Gly Thr Val Ser Gly Val Pro Ala Asn Ala Asn Pro Ala Glu
       290                 295                 300
   gtg atc aac atg tcg ctg ggc ggc ggt ggc acc tgc tcg acc acc tac    960
   Val Ile Asn Met Ser Leu Gly Gly Gly Gly Thr Cys Ser Thr Thr Tyr
   305                 310                 315                 320
   cag aac gcg atc aac ggc gcg gtg tcg cgc ggc acg acg gtg gtg gtg    1008
   Gln Asn Ala Ile Asn Gly Ala Val Ser Arg Gly Thr Thr Val Val Val
                   325                 330                 335
   gcg gcg ggc aac agc aac acc aac gtg tcc tcg tcg gtg ccg gcc aac    1056
   Ala Ala Gly Asn Ser Asn Thr Asn Val Ser Ser Ser Val Pro Ala Asn
               340                 345                 350
   tgc gcc aac gtg atc gcg gtg gcg gcc acg acg tcg gcc ggc gcg cgc    1104
   Cys Ala Asn Val Ile Ala Val Ala Ala Thr Thr Ser Ala Gly Ala Arg
           355                 360                 365
   gcg agc ttc tcg aac tac ggt gcg ggc atc gat att tcg gcg ccg ggc    1152
   Ala Ser Phe Ser Asn Tyr Gly Ala Gly Ile Asp Ile Ser Ala Pro Gly
       370                 375                 380
   cag gcg atc ctg tcc acg ctc aac agc ggt acg acg gtg ccg ggc acg    1200
   Gln Ala Ile Leu Ser Thr Leu Asn Ser Gly Thr Thr Val Pro Gly Thr
   385                 390                 395                 400
   gcg tcc tac gcg tcc tac aac ggg acg tcg atg gcg gcg ccg cac gtg    1248
   Ala Ser Tyr Ala Ser Tyr Asn Gly Thr Ser Met Ala Ala Pro His Val
                   405                 410                 415
   gcc ggc gtg gtg gcg ctg gtg cag tcg gtg gca ccg acg gcg ttg acg    1296
   Ala Gly Val Val Ala Leu Val Gln Ser Val Ala Pro Thr Ala Leu Thr
               420                 425                 430
   ccg gcg gcg atc gag acg ttg ctg aag aac acg gca cgg gca ttg ccg    1344
   Pro Ala Ala Ile Glu Thr Leu Leu Lys Asn Thr Ala Arg Ala Leu Pro
           435                 440                 445
   ggc gca tgc agc ggt ggg tgc ggc gcg ggc atc gtg gac gcc gat gcg    1392
   Gly Ala Cys Ser Gly Gly Cys Gly Ala Gly Ile Val Asp Ala Asp Ala
       450                 455                 460
   gcg gtc acg gcg gcg ctg ggc ggg acg aat ccg aac ccg ggc acg ggg      1440
   Ala Val Thr Ala Ala Leu Gly Gly Thr Asn Pro Asn Pro Gly Thr Gly
   465                 470                 475                 480
   acg gtg ctg cag aac aat gtg ccg gtg agc ggt ctg ggc gcg gcc agc      1488
   Thr Val Leu Gln Asn Asn Val Pro Val Ser Gly Leu Gly Ala Ala Ser
                   485                 490                 495
   ggg gca tcg ctg tcc tat acg gtg gtg gtg ccg tcg ggc cgt tcg cag      1536
   Gly Ala Ser Leu Ser Tyr Thr Val Val Val Pro Ser Gly Arg Ser Gln
               500                 505                 510
   ctg aag gtg agc atc gcc ggt ggc agt ggt gat gcg gat ctg tac gtg      1584
   Leu Lys Val Ser Ile Ala Gly Gly Ser Gly Asp Ala Asp Leu Tyr Val
           515                 520                 525
   cgt tcg ggc agc gcg ccg acc gac acg gtg tac aac tgc cgt ccg tac      1632
   Arg Ser Gly Ser Ala Pro Thr Asp Thr Val Tyr Asn Cys Arg Pro Tyr
       530                 535                 540
   ctg agc ggc aac aac gag acc tgc acg atc act tca ccg gcg gcc ggt      1680
   Leu Ser Gly Asn Asn Glu Thr Cys Thr Ile Thr Ser Pro Ala Ala Gly
   545                 550                 555                 560
   acc tgg cac gtg cgg gtg aag ggc tac tcg acc ttc tcc ggg gtc acc      1728
   Thr Trp His Val Arg Val Lys Gly Tyr Ser Thr Phe Ser Gly Val Thr
                   565                 570                 575
   ctg acc gcg cag tac tga gcccagatcc ctctccaatg ggcacgcccc g           1777
   Leu Thr Ala Gln Tyr
               580
<210> 4
   <211> 581
   <212> PRT
   <213> Unbekannt
   <223> Beschreibung des unbekannten Organismus:
   DNA-Isolat
<400> 4
   Met Ser His Asp Ser Gln Pro Arg Leu Arg Gln Arg Ala Leu Val Val
    1               5                  10                  15
   Leu Gly Ala Ser Val Leu Ser Thr Leu Leu Leu Ala Ala Pro Ala Phe
               20                  25                  30
   Ala Gly Asp Val Gln Leu Ser Gly Leu Ser Ser Ala Pro Thr His Gln
           35                  40                  45
   Arg Phe Ile Val Lys Tyr Lys Asp Gly Ala Asn Leu Val Ala Thr Pro
       50                  55                  60
   Thr Ala Leu Ala Ser Ser Leu Lys Ala Ala Ala Ser Ala Val Pro Ala
   65                  70                  75                  80
   Ala Gln Gly Arg Ala Leu Gly Leu Gln Lys Leu Arg Gln Leu Ala Ile
                   85                  90                  95
   Gly Pro Thr Val Val Lys Ala Asp Arg Pro Leu Asp Ala Ala Glu Ser
               100                 105                 110
   Glu Leu Leu Met Arg Arg Leu Ala Ala Asp Pro Asn Val Glu Tyr Val
           115                 120                 125
   Glu Val Asp Gln Leu Met His Ala Thr Leu Val Pro Asn Asp Ala Arg
   130 135 140
   Leu Ser Glu Gln Trp Gly Phe Gly Thr Ser Asn Ala Ser Ile Asn Val
   145 150 155 160
   Arg Pro Ala Trp Asp Lys Ala Thr Gly Thr Gly Val Val Val Ala Val
   165 170 175
   Ile Asp Thr Gly Ile Thr Asn His Pro Asp Leu Asn Ala Asn Ile Leu
   180 185 190
   Pro Gly Tyr Asp Phe Ile Ser Asp Ala Ala Met Ala Arg Asp Gly Gly
   195 200 205
   Gly Arg Asp Asn Asn Ala Asn Asp Glu Gly Asp Trp Tyr Ala Ala Asn
   210 215 220
   Glu Cys Gly Ser Gly Ile Pro Ala Ser Asn Ser Ser Trp His Gly Thr
   225 230 235 240
   His Val Ala Gly Thr Ile Ala Ala Val Thr Asn Asn Ser Thr Gly Val
   245 250 255
   Ala Gly Thr Ala Phe Asn Ala Lys Val Val Pro Val Arg Val Leu Gly
   260 265 270
   Lys Cys Gly Gly Tyr Thr Ser Asp Ile Ala Asp Ala Ile Val Trp Ala
   275 280 285
   Ser Gly Gly Thr Val Ser Gly Val Pro Ala Asn Ala Asn Pro Ala Glu
   290 295 300
   Val Ile Asn Met Ser Leu Gly Gly Gly Gly Thr Cys Ser Thr Thr Tyr
   305 310 315 320
   Gln Asn Ala Ile Asn Gly Ala Val Ser Arg Gly Thr Thr Val Val Val
   325 330 335
   Ala Ala Gly Asn Ser Asn Thr Asn Val Ser Ser Ser Val Pro Ala Asn
   340 345 350
   Cys Ala Asn Val Ile Ala Val Ala Ala Thr Thr Ser Ala Gly Ala Arg
   355 360 365
   Ala Ser Phe Ser Asn Tyr Gly Ala Gly Ile Asp Ile Ser Ala Pro Gly
   370 375 380
   Gln Ala Ile Leu Ser Thr Leu Asn Ser Gly Thr Thr Val Pro Gly Thr
   385 390 395 400
   Ala Ser Tyr Ala Ser Tyr Asn Gly Thr Ser Met Ala Ala Pro His Val
   405 410 415
   Ala Gly Val Val Ala Leu Val Gln Ser Val Ala Pro Thr Ala Leu Thr
   420 425 430
   Pro Ala Ala Ile Glu Thr Leu Leu Lys Asn Thr Ala Arg Ala Leu Pro
   435 440 445
   Gly Ala Cys Ser Gly Gly Cys Gly Ala Gly Ile Val Asp Ala Asp Ala
   450 455 460
   Ala Val Thr Ala Ala Leu Gly Gly Thr Asn Pro Asn Pro Gly Thr Gly
   465 470 475 480
   Thr Val Leu Gln Asn Asn Val Pro Val Ser Gly Leu Gly Ala Ala Ser
   485 490 495
   Gly Ala Ser Leu Ser Tyr Thr Val Val Val Pro Ser Gly Arg Ser Gln
   500 505 510
   Leu Lys Val Ser Ile Ala Gly Gly Ser Gly Asp Ala Asp Leu Tyr Val
   515 520 525
   Arg Ser Gly Ser Ala Pro Thr Asp Thr Val Tyr Asn Cys Arg Pro Tyr
   530 535 540
   Leu Ser Gly Asn Asn Glu Thr Cys Thr Ile Thr Ser Pro Ala Ala Gly
   545 550 555 560
   Thr Trp His Val Arg Val Lys Gly Tyr Ser Thr Phe Ser Gly Val Thr
   565 570 575
   Leu Thr Ala Gln Tyr
   580
<210> 5
   <211> 470
   <212> PRT
   <213> Künstliche Sequenz
<220>
   <223> Beschreibung der künstlichen Sequenz: DNA-Isolat,
   Delta C
<220>
   <221> SIGNAL
   <222> (1)..(32)
<400> 5
   Met Ser His Asp Ser Gln Pro Arg Leu Arg Gln Arg Ala Leu Val Val
   1 5 10 15
   Leu Gly Ala Ser Val Leu Ser Thr Leu Leu Leu Ala Ala Pro Ala Phe
   20 25 30
   Ala Gly Asp Val Gln Leu Ser Gly Leu Ser Ser Ala Pro Thr His Gln
   35 40 45
   Arg Phe Ile Val Lys Tyr Lys Asp Gly Ala Asn Leu Val Ala Thr Pro
   50 55 60
   Thr Ala Leu Ala Ser Ser Leu Lys Ala Ala Ala Ser Ala Val Pro Ala
   65 70 75 80
   Ala Gln Gly Arg Ala Leu Gly Leu Gln Lys Leu Arg Gln Leu Ala Ile
   85 90 95
   Gly Pro Thr Val Val Lys Ala Asp Arg Pro Leu Asp Ala Ala Glu Ser
   100 105 110
   Glu Leu Leu Met Arg Arg Leu Ala Ala Asp Pro Asn Val Glu Tyr Val
   115 120 125
   Glu Val Asp Gln Leu Met His Ala Thr Leu Val Pro Asn Asp Ala Arg
   130 135 140
   Leu Ser Glu Gln Trp Gly Phe Gly Thr Ser Asn Ala Ser Ile Asn Val
   145 150 155 160
   Arg Pro Ala Trp Asp Lys Ala Thr Gly Thr Gly Val Val Val Ala Val
   165 170 175
   Ile Asp Thr Gly Ile Thr Asn His Pro Asp Leu Asn Ala Asn Ile Leu
   180 185 190
   Pro Gly Tyr Asp Phe Ile Ser Asp Ala Ala Met Ala Arg Asp Gly Gly
   195 200 205
   Gly Arg Asp Asn Asn Ala Asn Asp Glu Gly Asp Trp Tyr Ala Ala Asn
   210 215 220
   Glu Cys Gly Ser Gly Ile Pro Ala Ser Asn Ser Ser Trp His Gly Thr
   225 230 235 240
   His Val Ala Gly Thr Ile Ala Ala Val Thr Asn Asn Ser Thr Gly Val
                   245                 250                 255
   Ala Gly Thr Ala Phe Asn Ala Lys Val Val Pro Val Arg Val Leu Gly
               260                 265                 270
   Lys Cys Gly Gly Tyr Thr Ser Asp Ile Ala Asp Ala Ile Val Trp Ala
           275                 280                 285
   Ser Gly Gly Thr Val Ser Gly Val Pro Ala Asn Ala Asn Pro Ala Glu
       290                 295                 300
   Val Ile Asn Met Ser Leu Gly Gly Gly Gly Thr Cys Ser Thr Thr Tyr
   305                 310                 315                 320
   Gln Asn Ala Ile Asn Gly Ala Val Ser Arg Gly Thr Thr Val Val Val
                   325                 330                 335
   Ala Ala Gly Asn Ser Asn Thr Asn Val Ser Ser Ser Val Pro Ala Asn
               340                 345                 350
   Cys Ala Asn Val Ile Ala Val Ala Ala Thr Thr Ser Ala Gly Ala Arg
           355                 360                 365
   Ala Ser Phe Ser Asn Tyr Gly Ala Gly Ile Asp Ile Ser Ala Pro Gly
       370                 375                 380
   Gln Ala Ile Leu Ser Thr Leu Asn Ser Gly Thr Thr Val Pro Gly Thr
   385                 390                 395                 400
   Ala Ser Tyr Ala Ser Tyr Asn Gly Thr Ser Met Ala Ala Pro His Val
                   405                 410                 415
   Ala Gly Val Val Ala Leu Val Gln Ser Val Ala Pro Thr Ala Leu Thr
               420                 425                 430
   Pro Ala Ala Ile Glu Thr Leu Leu Lys Asn Thr Ala Arg Ala Leu Pro
           435                 440                 445
   Gly Ala Cys Ser Gly Gly Cys Gly Ala Gly Ile Val Asp Ala Asp Ala
       450                 455                 460
   Ala Val Thr Ala Ala Leu
   465                 470
<210> 6
   <211> 1761
   <212> DNA
   <213> Unbekannt
<220>
   <223> Beschreibung des unbekannten Organismus:
   DNA-Isolat
<220>
   <221> sig_peptide
   <222> (1)..(114)
<220>
   <221> CDS
   <222> (1)..(1761)
<400> 6
   atg att acg aat tcg agc tcg gta ccc ggg gat ccg cag cgc ttg cgt    48
   Met Ile Thr Asn Ser Ser Ser Val Pro Gly Asp Pro Gln Arg Leu Arg
    1               5                  10                  15
   cag cgt gcc ttg gtt gta ctc ggt ggt tcg gtg ctt tcg acc ctg ctc    96
   Gln Arg Ala Leu Val Val Leu Gly Gly Ser Val Leu Ser Thr Leu Leu
               20                  25                  30
   ctg gcg gcg ccg gca ttc gcc ggc gac gtg cag ttg agt ggc ctg gcc    144
   Leu Ala Ala Pro Ala Phe Ala Gly Asp Val Gln Leu Ser Gly Leu Ala
           35                  40                  45
   tcg gcc ccg acc cac cag cgt ttc atc gtc aag tac aag gac ggc gcc    192
   Ser Ala Pro Thr His Gln Arg Phe Ile Val Lys Tyr Lys Asp Gly Ala
       50                  55                  60
   acc gac gtg gcc acc ccg acc gca ctg gcc agt tcg ctc aag gcc gcc    240
   Thr Asp Val Ala Thr Pro Thr Ala Leu Ala Ser Ser Leu Lys Ala Ala
    65                  70                  75                  80
   gcc caa gcc gtt ccc gcc gcg cag ggg cgc gcg ctg ggc ctg cag aag    288
   Ala Gln Ala Val Pro Ala Ala Gln Gly Arg Ala Leu Gly Leu Gln Lys
                   85                  90                  95
   ctg cgc cag ctg gcc atc ggc ccg acc gtg gtc aag gcc gac cgc ccg    336
   Leu Arg Gln Leu Ala Ile Gly Pro Thr Val Val Lys Ala Asp Arg Pro
               100                 105                 110
   ctg gat gcc gcc gaa tcg gaa ctg ctg atg cgt cgc ctg gcc gcc gat    384
   Leu Asp Ala Ala Glu Ser Glu Leu Leu Met Arg Arg Leu Ala Ala Asp
           115                 120                 125
   ccg aac gtg gaa tac gtc gaa gtc gac cag ctg atg cac gcc acc ctg    432
   Pro Asn Val Glu Tyr Val Glu Val Asp Gln Leu Met His Ala Thr Leu
       130                 135                 140
   gtg ccc aac gac agc cgc ctg tcc gag cag tgg ggc ttc ggc acc agc    480
   Val Pro Asn Asp Ser Arg Leu Ser Glu Gln Trp Gly Phe Gly Thr Ser
   145                 150                 155                 160
   aac gcc tcg atc aac gtg cgc ccg gcc tgg gac aag gcc acg ggg acc    528
   Asn Ala Ser Ile Asn Val Arg Pro Ala Trp Asp Lys Ala Thr Gly Thr
                   165                 170                 175
   ggc gtg gtg gtg gcg gtg atc gat acc ggc atc acc aac cat ccg gat    576
   Gly Val Val Val Ala Val Ile Asp Thr Gly Ile Thr Asn His Pro Asp
               180                 185                 190
   ctg aac gcc aac atc ctg ccc ggc tat gac ttc atc agc gat gcc gcg    624
   Leu Asn Ala Asn Ile Leu Pro Gly Tyr Asp Phe Ile Ser Asp Ala Ala
           195                 200                 205
   atg gcg cgc gat ggc ggt ggc cgc gac aac aat gcc aac gac gaa ggc    672
   Met Ala Arg Asp Gly Gly Gly Arg Asp Asn Asn Ala Asn Asp Glu Gly
       210                 215                 220
   gac tgg tat gcc gcc aac gaa tgc ggc gcc ggc tac ccg gcc tcc aat      720
   Asp Trp Tyr Ala Ala Asn Glu Cys Gly Ala Gly Tyr Pro Ala Ser Asn
   225                 230                 235                 240
   tcc agc tgg cac ggc acc cac gtg gcc ggc acc atc gcc gcg gtg acc      768
   Ser Ser Trp His Gly Thr His Val Ala Gly Thr Ile Ala Ala Val Thr
                   245                 250                 255
   aac aac acc acc ggc gtg gcc ggc acc gcc tac aac gcc aag gtc gtt      816
   Asn Asn Thr Thr Gly Val Ala Gly Thr Ala Tyr Asn Ala Lys Val Val
               260                 265                 270
   ccg gtg cgc gtg ctg ggc aag tgc ggc ggc tat acc tcc gac atc gcc      864
   Pro Val Arg Val Leu Gly Lys Cys Gly Gly Tyr Thr Ser Asp Ile Ala
           275                 280                 285
   gat gcg atc gtg tgg gca tcc ggc ggc acc gtc agc ggc gtg ccg gcc      912
   Asp Ala Ile Val Trp Ala Ser Gly Gly Thr Val Ser Gly Val Pro Ala
       290                 295                 300
   aat gcc aac ccg gcc gaa gtg atc aac atg tcc ctc ggc ggc ggc ggc      960
   Asn Ala Asn Pro Ala Glu Val Ile Asn Met Ser Leu Gly Gly Gly Gly
   305                 310                 315                 320
   agc tgc tcg acc acc tac cag aac gcc atc aac ggc gcg gtg tcg cgc      1008
   Ser Cys Ser Thr Thr Tyr Gln Asn Ala Ile Asn Gly Ala Val Ser Arg
                   325                 330                 335
   ggc acc acc gtg gtg gtg gca gcg ggc aac agc aac acc aac gtg tcc      1056
   Gly Thr Thr Val Val Val Ala Ala Gly Asn Ser Asn Thr Asn Val Ser
               340                 345                 350
   tcg tcg gtg ccg gcc aac tgc gcc aac gtg atc gcg gtg gcc gcc acc      1104
   Ser Ser Val Pro Ala Asn Cys Ala Asn Val Ile Ala Val Ala Ala Thr
           355                 360                 365
   acc tcg gcc ggc gcc cgc gcc agc ttc tcc aac tac ggt gcc ggc atc      1152
   Thr Ser Ala Gly Ala Arg Ala Ser Phe Ser Asn Tyr Gly Ala Gly Ile
       370                 375                 380
   gat gtc tcg gcg ccg ggc cag gcg atc ctg tcc acg ctc aac agc ggc      1200
   Asp Val Ser Ala Pro Gly Gln Ala Ile Leu Ser Thr Leu Asn Ser Gly
   385                 390                 395                 400
   acc acc gtg ccg ggc gct gcg tcc tat gcg tcg tac aac ggc acc tcg      1248
   Thr Thr Val Pro Gly Ala Ala Ser Tyr Ala Ser Tyr Asn Gly Thr Ser
                   405                 410                 415
   atg gcg gcc ccg cac gtg gcc ggc gtg gtc gcg ctg gtg cag tcg gtc      1296
   Met Ala Ala Pro His Val Ala Gly Val Val Ala Leu Val Gln Ser Val
               420                 425                 430
   gcg ccc acc gcg ctg tcg ccg gca gcc atc gag acg ctg ctc aag aac      1344
   Ala Pro Thr Ala Leu Ser Pro Ala Ala Ile Glu Thr Leu Leu Lys Asn
           435                 440                 445
   acc gca cgg gcc ctg ccg ggc gcc tgc agc ggc ggc tgc ggc gcg ggc      1392
   Thr Ala Arg Ala Leu Pro Gly Ala Cys Ser Gly Gly Cys Gly Ala Gly
       450                 455                 460
   atc gtc gat gcg gat gcg gcc gtc acc gcc gcg ctg ggc ggg acc aac      1440
   Ile Val Asp Ala Asp Ala Ala Val Thr Ala Ala Leu Gly Gly Thr Asn
   465                 470                 475                 480
   ccg aac ccg ggc acc ggc acg ctg cag aac aac gtg ccg gtc agc ggc      1488
   Pro Asn Pro Gly Thr Gly Thr Leu Gln Asn Asn Val Pro Val Ser Gly
                   485                 490                 495
   ctg ggt gct tcc agc ggt gca tcg ctg gcc tac acc gtg gcc gtg ccc      1536
   Leu Gly Ala Ser Ser Gly Ala Ser Leu Ala Tyr Thr Val Ala Val Pro
               500                 505                 510
   tcg ggt cgc tcg cag ctg aag gtg acc atc gcc ggc ggc acg ggc gat      1584
   Ser Gly Arg Ser Gln Leu Lys Val Thr Ile Ala Gly Gly Thr Gly Asp
           515                 520                 525
   gcg gac ctg tac gtg cgc tcg ggc agc gcg ccc acc gac acc gtg tac      1632
   Ala Asp Leu Tyr Val Arg Ser Gly Ser Ala Pro Thr Asp Thr Val Tyr
       530                 535                 540
   acc tgc cgc ccg tac ctg agc ggc aac aac gaa acc tgc acg atc acc      1680
   Thr Cys Arg Pro Tyr Leu Ser Gly Asn Asn Glu Thr Cys Thr Ile Thr
   545                 550                 555                 560
   gcc ccg gcc gcg ggg acc tgg cat gtc cgg gtg aag gcc tac agc acc      1728
   Ala Pro Ala Ala Gly Thr Trp His Val Arg Val Lys Ala Tyr Ser Thr
                   565                 570                 575
   ttc tcc ggc gtg acc ctg acc gcg cag tat tga                          1761
   Phe Ser Gly Val Thr Leu Thr Ala Gln Tyr
               580                 585
<210> 7
   <211> 586
   <212> PRT
   <213> Unbekannt
   <223> Beschreibung des unbekannten Organismus:
   DNA-Isolat
<400> 7
   Met Ile Thr Asn Ser Ser Ser Val Pro Gly Asp Pro Gln Arg Leu Arg
    1               5                  10                  15
   Gln Arg Ala Leu Val Val Leu Gly Gly Ser Val Leu Ser Thr Leu Leu
               20                  25                  30
   Leu Ala Ala Pro Ala Phe Ala Gly Asp Val Gln Leu Ser Gly Leu Ala
           35                  40                  45
   Ser Ala Pro Thr His Gln Arg Phe Ile Val Lys Tyr Lys Asp Gly Ala
       50                  55                  60
   Thr Asp Val Ala Thr Pro Thr Ala Leu Ala Ser Ser Leu Lys Ala Ala
   65                  70                  75                  80
   Ala Gln Ala Val Pro Ala Ala Gln Gly Arg Ala Leu Gly Leu Gln Lys
                   85                  90                  95
   Leu Arg Gln Leu Ala Ile Gly Pro Thr Val Val Lys Ala Asp Arg Pro
               100                 105                 110
   Leu Asp Ala Ala Glu Ser Glu Leu Leu Met Arg Arg Leu Ala Ala Asp
   115 120 125
   Pro Asn Val Glu Tyr Val Glu Val Asp Gln Leu Met His Ala Thr Leu
   130 135 140
   Val Pro Asn Asp Ser Arg Leu Ser Glu Gln Trp Gly Phe Gly Thr Ser
   145 150 155 160
   Asn Ala Ser Ile Asn Val Arg Pro Ala Trp Asp Lys Ala Thr Gly Thr
   165 170 175
   Gly Val Val Val Ala Val Ile Asp Thr Gly Ile Thr Asn His Pro Asp
   180 185 190
   Leu Asn Ala Asn Ile Leu Pro Gly Tyr Asp Phe Ile Ser Asp Ala Ala
   195 200 205
   Met Ala Arg Asp Gly Gly Gly Arg Asp Asn Asn Ala Asn Asp Glu Gly
   210 215 220
   Asp Trp Tyr Ala Ala Asn Glu Cys Gly Ala Gly Tyr Pro Ala Ser Asn
   225 230 235 240
   Ser Ser Trp His Gly Thr His Val Ala Gly Thr Ile Ala Ala Val Thr
   245 250 255
   Asn Asn Thr Thr Gly Val Ala Gly Thr Ala Tyr Asn Ala Lys Val Val
   260 265 270
   Pro Val Arg Val Leu Gly Lys Cys Gly Gly Tyr Thr Ser Asp Ile Ala
   275 280 285
   Asp Ala Ile Val Trp Ala Ser Gly Gly Thr Val Ser Gly Val Pro Ala
   290 295 300
   Asn Ala Asn Pro Ala Glu Val Ile Asn Met Ser Leu Gly Gly Gly Gly
   305 310 315 320
   Ser Cys Ser Thr Thr Tyr Gln Asn Ala Ile Asn Gly Ala Val Ser Arg
   325 330 335
   Gly Thr Thr Val Val Val Ala Ala Gly Asn Ser Asn Thr Asn Val Ser
   340 345 350
   Ser Ser Val Pro Ala Asn Cys Ala Asn Val Ile Ala Val Ala Ala Thr
   355 360 365
   Thr Ser Ala Gly Ala Arg Ala Ser Phe Ser Asn Tyr Gly Ala Gly Ile
   370 375 380
   Asp Val Ser Ala Pro Gly Gln Ala Ile Leu Ser Thr Leu Asn Ser Gly
   385 390 395 400
   Thr Thr Val Pro Gly Ala Ala Ser Tyr Ala Ser Tyr Asn Gly Thr Ser
   405 410 415
   Met Ala Ala Pro His Val Ala Gly Val Val Ala Leu Val Gln Ser Val
   420 425 430
   Ala Pro Thr Ala Leu Ser Pro Ala Ala Ile Glu Thr Leu Leu Lys Asn
   435 440 445
   Thr Ala Arg Ala Leu Pro Gly Ala Cys Ser Gly Gly Cys Gly Ala Gly
   450 455 460
   Ile Val Asp Ala Asp Ala Ala Val Thr Ala Ala Leu Gly Gly Thr Asn
   465 470 475 480
   Pro Asn Pro Gly Thr Gly Thr Leu Gln Asn Asn Val Pro Val Ser Gly
   485 490 495
   Leu Gly Ala Ser Ser Gly Ala Ser Leu Ala Tyr Thr Val Ala Val Pro
   500 505 510
   Ser Gly Arg Ser Gln Leu Lys Val Thr Ile Ala Gly Gly Thr Gly Asp
   515 520 525
   Ala Asp Leu Tyr Val Arg Ser Gly Ser Ala Pro Thr Asp Thr Val Tyr
   530 535 540
   Thr Cys Arg Pro Tyr Leu Ser Gly Asn Asn Glu Thr Cys Thr Ile Thr
   545 550 555 560
   Ala Pro Ala Ala Gly Thr Trp His Val Arg Val Lys Ala Tyr Ser Thr
   565 570 575
   Phe Ser Gly Val Thr Leu Thr Ala Gln Tyr
   580 585
<210> 8
   <211> 470
   <212> PRT
   <213> Künstliche Sequenz
<220>
   <223> Beschreibung der künstlichen Sequenz: DNA-Isolat,
   Delta C
<220>
   <221> SIGNAL
   <222> (1)..(32)
   <223> Signal peptide from HP70
<400> 8
   Met Ser His Asp Ser Gln Pro Arg Leu Arg Gln Arg Ala Leu Val Val
   1 5 10 15
   Leu Gly Ala Ser Val Leu Ser Thr Leu Leu Leu Ala Ala Pro Ala Phe
   20 25 30
   Ala Gly Asp Val Gln Leu Ser Gly Leu Ala Ser Ala Pro Thr His Gln
   35 40 45
   Arg Phe Ile Val Lys Tyr Lys Asp Gly Ala Thr Asp Val Ala Thr Pro
   50 55 60
   Thr Ala Leu Ala Ser Ser Leu Lys Ala Ala Ala Gln Ala Val Pro Ala
   65 70 75 80
   Ala Gln Gly Arg Ala Leu Gly Leu Gln Lys Leu Arg Gln Leu Ala Ile
   85 90 95
   Gly Pro Thr Val Val Lys Ala Asp Arg Pro Leu Asp Ala Ala Glu Ser
   100 105 110
   Glu Leu Leu Met Arg Arg Leu Ala Ala Asp Pro Asn Val Glu Tyr Val
   115 120 125
   Glu Val Asp Gln Leu Met His Ala Thr Leu Val Pro Asn Asp Ser Arg
   130 135 140
   Leu Ser Glu Gln Trp Gly Phe Gly Thr Ser Asn Ala Ser Ile Asn Val
   145 150 155 160
   Arg Pro Ala Trp Asp Lys Ala Thr Gly Thr Gly Val Val Val Ala Val
   165 170 175
   Ile Asp Thr Gly Ile Thr Asn His Pro Asp Leu Asn Ala Asn Ile Leu
   180 185 190
   Pro Gly Tyr Asp Phe Ile Ser Asp Ala Ala Met Ala Arg Asp Gly Gly
   195 200 205
   Gly Arg Asp Asn Asn Ala Asn Asp Glu Gly Asp Trp Tyr Ala Ala Asn
   210 215 220
   Glu Cys Gly Ala Gly Tyr Pro Ala Ser Asn Ser Ser Trp His Gly Thr
   225 230 235 240
   His Val Ala Gly Thr Ile Ala Ala Val Thr Asn Asn Thr Thr Gly Val
   245 250 255
   Ala Gly Thr Ala Tyr Asn Ala Lys Val Val Pro Val Arg Val Leu Gly
   260 265 270
   Lys Cys Gly Gly Tyr Thr Ser Asp Ile Ala Asp Ala Ile Val Trp Ala
   275 280 285
   Ser Gly Gly Thr Val Ser Gly Val Pro Ala Asn Ala Asn Pro Ala Glu
   290 295 300
   Val Ile Asn Met Ser Leu Gly Gly Gly Gly Ser Cys Ser Thr Thr Tyr
   305 310 315 320
   Gln Asn Ala Ile Asn Gly Ala Val Ser Arg Gly Thr Thr Val Val Val
   325 330 335
   Ala Ala Gly Asn Ser Asn Thr Asn Val Ser Ser Ser Val Pro Ala Asn
   340 345 350
   Cys Ala Asn Val Ile Ala Val Ala Ala Thr Thr Ser Ala Gly Ala Arg
   355 360 365
   Ala Ser Phe Ser Asn Tyr Gly Ala Gly Ile Asp Val Ser Ala Pro Gly
   370 375 380
   Gln Ala Ile Leu Ser Thr Leu Asn Ser Gly Thr Thr Val Pro Gly Ala
   385 390 395 400
   Ala Ser Tyr Ala Ser Tyr Asn Gly Thr Ser Met Ala Ala Pro His Val
   405 410 415
   Ala Gly Val Val Ala Leu Val Gln Ser Val Ala Pro Thr Ala Leu Ser
   420 425 430
   Pro Ala Ala Ile Glu Thr Leu Leu Lys Asn Thr Ala Arg Ala Leu Pro
   435 440 445
   Gly Ala Cys Ser Gly Gly Cys Gly Ala Gly Ile Val Asp Ala Asp Ala
   450 455 460
   Ala Val Thr Ala Ala Leu
   465 470
<210> 9
   <211> 587
   <212> PRT
   <213> Künstliche Sequenz
<220>
   <223> Beschreibung der künstlichen Sequenz:
   Consensus-Sequenz
<220>
   <221> VARIANT
   <222> (2)
   <223> - or Ile
<220>
   <221> VARIANT
   <222> (3)
   <223> Ser or Thr
<220>
   <221> VARIANT
   <222> (4)
   <223> His or Asn
<220>
   <221> VARIANT
   <222> (5)
   <223> Asp or Ser
<220>
   <221> VARIANT
   <222> (7)
   <223> - or Ser
<220>
   <221> VARIANT
   <222> (8)
   <223> - or Val
<220>
   <221> VARIANT
   <222> (9)
   <223> - or Pro
<220>
   <221> VARIANT
   <222> (10)
   <223> - or Gly
<220>
   <221> VARIANT
   <222> (11)
   <223> - or Asp
<220>
   <221> VARIANT
   <222> (12)
   <223> Gln or Pro
<220>
   <221> VARIANT
   <222> (13)
   <223> Pro or Gln
<220>
   <221> VARIANT
   <222> (25)
   <223> Ala or Gly
<220>
   <221> VARIANT
   <222> (48)
   <223> Ser or Ala
<220>
   <221> VARIANT
   <222> (65)
   <223> Asn or Thr
<220>
   <221> VARIANT
   <222> (66)
   <223> Leu or Asp
<220>
   <221> VARIANT
   <222> (82)
   <223> Ser or Gln
<220>
   <221> VARIANT
   <222> (149)
   <223> Ala or Ser
<220>
   <221> VARIANT
   <222> (234)
   <223> Ser or Ala
<220>
   <221> VARIANT
   <222> (236)
   <223> Ile or Tyr
<220>
   <221> VARIANT
   <222> (259)
   <223> Ser or Thr
<220>
   <221> VARIANT
   <222> (267)
   <223> Phe or Tyr
<220>
   <221> VARIANT
   <222> (321)
   <223> Thr or Ser
<220>
   <221> VARIANT
   <222> (386)
   <223> Ile or Val
<220>
   <221> VARIANT
   <222> (406)
   <223> Thr or Ala
<220>
   <221> VARIANT
   <222> (438)
   <223> Thr or Ser
<220>
   <221> VARIANT
   <222> (487)
   <223> Thr or -
<220>
   <221> VARIANT
   <222> (488)
   <223> Val or Thr
<220>
   <221> VARIANT
   <222> (501)
   <223> Ala or Ser
<220>
   <221> VARIANT
   <222> (507)
   <223> Ser or Ala
<220>
   <221> VARIANT
   <222> (511)
   <223> Val or Ala
<220>
   <221> VARIANT
   <222> (522)
   <223> Ser or Thr
<220>
   <221> VARIANT
   <222> (527)
   <223> Ser or Thr
<220>
   <221> VARIANT
   <222> (546)
   <223> Asn or Thr
<220>
   <221> VARIANT
   <222> (562)
   <223> Ser or Ala
<220>
   <221> VARIANT
   <222> (574)
   <223> Gly or Ala
<400> 9
   Met Xaa Xaa Xaa Xaa Ser Xaa Xaa Xaa Xaa Xaa Xaa Xaa Arg Leu Arg
   1               5                   10                  15
   Gln Arg Ala Leu Val Val Leu Gly Xaa Ser Val Leu Ser Thr Leu Leu
               20                  25                  30
   Leu Ala Ala Pro Ala Phe Ala Gly Asp Val Gln Leu Ser Gly Leu Xaa
           35                  40                  45
   Ser Ala Pro Thr His Gln Arg Phe Ile Val Lys Tyr Lys Asp Gly Ala
       50                  55                  60
   Xaa Xaa Val Ala Thr Pro Thr Ala Leu Ala Ser Ser Leu Lys Ala Ala
   65                  70                  75                  80
   Ala Xaa Ala Val Pro Ala Ala Gln Gly Arg Ala Leu Gly Leu Gln Lys
                   85                  90                  95
   Leu Arg Gln Leu Ala Ile Gly Pro Thr Val Val Lys Ala Asp Arg Pro
               100                 105                 110
   Leu Asp Ala Ala Glu Ser Glu Leu Leu Met Arg Arg Leu Ala Ala Asp
           115                 120                 125
   Pro Asn Val Glu Tyr Val Glu Val Asp Gln Leu Met His Ala Thr Leu
       130                 135                 140
   Val Pro Asn Asp Xaa Arg Leu Ser Glu Gln Trp Gly Phe Gly Thr Ser
   145                 150                 155                 160
   Asn Ala Ser Ile Asn Val Arg Pro Ala Trp Asp Lys Ala Thr Gly Thr
                   165                 170                 175
   Gly Val Val Val Ala Val Ile Asp Thr Gly Ile Thr Asn His Pro Asp
               180                 185                 190
   Leu Asn Ala Asn Ile Leu Pro Gly Tyr Asp Phe Ile Ser Asp Ala Ala
           195                 200                 205
   Met Ala Arg Asp Gly Gly Gly Arg Asp Asn Asn Ala Asn Asp Glu Gly
       210                 215                 220
   Asp Trp Tyr Ala Ala Asn Glu Cys Gly Xaa Gly Xaa Pro Ala Ser Asn
   225                 230                 235                 240
   Ser Ser Trp His Gly Thr His Val Ala Gly Thr Ile Ala Ala Val Thr
                   245                 250                 255
   Asn Asn Xaa Thr Gly Val Ala Gly Thr Ala Xaa Asn Ala Lys Val Val
               260                 265                 270
   Pro Val Arg Val Leu Gly Lys Cys Gly Gly Tyr Thr Ser Asp Ile Ala
           275                 280                 285
   Asp Ala Ile Val Trp Ala Ser Gly Gly Thr Val Ser Gly Val Pro Ala
       290                 295                 300
   Asn Ala Asn Pro Ala Glu Val Ile Asn Met Ser Leu Gly Gly Gly Gly
   305                 310                 315                 320
   Xaa Cys Ser Thr Thr Tyr Gln Asn Ala Ile Asn Gly Ala Val Ser Arg
                   325                 330                 335
   Gly Thr Thr Val Val Val Ala Ala Gly Asn Ser Asn Thr Asn Val Ser
               340                 345                 350
   Ser Ser Val Pro Ala Asn Cys Ala Asn Val Ile Ala Val Ala Ala Thr
           355                 360                 365
   Thr Ser Ala Gly Ala Arg Ala Ser Phe Ser Asn Tyr Gly Ala Gly Ile
       370                 375                 380
   Asp Xaa Ser Ala Pro Gly Gln Ala Ile Leu Ser Thr Leu Asn Ser Gly
   385                 390                 395                 400
   Thr Thr Val Pro Gly Xaa Ala Ser Tyr Ala Ser Tyr Asn Gly Thr Ser
                   405                 410                 415
   Met Ala Ala Pro His Val Ala Gly Val Val Ala Leu Val Gln Ser Val
               420                 425                 430
   Ala Pro Thr Ala Leu Xaa Pro Ala Ala Ile Glu Thr Leu Leu Lys Asn
           435                 440                 445
   Thr Ala Arg Ala Leu Pro Gly Ala Cys Ser Gly Gly Cys Gly Ala Gly
       450                 455                 460
   Ile Val Asp Ala Asp Ala Ala Val Thr Ala Ala Leu Gly Gly Thr Asn
   465                 470                 475                 480
   Pro Asn Pro Gly Thr Gly Xaa Xaa Leu Gln Asn Asn Val Pro Val Ser
                   485                 490                 495
   Gly Leu Gly Ala Xaa Ser Gly Ala Ser Leu Xaa Tyr Thr Val Xaa Val
               500                 505                 510
   Pro Ser Gly Arg Ser Gln Leu Lys Val Xaa Ile Ala Gly Gly Xaa Gly
           515                 520                 525
   Asp Ala Asp Leu Tyr Val Arg Ser Gly Ser Ala Pro Thr Asp Thr Val
       530                 535                 540
   Tyr Xaa Cys Arg Pro Tyr Leu Ser Gly Asn Asn Glu Thr Cys Thr Ile
   545                 550                 555                 560
   Thr Xaa Pro Ala Ala Gly Thr Trp His Val Arg Val Lys Xaa Tyr Ser
                   565                 570                 575
   Thr Phe Ser Gly Val Thr Leu Thr Ala Gln Tyr
               580                 585
<210> 10
   <211> 27
   <212> DNA
   <213> Künstliche Sequenz
<220>
   <223> Beschreibung der künstlichen Sequenz: Primer HP70f
<400> 10
   gaattcgatg tctcatgatt cgcaacc 27
<210> 11
   <211> 29
   <212> DNA
   <213> Künstliche Sequenz
<220>
   <223> Beschreibung der künstlichen Sequenz: Primer HP70r
<400> 11
   ggatcctcac agcgccgccg tgaccgccg 29
<210> 12
   <211> 25
   <212> DNA
   <213> Künstliche Sequenz
<220>
   <223> Beschreibung der künstlichen Sequenz: Primer HP53f
<400> 12
   gctgagcggc ctggcctcgg ccccg 25
<210> 13
   <211> 29
   <212> DNA
   <213> Künstliche Sequenz
<220>
   <223> Beschreibung der künstlichen Sequenz: Primer HP53r
<400> 13
   ggatcctcac agcgccgccg tgacggccg 29